# EUROPEAN PATENT SPECIFICATION

(11) **EP 1 751 304 B1**
(45) Date of publication and mention of the grant of the patent: **01.12.2010**
(21) Application number: 05744466.3
(22) Date of filing: 03.05.2005
(51) Int. Cl.: C12Q 1/68

(54) **DIAGNOSTICS AND THERAPEUTICS FOR DISEASES ASSOCIATED WITH AN IL-1 INFLAMMATORY HAPLOTYPE**
DIAGNOSTIKA UND THERAPEUTIKA FÜR MIT EINEM ENTZÜNDLICHEN IL-1-HAPLOTYP ASSOZIIERTE KRANKHEITEN
MOYENS DIAGNOSTIQUES ET THERAPEUTIQUES POUR MALADIES ASSOCIEES A UN HAPLOTYPE INFLAMMATOIRE D'IL-1

(30) Priority: 03.05.2004 US 567727 P
(43) Date of publication of application: 14.02.2007
(73) Proprietor: Interleukin Genetics, Inc., Waltham, MA 02452 (US)
(72) Inventor: DUFF, Gordon, W., Sheffield S10 3BZ (GB); KORNMAN, Kenneth, Newton, MA 02459 (US); WILKINS, Leon, North Andover, MA 01845 (US); CHEN, Hong, Min, Devens, MA 01432 (US); ROGUS, John, North Andover, MA 01845 (US)
(74) Representative: Peter, Beate
(86) International application number: PCT/US2005/015267
(87) International publication number: WO 2005/108619

(56) References cited:
- US-A1- 2003 152 947
- US-A1- 2003 235 890
- DENNIS RICHARD A ET AL: "Interleukin-1 polymorphisms are associated with the inflammatory response in human muscle to acute resistance exercise" JOURNAL OF PHYSIOLOGY (OXFORD), vol. 560, no. 3, 1 November 2004 (2004-11-01), pages 617-626, XP002359027 ISSN: 0022-3751
- KORNMAN K S ET AL: "INTERLEUKIN-1 GENOTYPES AND THE ASSOCIATO BETWEEN PERIODONTITIS AND CARDIOVASCULAR DISEASE" JOURNAL OF PERIODONTOLOGY, AMERICAN ACADEMY OF PERIODONTOLOGY, CHICAGO, IL, US, vol. 34, no. 7, October 1999 (1999-10), pages 353-357, XP009004778 ISSN: 0022-3492

## Description

### 1. Background of the Invention

### Genetics of the IL-1 Gene Cluster

The IL-1 gene cluster is on the long arm of chromosome 2 (2q13) and contains at least the genes for IL-1α (IL-1A), IL-1β (IL-1B), and the IL-1 receptor antagonist (IL-1RN), within a region of 430 Kb (Nicklin, et al. (1994) Genomics, 19: 382-4). The agonist molecules, IL-1 a and IL-1β, have potent pro-inflammatory activity and are at the head of many inflammatory cascades. Their actions, often via the induction of other cytokines such as IL-6 and IL-8, lead to activation and recruitment of leukocytes into damaged tissue, local production of vasoactive agents, fever response in the brain and hepatic acute phase response. All three IL-1 molecules bind to type I and to type II IL-1 receptors, but only the type I receptor transduces a signal to the interior of the cell. In contrast, the type II receptor is shed from the cell membrane and acts as a decoy receptor. The receptor antagonist and the type II receptor, therefore, are both anti-inflammatory in their actions.

Inappropriate production of IL-1 plays a central role in the pathology of many autoimmune and inflammatory diseases, including rheumatoid arthritis; inflammatory bowel disorder, psoriasis, and the like. In addition, there are stable inter-individual differences in the rates of production of IL-1, and some of this variation may be accounted for by genetic differences at IL-1 gene loci. Thus, the IL-1 genes are reasonable candidates for determining part of the genetic susceptibility to inflammatory diseases, most of which have a multifactorial etiology with a polygenic component.

Certain alleles from the IL-1 gene cluster are known to be associated with particular disease states. For example, IL-1RN (VNTR) allele 2 has been shown to be associated with osteoporosis (U.S. Patent No. 5,698,399), nephropathy in diabetes mellitus (Blakemore, et al. (1996) Hum. Genet 97(3): 369-74), alopecia areata (Cork, et al., (1995) J. Invest. Dermatol. 104(5 Supp.): 15S-16S; Cork et al. (1996) Dermatol Clin 14: 671-8), Graves disease (Blakemore, et al. (1995) J. Clin. Endocrinol. 80(1): 111-5), systemic lupus erythematosus (Blakemore, et al. (1994) Arthritis Rheum. 37: 1380-85), lichen sclerosis (Clay, et al. (1994) Hum. Genet 94: 407-10), and ulcerative colitis (Mansfield, et al. (1994) Gastoenterol. 106(3): 637-42)).

In addition, the IL-1A allele 2 from marker -889 and IL-1B (TaqI) allele 2 from marker +3954 have been found to be associated with periodontal disease (U.S. Patent No. 5,686,246; Kornman and diGiovine (1998) Ann Periodont 3: 327-38; Hart and Kornman (1997) Periodontol 2000 14: 202-15; Newman (1997) Compend Contin Educ Dent 18: 881-4; Kornman et al. (1997) J. Clin Periodontol 24: 72-77). The IL-1A allele 2 from marker - 889 has also been found to be associated with juvenile chronic arthritis, particularly chronic iridocyclitis (McDowell, et al. (1995) Arthritis Rheum. 38: 221-28 ). The IL-1B (TaqI) allele 2 from marker +3954 of IL-1B has also been found to be associated with psoriasis and insulin dependent diabetes in DR3/4 patients (di Giovine, et al. (1995) Cytokine 7: 606; Pociot, et al. (1992) Eur J. Clin. Invest. 22: 396-402). Additionally, the IL-1RN (VNTR) allele 1 has been found to be associated with diabetic retinopathy (see USSN 09/037472, and PCT/GB97/02790). Furthermore allele 2 of IL-1RN (VNTR) has been found to be associated with ulcerative colitis in Caucasian populations from North America and Europe (Mansfield, J. et al., (1994) Gastroenterology 106: 637-42). Interestingly, this association is particularly strong within populations of ethnically related Ashkenazi Jews (PCT WO97/25445).

### Genotype Screening

Traditional methods for the screening of heritable diseases have depended on either the identification of abnormal gene products (e.g., sickle cell anemia) or an abnormal phenotype (e.g., mental retardation). These methods are of limited utility for heritable diseases with late onset and no easily identifiable phenotypes such as, for example, vascular disease. With the development of simple and inexpensive genetic screening methodology, it is now possible to identify polymorphisms that indicate a propensity to develop disease, even when the disease is of polygenic origin. The number of diseases that can be screened by molecular biological methods continues to grow with increased understanding of the genetic basis of multifactorial disorders.

Genetic screening (also called genotyping or molecular screening), can be broadly defined as testing to determine if a patient has mutations (alleles or polymorphisms) that either cause a disease state or are "linked" to the mutation causing a disease state. Linkage refers to the phenomenon that DNA sequences which are close together in the genome have a tendency to be inherited together. Two sequences may be linked because of some selective advantage of co-inheritance. More typically, however, two polymorphic sequences are co-inherited because of the relative infrequency with which meiotic recombination events occur within the region between the two polymorphisms. The co-inherited polymorphic alleles are said to be in linkage disequilibrium with one another because, in a given human population, they tend to either both occur together or else not occur at all in any particular member of the population. Indeed, where multiple polymorphisms in a given chromosomal region are found to be in linkage disequilibrium with one another, they define a quasi-stable genetic "haplotype." In contrast, recombination events occurring between two polymorphic loci cause them to become separated onto distinct homologous chromosomes. If meiotic recombination between two physically linked polymorphisms occurs frequently enough, the two polymorphisms will appear to segregate independently and are said to be in linkage equilibrium.

While the frequency of meiotic recombination between two markers is generally proportional to the physical distance between them on the chromosome, the occurrence of "hot spots" as well as regions of repressed chromosomal recombination can result in discrepancies between the physical and recombinational distance between two markers. Thus, in certain chromosomal regions, multiple polymorphic loci spanning a broad chromosomal domain may be in linkage disequilibrium with one another, and thereby define a broad-spanning genetic haplotype. Furthermore, where a disease-causing mutation is found within or in linkage with this haplotype, one or more polymorphic alleles of the haplotype can be used as a diagnostic or prognostic indicator of the likelihood of developing the disease. This association between otherwise benign polymorphisms and a disease-causing polymorphism occurs if the disease mutation arose in the recent past, so that sufficient time has not elapsed for equilibrium to be achieved through recombination events. Therefore identification of a human haplotype which spans or is linked to a disease-causing mutational change, serves as a predictive measure of an individual's likelihood of having inherited that disease-causing mutation. Importantly, such prognostic or diagnostic procedures can be utilized without necessitating the identification and isolation of the actual disease-causing lesion. This is significant because the precise determination of the molecular defect involved in a disease process can be difficult and laborious, especially in the case of multifactorial diseases such as inflammatory disorders.

Indeed, the statistical correlation between an inflammatory disorder and an IL-1 polymorphism does not necessarily indicate that the polymorphism directly causes the disorder. Rather the correlated polymorphism may be a benign allelic variant which is linked to (i.e. in linkage disequilibrium with) a disorder-causing mutation which has occurred in the recent human evolutionary past, so that sufficient time has not elapsed for equilibrium to be achieved through recombination events in the intervening chromosomal segment. Thus, for the purposes of diagnostic and prognostic assays for a particular disease, detection of a polymorphic allele associated with that disease can be utilized without consideration of whether the polymorphism is directly involved in the etiology of the disease. Furthermore, where a given benign polymorphic locus is in linkage disequilibrium with an apparent disease-causing polymorphic locus, still other polymorphic loci which are in linkage disequilibrium with the benign polymorphic locus are also likely to be in linkage disequilibrium with the disease-causing polymorphic locus. Thus these other polymorphic loci will also be prognostic or diagnostic of the likelihood of having inherited the disease-causing polymorphic locus. Indeed, a broad-spanning human haplotype (describing the typical pattern of co-inheritance of alleles of a set of linked polymorphic markers) can be targeted for diagnostic purposes once an association has been drawn between a particular disease or condition and a corresponding human haplotype. Thus, the determination of an individual's likelihood for developing a particular disease of condition can be made by characterizing one or more disease-associated polymorphic alleles (or even one or more disease-associated haplotypes) without necessarily determining or characterizing the causative genetic variation.

### 2. Summary of the Invention

The present invention provides a method for predicting a human subject's susceptibility to periodontal disease comprising the step of identifying in a genomic DNA sample from the subject an allelic pattern that comprises one of the following sets of IL-1 B alleles:
(1) (-511) allele 1, (-1468) allele 1, and (-3737) allele 2;
(2) (-511) allele 2, (-1468) allele 2, and (-3737) allele 1;
(3) (-511) allele 1, (-1468) allele 1, and (-3737) allele 1; or
(4) (-511) allele 2, (-1468) allele 1, and (-3737) allele 1,
wherein the presence of any one of allelic patterns 1,3 or 4 indicates that said subject has increased susceptibility to periodontal disease, and wherein the presence of allelic pattern 2 indicates that the subject has a decreased susceptibility to periodontal disease.

The subject is homozygous for each of the allele. Altered IL-1B transcription results in altered IL-1B production. The subject is of Asian ethnicity such as Japanese, Chinese, Taiwanese, or Vietnamese.

An allele comprising an IL-1 inflammatory haplotype can be detected by any of a variety of available techniques, including: 1) performing a hybridization reaction between a nucleic acid sample and a probe that is capable of hybridizing to the allele; 2) sequencing at least a portion of the allele; or 3) determining the electrophoretic mobility of the allele or fragments thereof (e.g., fragments generated by endonuclease digestion). The allele can optionally be subjected to an amplification step prior to performance of the detection step. Amplification methods include for example the polymerase chain reaction (PCR), the ligase chain reaction (LCR), strand displacement amplification (SDA), cloning, and variations of the above (e.g. RT-PCR and allele specific amplification). Oligonucleotides necessary for amplification are selected, for example, from within the IL-1 gene loci, either flanking the marker of interest (as required for PCR amplification) or directly overlapping the marker (as in ASO hybridization). For example, the sample is hybridized with a set of primers, which hybridize 5' and 3' in a sense or antisense sequence to theIL-1 inflammatory haplotype associated allele, and is subjected to a PCR amplification.

An allele containing an IL-1 inflammatory haplotype is also detected indirectly, e.g. by analyzing the protein product encoded by the DNA. For example, where the marker in question results in the translation of a mutant protein, the protein is detected by any of a variety of protein detection methods. Such methods include immunodetection and biochemical tests, such as size fractionation, where the protein has a change in apparent molecular weight either through truncation, elongation, altered folding or altered post-translational modifications.

Disclosed are kits for performing the above-described assays. The kit can include a nucleic acid sample collection means and a means for determining whether a subject carries at least one allele comprising an IL-1 inflammatory haplotype. Optionally, the kit contains a control sample either positive or negative or a standard and/or an algorithmic device for assessing the results and additional reagents and components including for example DNA amplification reagents, DNA polymerase, nucleic acid amplification reagents, restrictive enzymes, buffers, a nucleic acid sampling device, DNA purification device, deoxynucleotides, oligonucleotides (e.g. probes and primers) etc.

The control may be a positive or negative control. Further, the control sample may contain the positive (or negative) products of the allele detection technique employed. For example, where the allele detection technique is PCR amplification, followed by size fractionation, the control sample may comprise DNA fragments of the appropriate size. Likewise, where the allele detection technique involves detection of a mutated protein, the control sample may comprise a sample of mutated protein. However, it is preferred that the control sample comprises the material to be tested. For example, the controls may be a sample of genomic DNA or a cloned portion of the IL-1 gene cluster. Preferably, the control sample is a highly purified sample of genomic DNA where the sample to be tested is genomic DNA.

The oligonucleotides present in said kit are used for amplification of the region of interest or for direct allele specific oligonucleotide (ASO) hybridization to the markers in question. Thus, the oligonucleotides either flank the marker of interest (as required for PCR amplification) or directly overlap the marker (as in ASO hybridization).

Also provided are isolated nucleic acids less than 250, 200, 150, 100, 50, 25 or less nucleotides in length containing the nucleic acid sequence of SEQ ID NO;36-39. Optionally, the nucleic acid is affixed to a solid or semi-solid support or is present on an oligonucleotide array.

Information obtained using the assays and kits described herein (alone or in conjunction with information on another genetic defect or environmental factor, which contributes to the disease or condition that is associated with an IL-1 inflammatory haplotype) is useful for determining whether a non-symptomatic subject has or is likely to develop the particular disease or condition. In addition, the information can allow a more customized approach to preventing the onset or progression of the disease or condition. For example, this information can enable a clinician to more effectively prescribe a therapy that will address the molecular basis of the disease or condition.

Disclosed are methods for treating or preventing the development of a disease or condition that is associated with an IL-1 inflammatory haplotype in a subject by administering to the subject an appropriate therapeutic of the invention. In still another aspect, the invention provides in vitro or in vivo assays for screening test compounds to identify therapeutics for treating or preventing the development of a disease or condition that is associated with an IL-1 inflammatory haplotype. The assay comprises contacting a cell transfected with a causative mutation that is operably linked to an appropriate promoter with a test compound and determining the level of expression of a protein in the cell in the presence and in the absence of the test compound. The causative mutation results in decreased production of IL-1 receptor antagonist, and increased production of the IL-1 receptor antagonist in the presence of the test compound indicates that the compound is an agonist of IL-1 receptor antagonist activity. Alternatively, the causative mutation results in increased production of IL-1α a or IL-1β, and decreased production of IL-1 α or IL-1β in the presence of the test compound indicates that the compound is an antagonist of IL-1α or IL-1β activity.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other embodiments and advantages are set forth in the following detailed description and claims.

### 3. Brief Description of the Figures

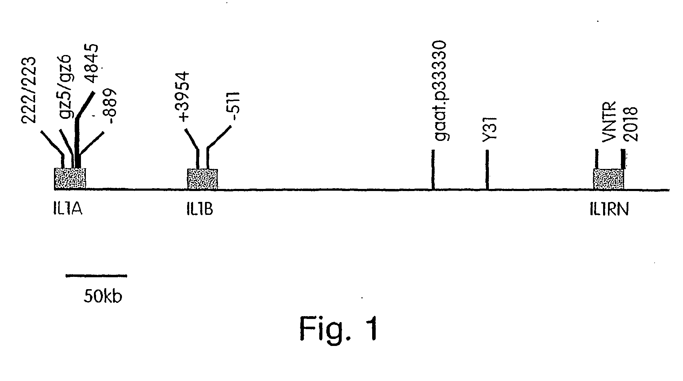

FIG. 1 is a schematic depiction of the IL-1 gene cluster including a few polymorphic markers.

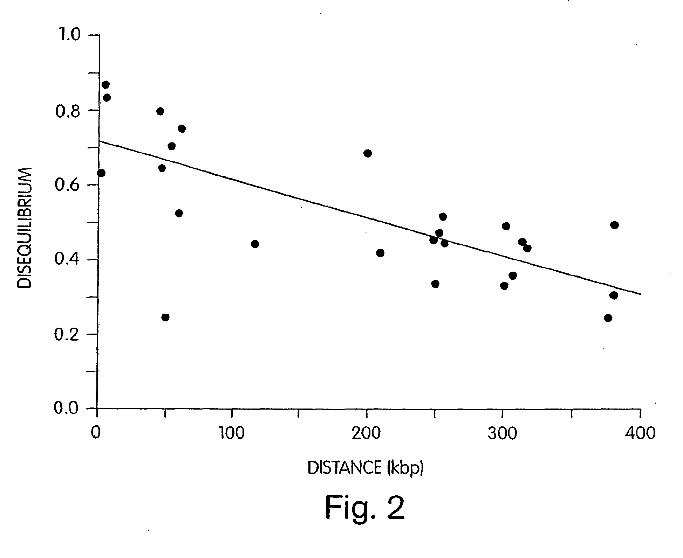

FIG. 2 is a graph which plots the correlation between disequilibrium values and
physical distance as described herein.

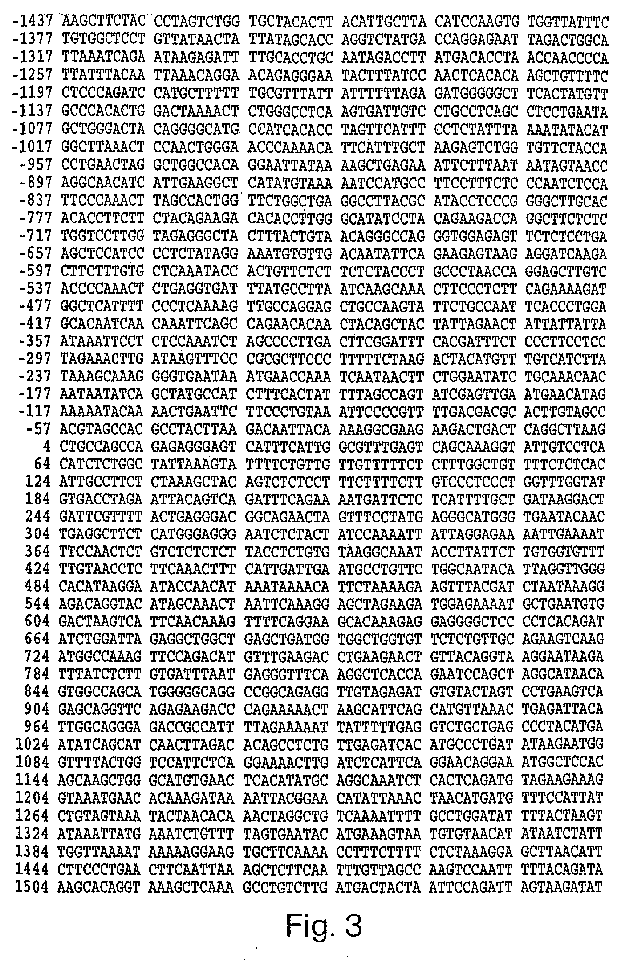

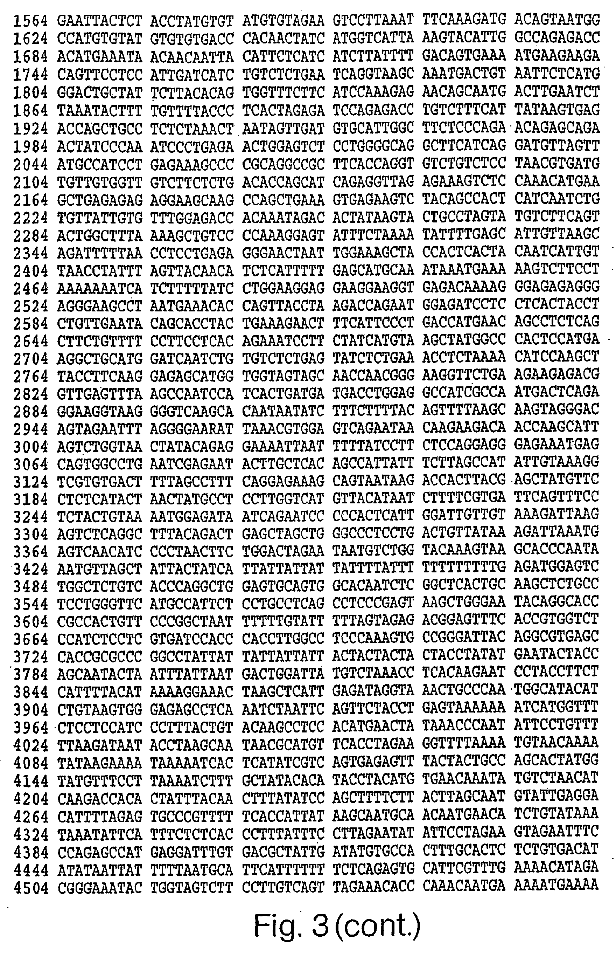

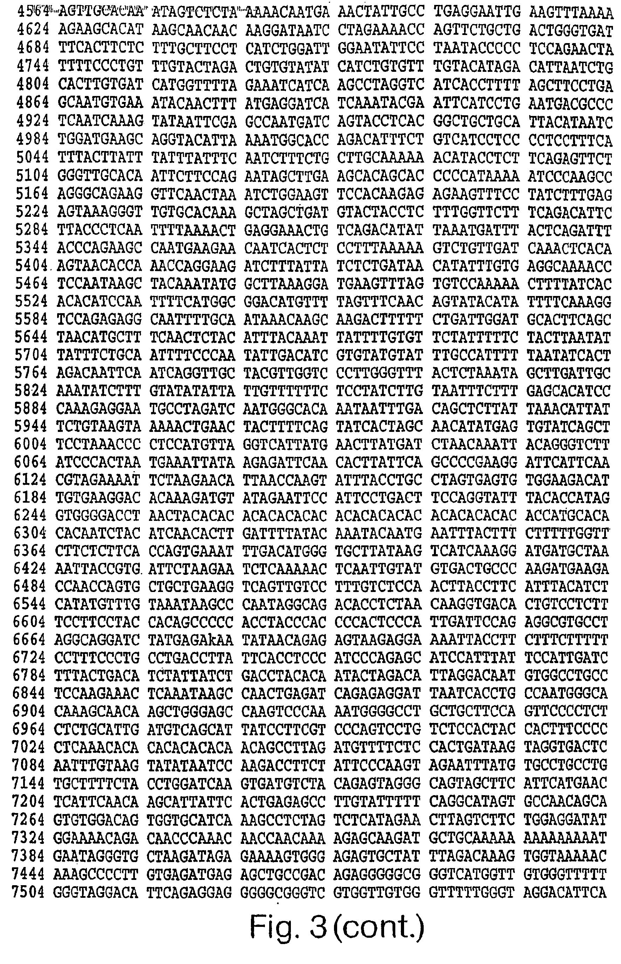

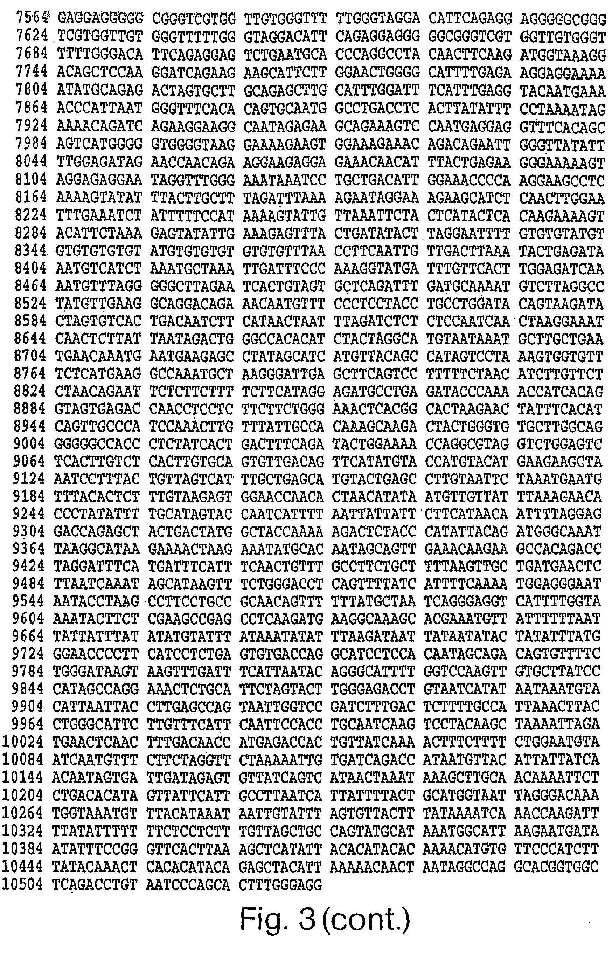

FIG. 3 shows the nucleic acid sequence for IL-1A (GEN X03833; SEQ ID No. 1).

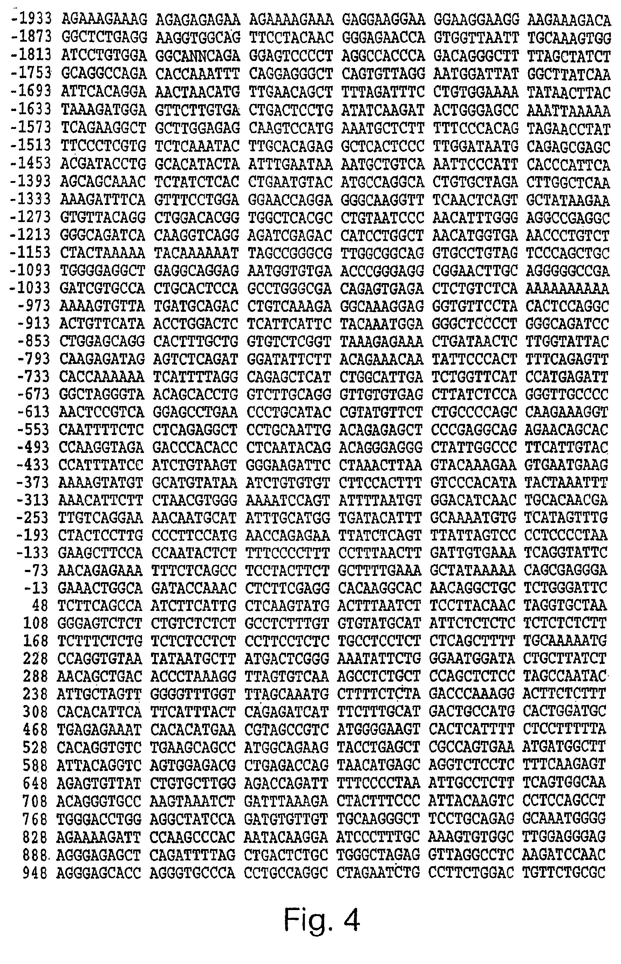

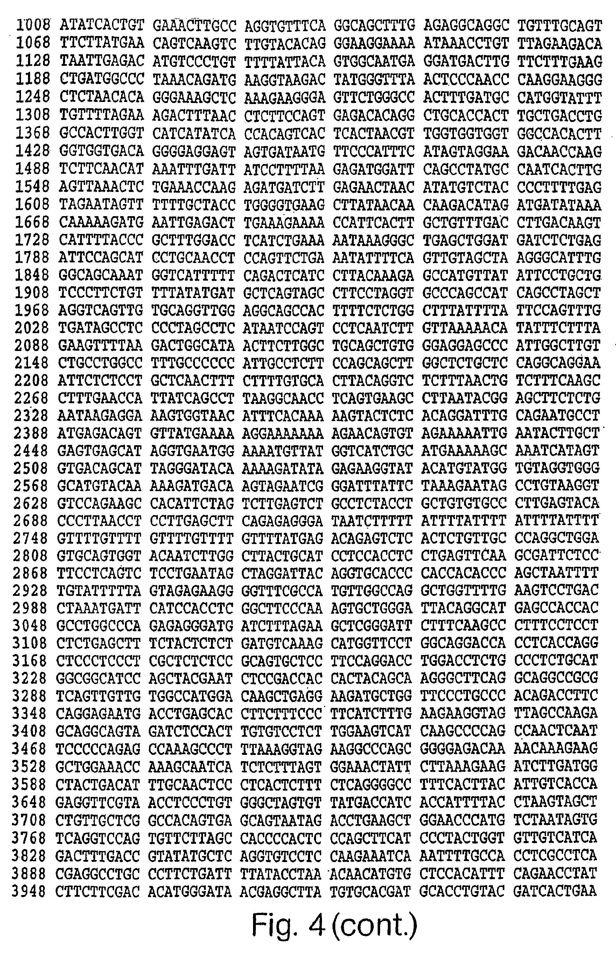

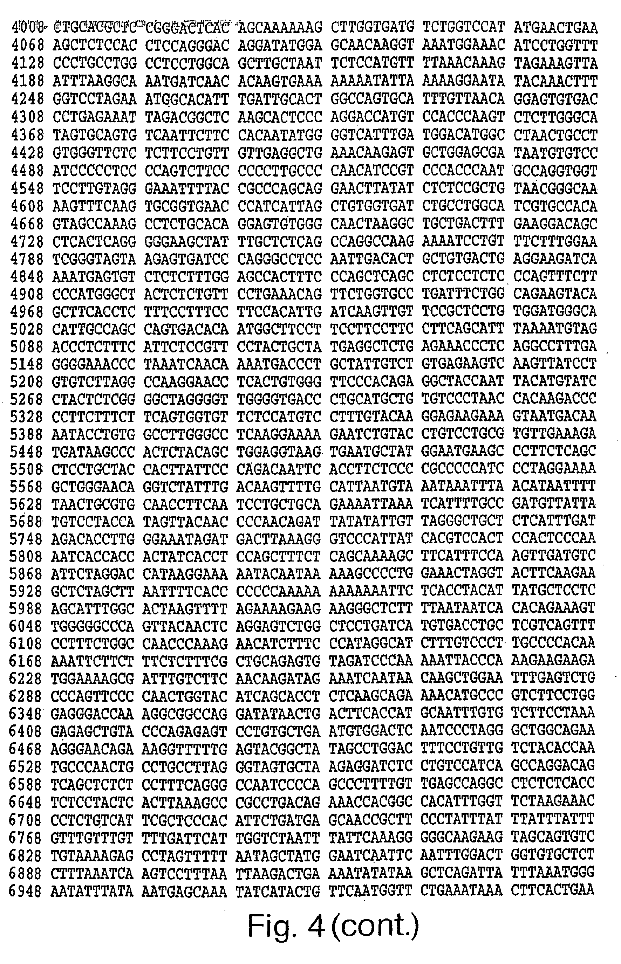

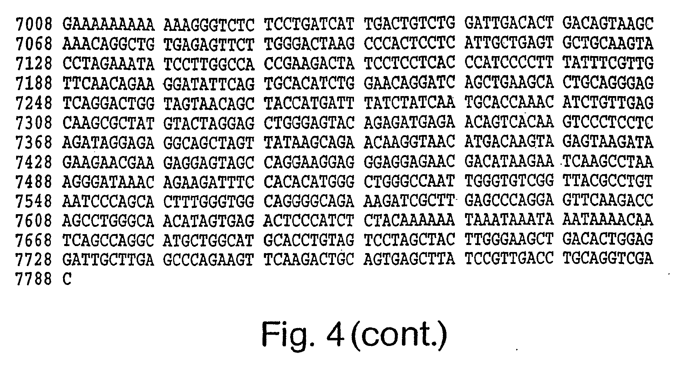

FIG. 4 shows the nucleic acid sequence for IL-1B (GEN X04500; SEQ ID No. 2).

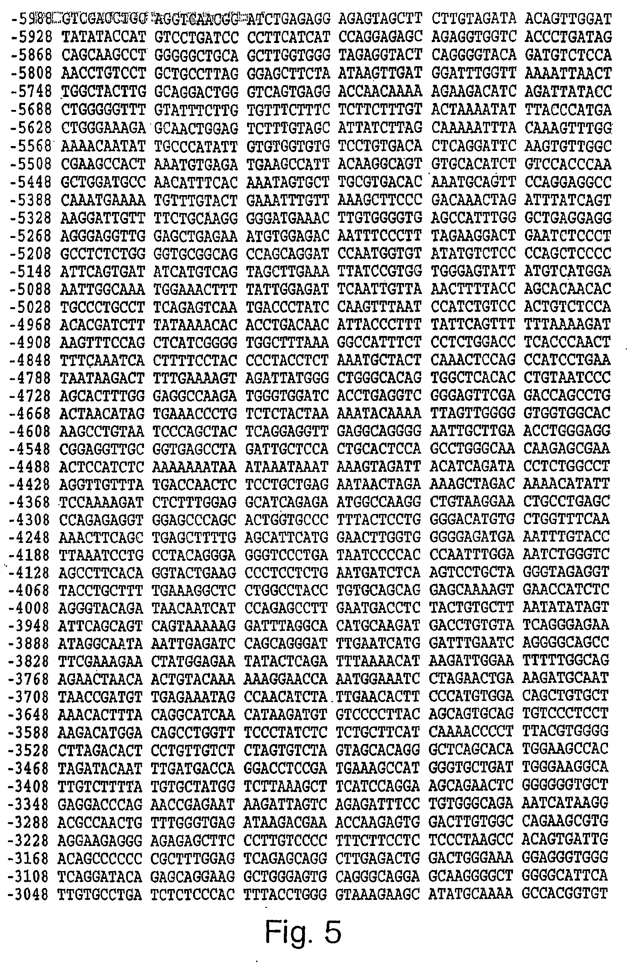

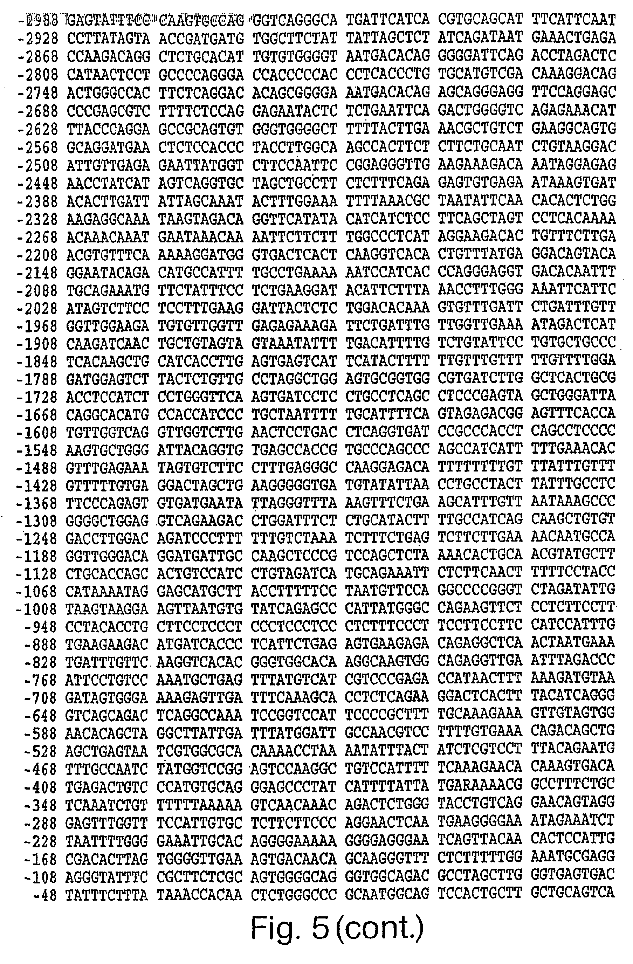

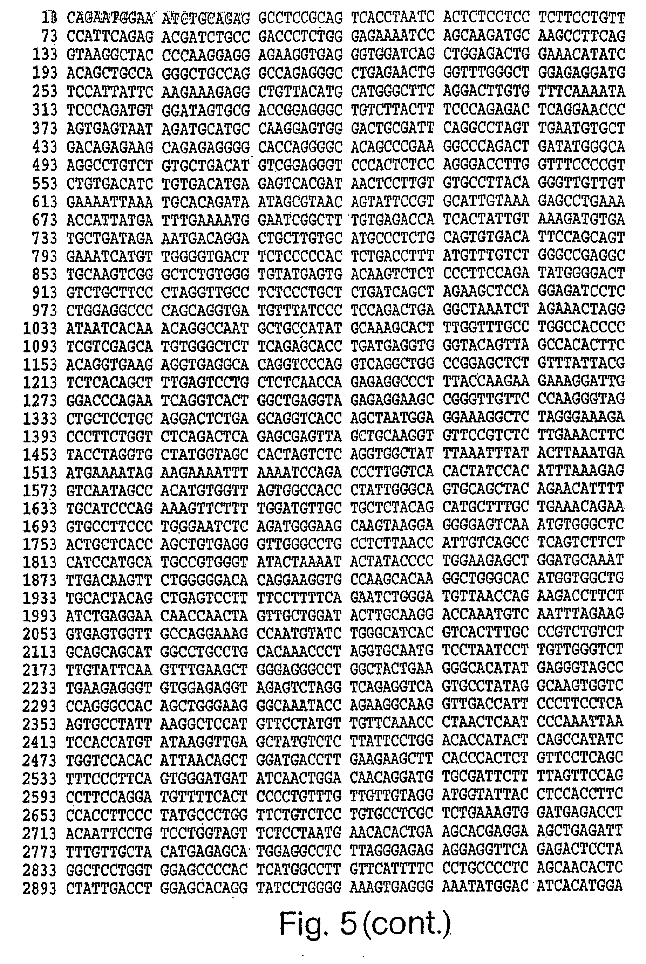

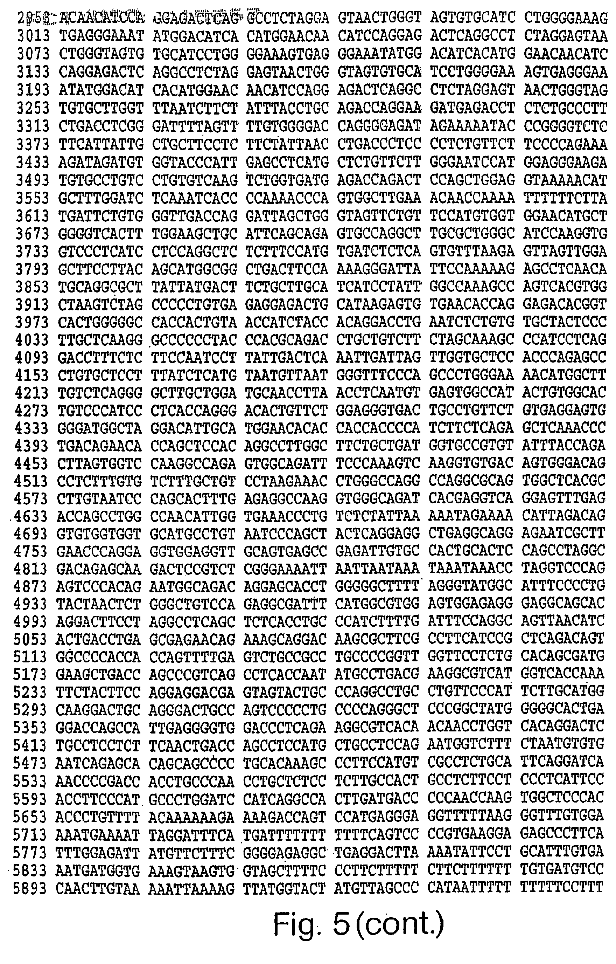

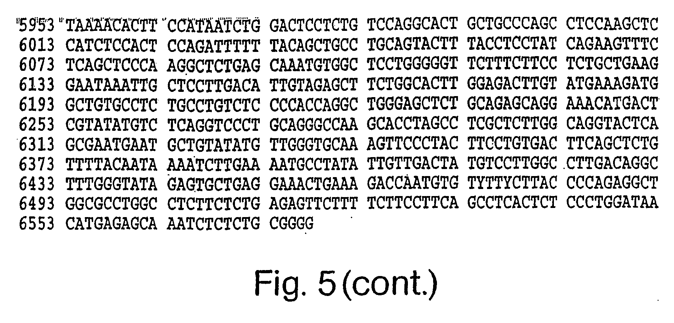

FIG. 5 shows the nucleic acid sequence for the secreted IL-1RN (GEN X64532; SEQ ID No. 3).

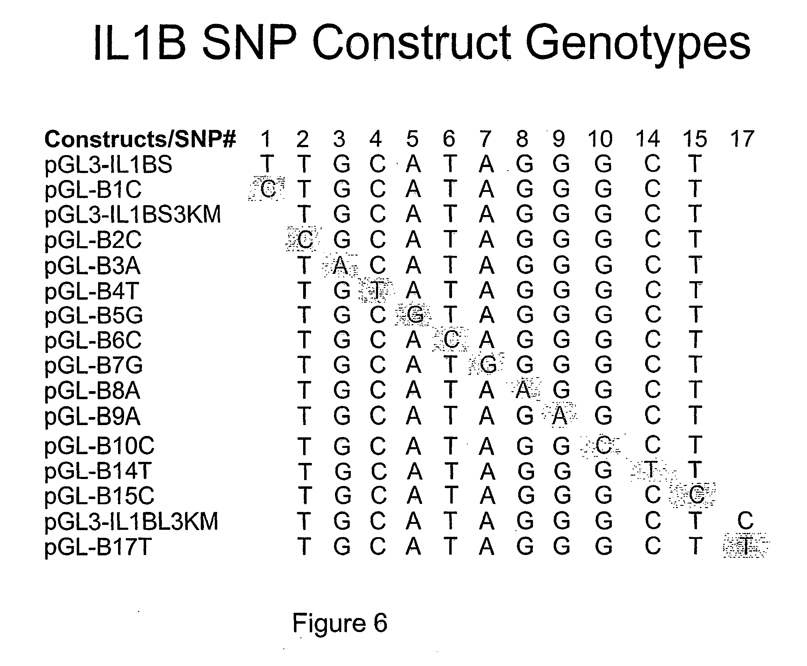

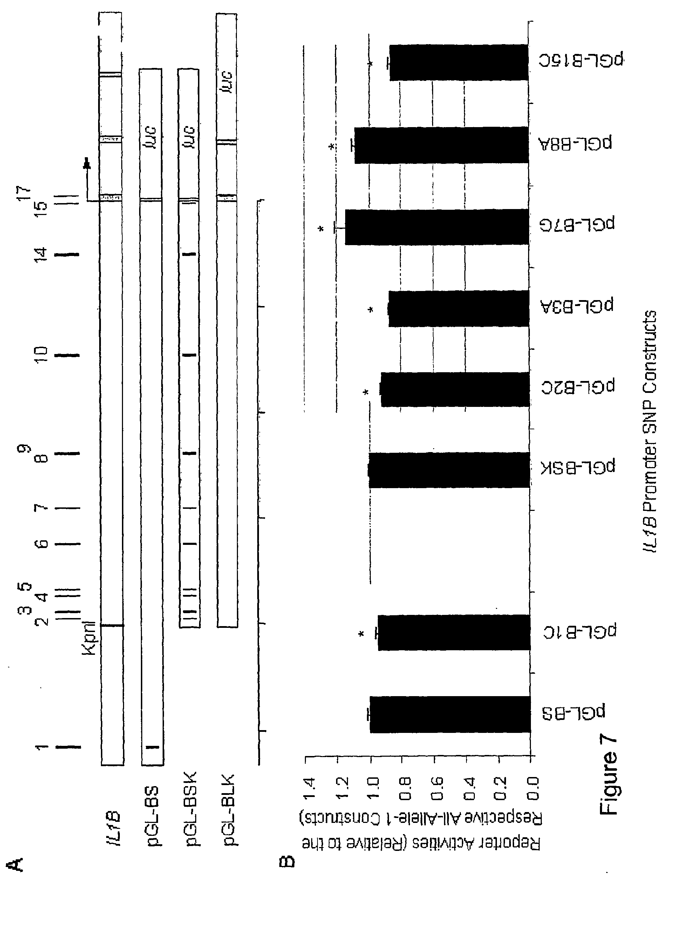

FIG. 6 shows constructs with SNPs within the IL-1B promoter region. The parental construct where all SNPs are allele 1 are indicated pGL3(IL1)BS for SNPs 1-15, pGL(3-IL1)BS(3)K(M) for SNPs 2-15, and pGL3-(IL1)BL(3)KM for SNPs 2-17 (parentheses indicate parts of construct identifiers that do not appear in the graph in Fig. 7). Below each parental series are constructs where individual SNP has been converted to allele 2 by site directed mutagenesis.
FIG.7A-B show location of SNPs and promoter constructs. In FIG. 7A, the SNP positions are indicated within the IL-1B gene promoter region on the top line. The lines below represent the lengths of the promoter region used in the different reporter (luciferase) constructs, the relative transcriptional activity results of which are shown in FIG. 7B. The Kpn I and Bam HI sites are indicated. In the IL-1BS series, the SNPs are indicated by vertical lines.

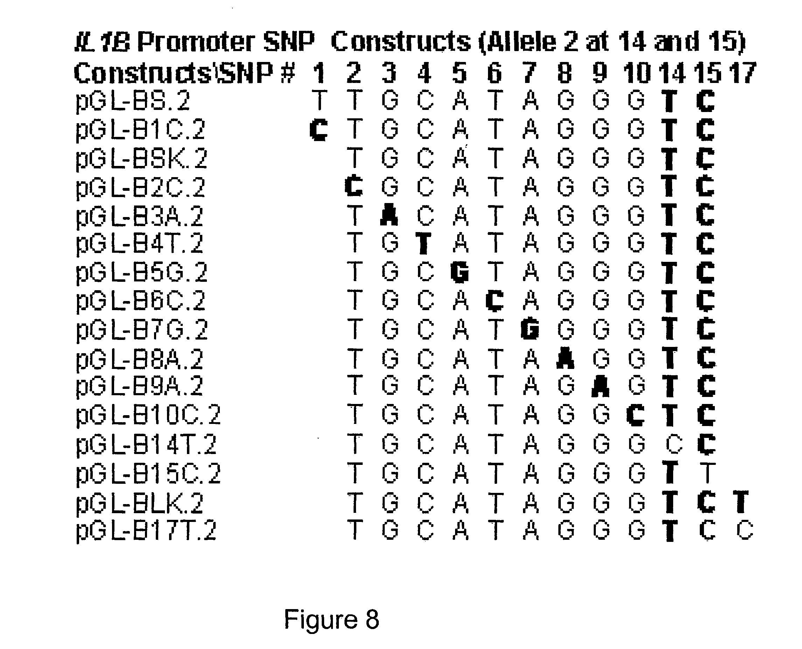

FIG. 8 shows plasmid constructs of SNPs within the IL-1B promoter region in which SNPs 14 and 15 have been mostly held constant at allele 2. The parental construct, where all SNPs except 14 and 15 are allele 1 is identified as pGL-BS.2. Below are constructs where individual SNP has been converted to allele 2 by site directed mutagenesis. Allele 2 SNPs are highlighted.

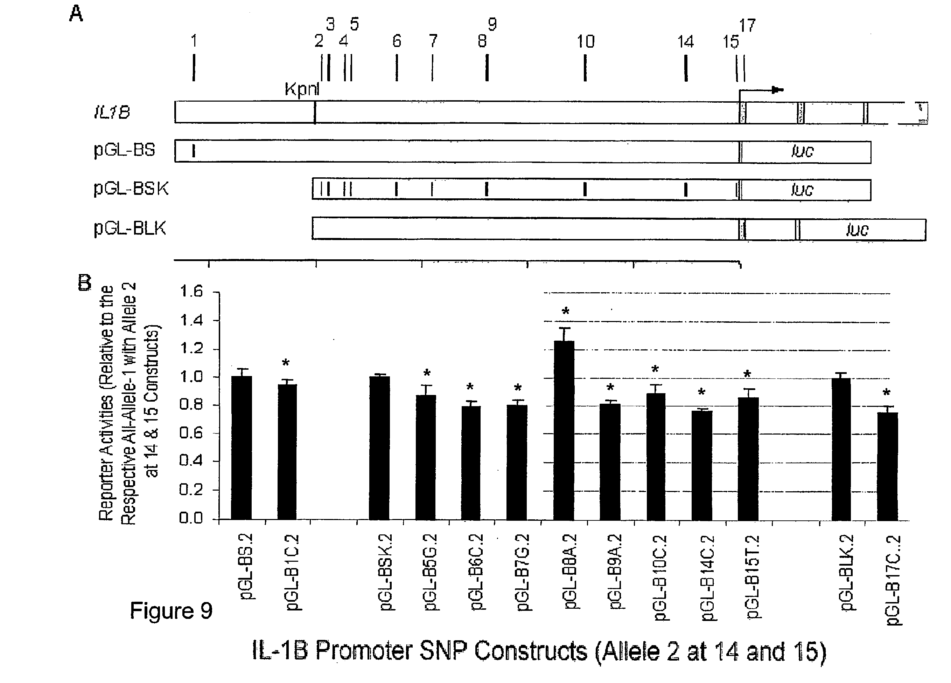

FIG. 9A-B show location of SNPs and promoter constructs. In FIG. 9A, the SNP positions are indicated within the IL-1B gene promoter region on the top line. The lines below represent the lengths of the promoter region used in the different reporter (luciferase) constructs, the relative transcriptional activity results of which are shown in FIG. 9B. The Kpn I and Bam HI sites are indicated. In the IL-1BS series, the SNPs are indicated by vertical lines.

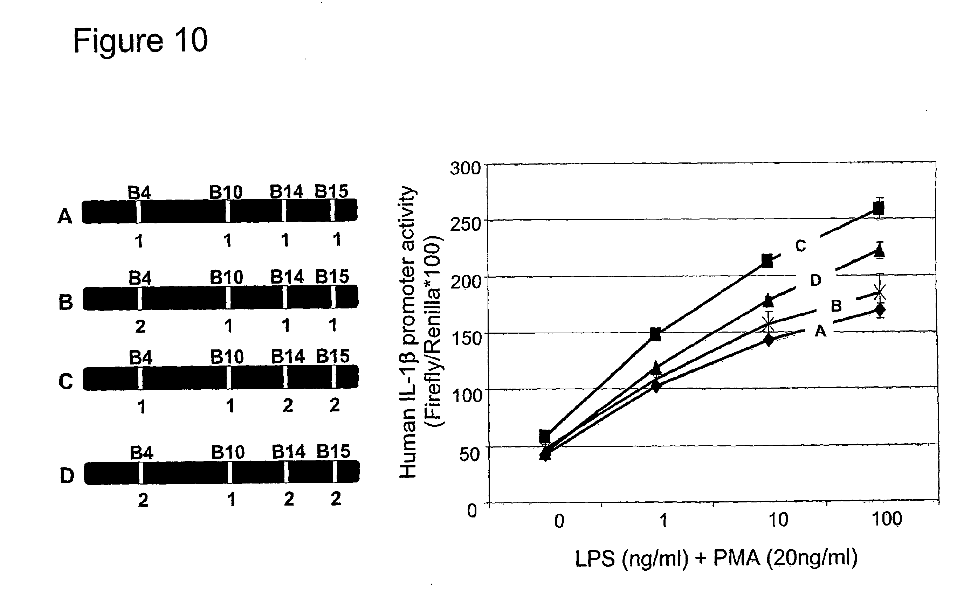

FIG. 10. is a chart demonstrating the transfection analysis of SNP 4 on differing allelic patterns of SNPs 14 and 15. The promoter activity of the construct alleles designated A-D as measured by Firefly luciferase activity are shown at different LPS doses (0, 1 10 and 100 ng/ml) and 20 ng/ml PMA. Firefly activity was normalized to *Renilla* activity to control for transfection efficiency. Each construct was transfected in triplicate and transfection for each construct was repeated at least three times.

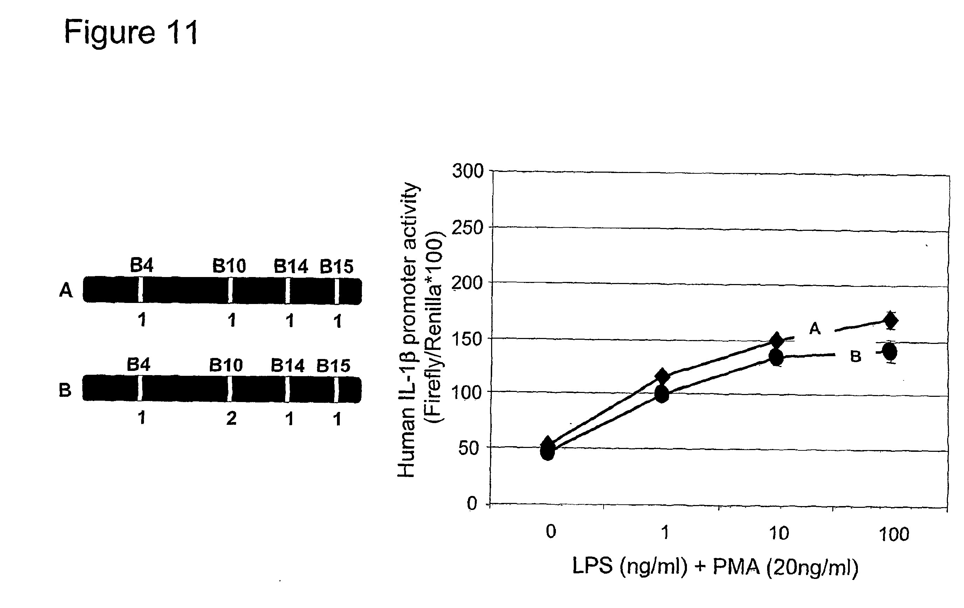

FIG. 11 is a chart demonstrating the transfection analysis of SNP 10. The promoter activity of constructs A (diamond) and B (circle) as measured by Firefly luciferase activity are shown at different LPS doses (0, 1 10 and 100 ng/ml) and 20 ng/ml PMA. Firefly activity was normalized to *Renilla* activity to control for transfection efficiency. Each construct was transfected in triplicate and transfection for each construct was repeated at least three times.

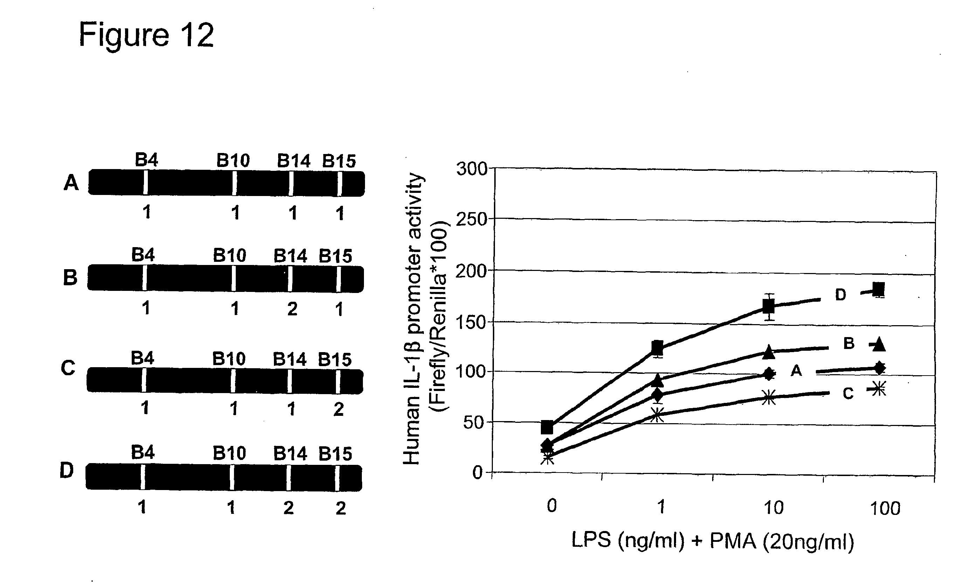

FIG. 12 is a chart demonstrating the transfection analysis of SNPs 14 and 15. The promoter activity of constructs with allele patterns represented as A-Das measured by Firefly luciferase activity are shown at different LPS doses (0, 1 10 and 100 ng/ml) and 20 ng/ml PMA. Firefly activity was normalized to *Renilla* activity to control for transfection efficiency. Each construct was transfected in triplicate and transfection for each construct was repeated at least three times.

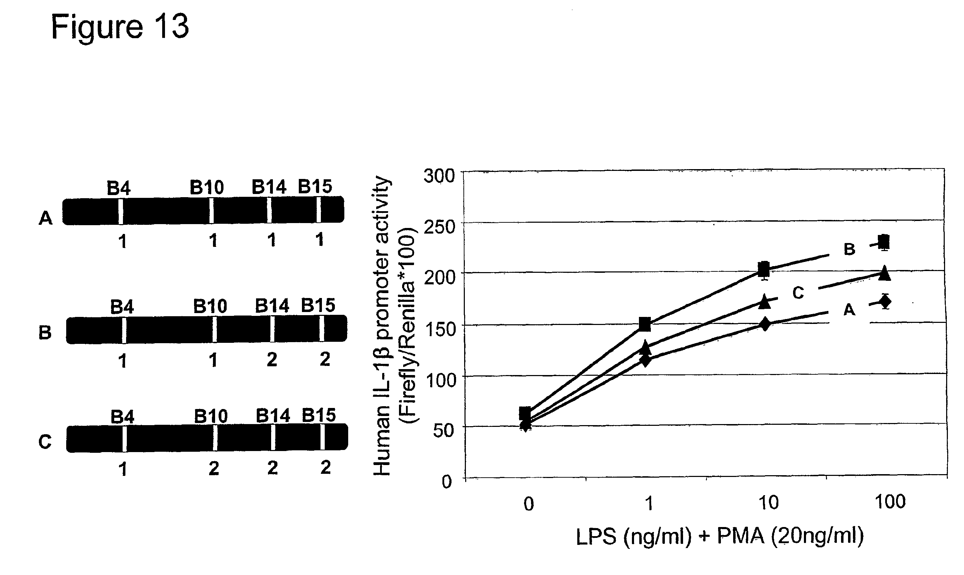

FIG. 13 is a chart demonstrating the transfection analysis of promoter haplotype comprising SNPS 10, 14 and 15. The allelic combinations are shown for plasmids A-C. The promoter activity of constructs C (diamond), B (square) and A (triangle) as measured by Firefly luciferase activity are shown at different LPS doses (0, 1 10 and 100 ng/ml) and 20 ng/ml PMA. Firefly activity was normalized to *Renilla* activity to control for transfection efficiency. Each construct was transfected in triplicate and transfection for each construct was repeated at least three times.

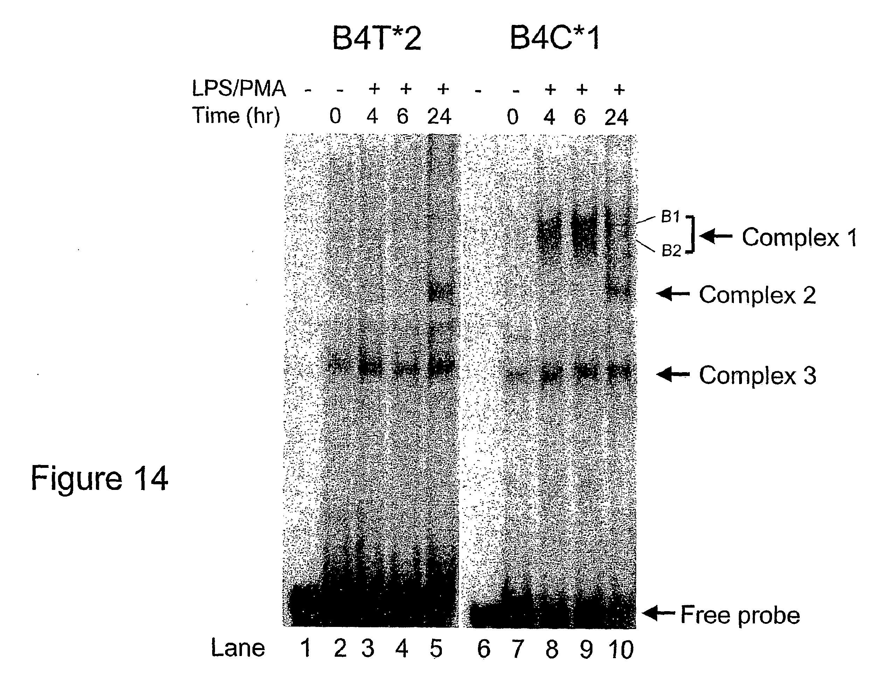

FIG. 14 is a photographic image of the results of an electromobility shift assay of SNP 4.
Labeled, double stranded oligomers -both allele 1 and 2, sequences of SNP 4 used for the EMSA assay are shown. Nuclear protein isolated from THP-1 cells treated with 0. 2, 4, 6 and 24 hours of LPS (100 ng/ml) and PMA (20 ng/ml) were used. Location of the free probe and retarded complexes are indicated by arrows.
FIG. 15 is a photographic image of the results of an electromobility shift assay of SNP 4 using unlabeled probes to compete out specific binding. Labeled, double stranded oligomers -both allele 1 and 2, sequences of SNP 4 used for the EMSA assay are shown. To determine specificity of binding, unlabeled double standed oligomers for allele 1 and 2 were used in the EMSA assay to compete for binding to the labeled probes.
FIG. 16 is a photographic image of the results of NF-kB antibody supershift assays for SNP 4. NF-kB antibody for both p50 and p65 subunits were used in the EMSA assay to determine the DNA-protein complexes formed were due to NF-kB proteins.
FIG. 17 is a photographic image of the results of an electromobility shift assay of SNP 10.
Labeled, double stranded oligomers -both allele 1 and 2, sequences of SNP 10 used for the EMSA assay are shown. Nuclear protein isolated from THP-1 cells treated with 0. 2, 4, 6 and 24 hours of LPS (100 ng/ml) and PMA (20 ng/ml) were used. Location of the free probe and retarded complexes are indicated by arrows.

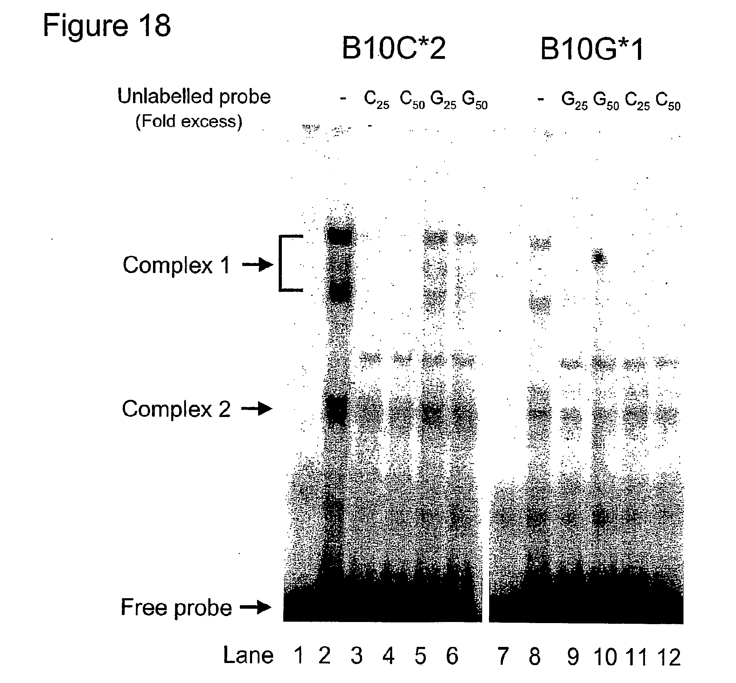

FIG. 18 is a photographic image of the results of an electromobility shift assay of SNP 10.
showing the specificity of complex formation for SNP 10. Labeled, double stranded oligomers -both allele 1 and 2, sequences of SNP 10 used for the EMSA assay are shown.
To determine specificity of binding, unlabeled double standed oligomers for allele 1 and 2 were used in the EMSA assay to compete for binding to the labeled probes.

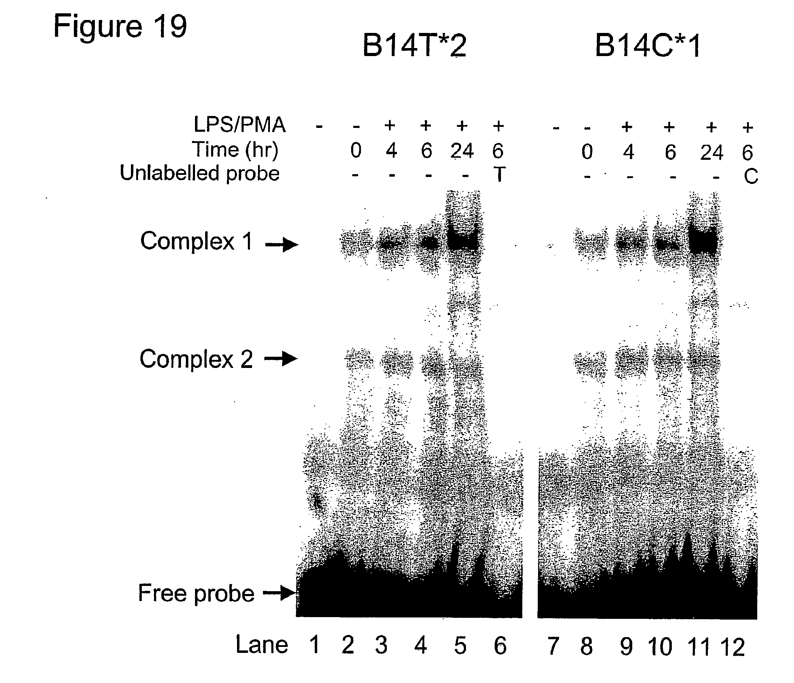

FIG. 19 is a photographic image of the results of an electromobility shift assay of SNP 14.
Labeled, double stranded oligomers -both allele 1 and 2, sequences of SNP 14 used for the EMSA assay are shown. Nuclear protein isolated from THP-1 cells treated with 0. 2, 4, 6 and 24 hours of LPS (100 ng/ml) and PMA (20 ng/ml) were used. Location of the free probe and retarded complexes are indicated by arrows.

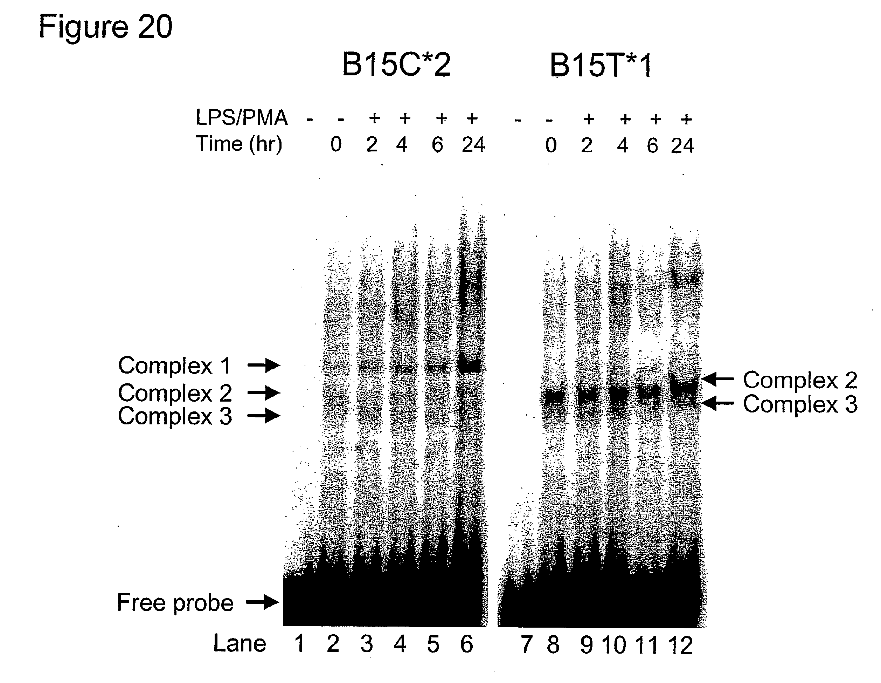

FIG. 20 is a photographic image of the results of an electromobility shift assay of SNP 15.
Labeled, double stranded oligomers -both allele 1 and 2, sequences of SNP 15 used for the EMSA assay are shown. Nuclear protein isolated from THP-1 cells treated with 0. 2, 4, 6 and 24 hours of LPS (100 ng/ml) and PMA (20 ng/ml) were used. Location of the free probe and retarded complexes are indicated by arrows.

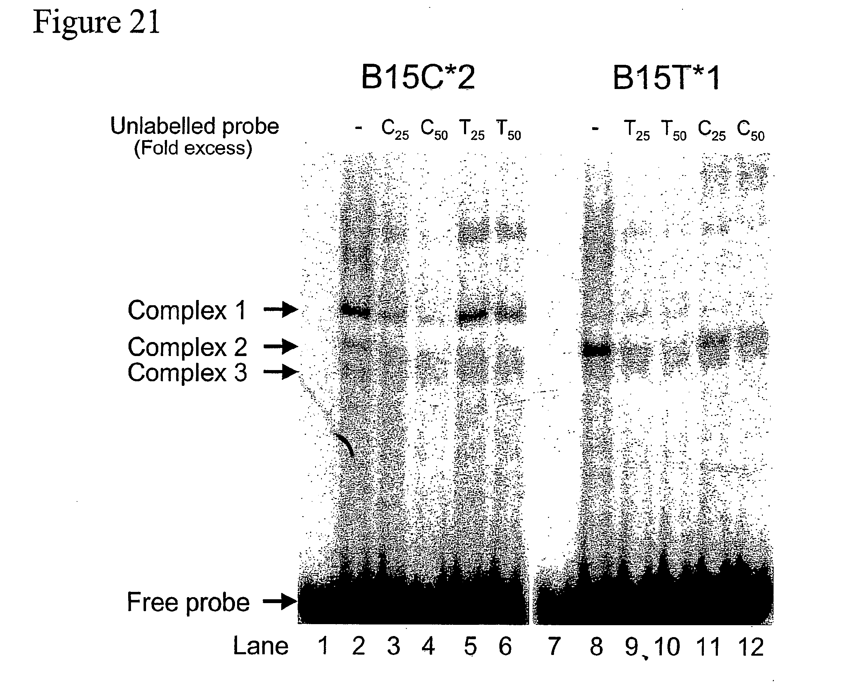

FIG. 21 is a photographic image of the results of an electromobility shift assay of SNP 15 showing the specificity of complex formation for SNP 15. Labeled, double stranded oligomers -both allele 1 and 2, sequences of SNP 15 used for the EMSA assay are shown.
To determine specificity of binding, unlabeled double standed oligomers for allele 1 and 2 were used in the EMSA assay to compete for binding to the labeled probes.

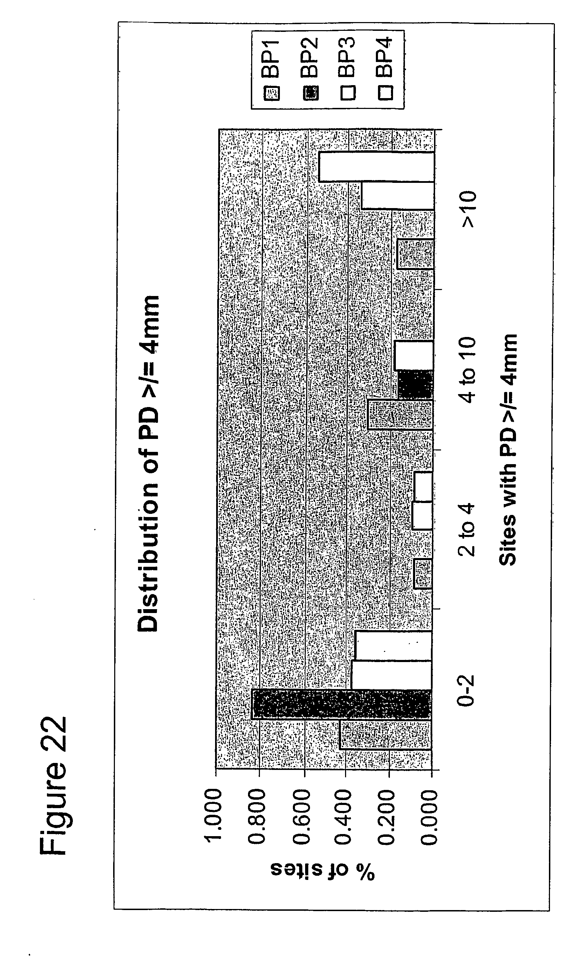

FIG. 22 is a chart demonstrating the severity of periodontitis in subjects by measuring pocket depth in the subject's gum pockets.

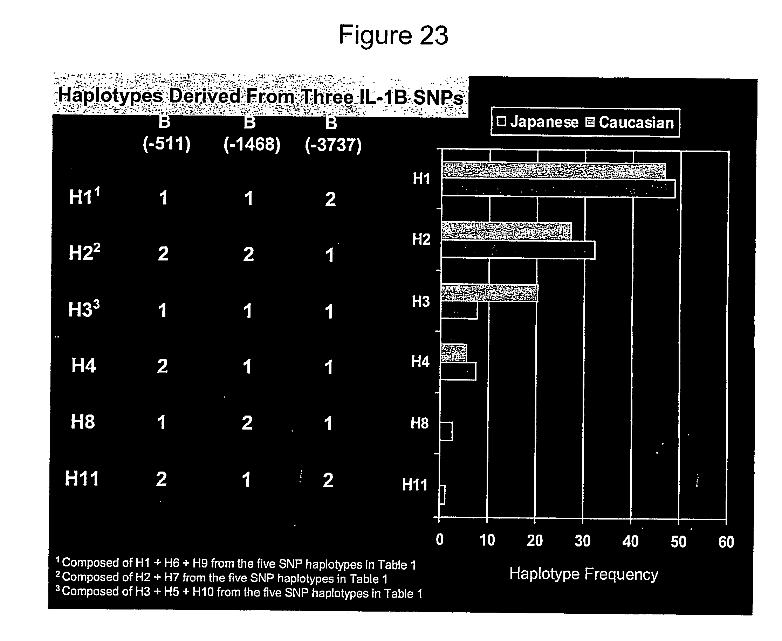

FIG. 23 is a chart demonstrating the frequency of IL-1B haplotypes in Japanese and Caucasian subjects.

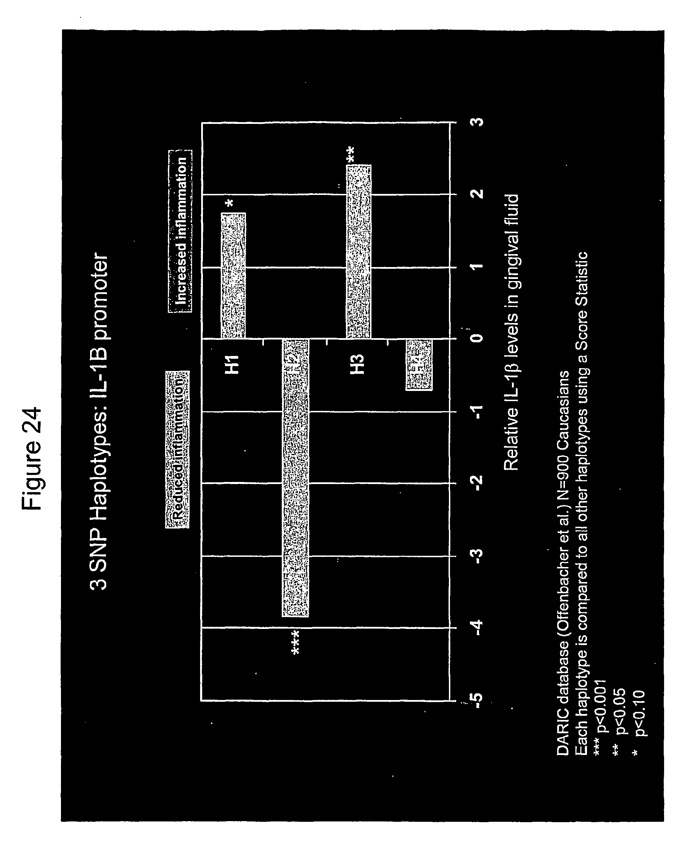

FIG. 24 is a chart demonstrating the relative amounts of IL-1β in gingival fluids of Caucasian subjects having specific IL-1B haplotypes.

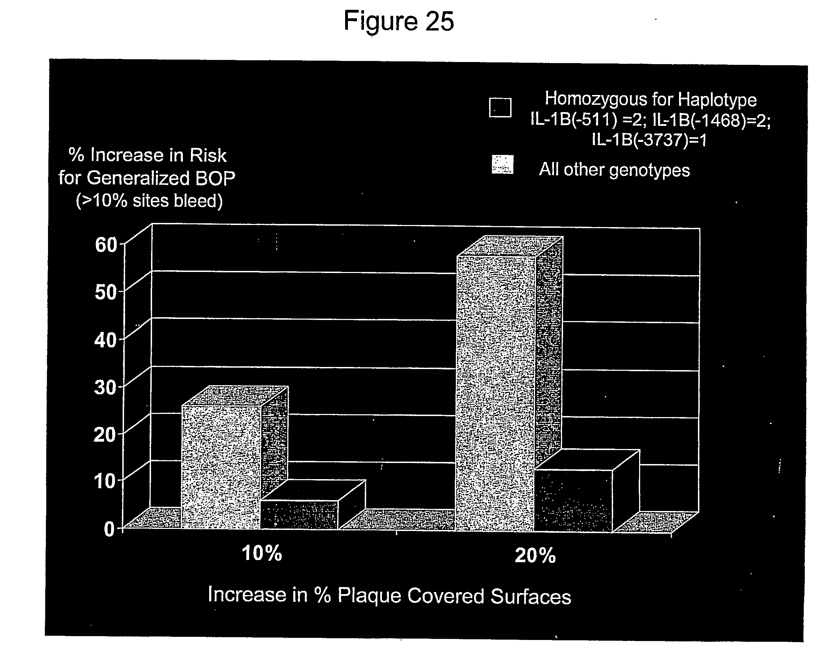

FIG. 25 is a chart demonstrating the reduced risk of periodontitis in Japanese subjects having a specific IL-1B genotype.

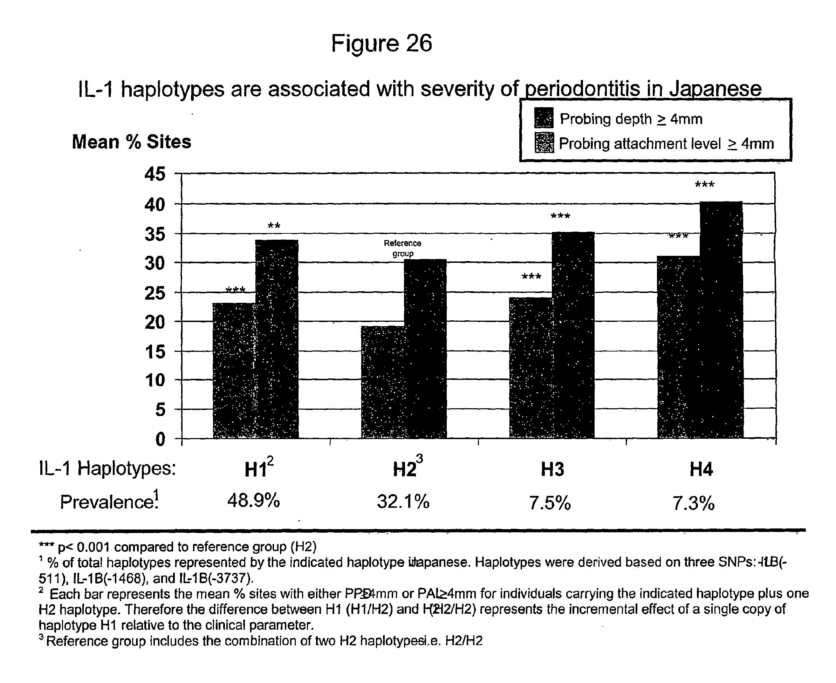

FIG. 26 is a chart demonstrating the association of IL-1B haplotypes and severity of periodontitis in Japanese subjects.

### 4. Detailed Description of the Invention

### 4.1 Definitions

For convenience, the meaning of certain terms and phrases employed in the specification, examples, and appended claims is provided below.

The term "allele" refers to the different sequence variants found at different polymorphic regions. For example, IL-1RN (VNTR) has at least five different alleles. The sequence variants may be single or multiple base changes, including without limitation insertions, deletions, or substitutions, or may be a variable number of sequence repeats.

The term "allelic pattern" refers to the identity of an allele or alleles at one or more polymorphic regions. For example, an allelic pattern may consist of a single allele at a polymorphic site, as for IL-1RN (VNTR) allele 1, which is an allelic pattern having at least one copy of IL-1RN allele 1 at the VNTR of the IL-1RN gene loci. Alternatively, an allelic pattern may consist of either a homozygous or heterozygous state at a single polymorphic site. For example, IL1-RN (VNTR) allele 2,2 is an allelic pattern in which there are two copies of the second allele at the VNTR marker of IL-1RN that corresponds to the homozygous IL-RN (VNTR) allele 2 state. Alternatively, an allelic pattern may consist of the identity of alleles at more than one polymorphic site.

The term "antibody" as used herein is intended to refer to a binding agent including a whole antibody or a binding fragment thereof which is specifically reactive with an IL-1 polypeptide. Antibodies can be fragmented using conventional techniques and the fragments screened for utility in the same manner as described above for whole antibodies. For example, F(ab)2 fragments can be generated by treating an antibody with pepsin. The resulting F(ab)2 fragment can be treated to reduce disulfide bridges to produce Fab fragments. The antibody of the present invention is further intended to include bispecific, single-chain, and chimeric and humanized molecules having affinity for an IL-1B polypeptide conferred by at least one CDR region of the antibody.

"Biological activity" or "bioactivity" or "activity" or "biological function", which are used interchangeably, for the purposes herein means an effector or antigenic function that is directly or indirectly performed by an IL-1 polypeptide (whether in its native or denatured conformation), or by any subsequence thereof. Biological activities include binding to a target peptide, e.g., an IL-1 receptor. Biological activities also include transcription of an IL-1 gene, such as IL-1B. An IL-1 bioactivity can be modulated by directly affecting an IL-1 polypeptide. Alternatively, an IL-1 bioactivity can be modulated by modulating the level of an IL-1 polypeptide, such as by modulating expression of an IL-1 gene.

As used herein the term "bioactive fragment of an IL-1 polypeptide" refers to a fragment of a full-length IL-1 polypeptide, wherein the fragment specifically mimics or antagonizes the activity of a wild-type IL-1 polypeptide. The bioactive fragment preferably is a fragment capable of interacting with an interleukin receptor.

The term "an aberrant activity", as applied to an activity of a polypeptide such as IL-1, refers to an activity which differs from the activity of the wild-type or native polypeptide or which differs from the activity of the polypeptide in a healthy subject. An activity of a polypeptide can be aberrant because it is stronger than the activity of its native counterpart. Alternatively, an activity can be aberrant because it is weaker or absent relative to the activity of its native counterpart. An aberrant activity can also be a change in an activity.

For example an aberrant polypeptide can interact with a different target peptide. A cell can have an aberrant IL-1 activity due to overexpression or underexpression of an IL-1 locus gene encoding an IL-1 locus polypeptide.

"Cells", "host cells" or "recombinant host cells" are terms used interchangeably herein to refer not only to the particular subject cell, but to the progeny or potential progeny of such a cell. Because certain modifications may occur in succeeding generations due to either mutation or environmental influences, such progeny may not, in fact be identical to the parent cell, but are still included within the scope of the term as used herein.

A "chimera," "mosaic," "chimeric mammal" and the like, refers to a transgenic non-human mammal with a knock-out or knock-in construct in at least some of its genome-containing cells.

The term "animal" as used herein never encompas human.

The terms "control" or "control sample" refer to any sample appropriate to the detection technique employed. The control sample may contain the products of the allele detection technique employed or the material to be tested. Further, the controls may be positive or negative controls. By way of example, where the allele detection technique is PCR amplification, followed by size fractionation, the control sample may comprise DNA fragments of an appropriate size. Likewise, where the allele detection technique involves detection of a mutated protein, the control sample may comprise a sample of a mutant protein. However, it is preferred that the control sample comprises the material to be tested. For example, the controls may be a sample of genomic DNA or a cloned portion of the IL-1 gene cluster. However, where the sample to be tested is genomic DNA, the control sample is preferably a highly purified sample of genomic DNA.

The phrase "diseases and conditions associated with IL-1 polymorphisms" refers to a variety of diseases or conditions, the susceptibility to which can be indicated in a subject based on the identification of one or more alleles within the IL-1 complex. Examples include: inflammatory or degenerative disease, including: Systemic Inflammatory Response (SIRS); Alzheimer's Disease (and associated conditions and symptoms including: chronic neuroinflammation, glial activation; increased microglia; neuritic plaque formation; and response to therapy); Amylotropic Lateral Sclerosis (ALS), arthritis (and associated conditions and symptoms including: acute joint inflammation, antigen-induced arthritis, arthritis associated with chronic lymphocytic thyroiditis, collagen-induced arthitis, juvenile chronic arthritis; juvenile rheumatoid arthritis, osteoarthritis, prognosis and streptococcus-induced arthritis), asthma (and associated conditions and symptoms, including: bronchial asthma; chronic obstructive airway disease; chronic obstructive pulmonary disease, juvenile asthma and occupational asthma); cardiovascular diseases (and associated conditions and symptoms, including atherosclerosis; autoimmune myocarditis, chronic cardiac hypoxia, congestive heart failure, coronary artery disease, cardiomyopathy and cardiac cell dysfunction, including: aortic smooth muscle cell activation; cardiac cell apoptosis; and immunomodulation of cardiac cell function; diabetes and associated conditions and symptoms, including autoimmune diabetes, insulin-dependent (Type 1) diabetes, diabetic periodontitis, diabetic retinopathy, and diabetic nephropathy); gastrointestinal inflammations (and related conditions and symptoms, including celiac disease, associated osteopenia, chronic colitis, Crohn's disease, inflammatory bowel disease and ulcerative colitis); gastric ulcers; hepatic inflammations, cholesterol gallstones and hepatic fibrosis, HIV infection (and associated conditions and symptoms, including degenerative responses, neurodegenerative responses, and HIV associated Hodgkin's Disease), Kawasaki's Syndrome (and associated diseases and conditions, including mucocutaneous lymph node syndrome, cervical lymphadenopathy, coronary artery lesions, edema, fever, increased leukocytes, mild anemia, skin peeling, rash, conjunctiva redness, thrombocytosis; multiple sclerosis, nephropathies (and associated diseases and conditions, including diabetic nephropathy, endstage renal disease, glomerulonephritis, Goodpasture's syndrome, hemodialysis survival and renal ischemic reperfusion injury), neurodegenerative diseases (and associated diseases and conditions, including acute neurodegeneration, induction of IL-1 in aging and neurodegenerative disease, IL-1 induced plasticity of hypothalamic neurons and chronic stress hyperresponsiveness), Qphthalmopathies (and associated diseases and conditions, including diabetic retinopathy, Graves' Ophthalmopathy, and uveitis, osteoporosis (and associated diseases and conditions, including alveolar, femoral, radial, vertebral or wrist bone loss or fracture incidence, postmenopausal bone loss, mass, fracture incidence or rate of bone loss), otitis media (adult or pediatric), pancreatis or pancreatic acinitis, periodontitis or periodontal disease (and associated diseases and conditions, including adult, early onset and diabetic); pulmonary diseases, including chronic lung disease, chronic sinusitis, hyaline membrane disease, hypoxia and pulmonary disease in SIDS; restenosis; rheumatism including rheumatoid arthritis , rheumatic aschoff bodies, rheumatic diseases and rheumatic myocarditis; thyroiditis including chronic lymphocytic thyroiditis;urinary tract infections including chronic prostatitis, chronic pelvic pain syndrome and urolithiasis. Immunological disorders, including autoimmune diseases, such as alopecia aerata, autoimmune myocarditis, Graves' disease, Graves ophthalmopathy, lichen sclerosis, multiple sclerosis, psoriasis, systemic lupus erythematosus, systemic sclerosis, thyroid diseases (e.g.goiter and struma lymphomatosa (Hashimoto's thyroiditis, lymphadenoid goiter), sleep disorders and chronic fatigue syndrome and obesity (non-diabetic or associated with diabetes). Resistance to infectious diseases, such as Leishmaniasis, Leprosy, Lyme Disease, Lyme Carditis, malaria, cerebral malaria, meningititis, tubulointestitial nephritis associated with malaria), which are caused by bacteria, viruses (e.g. cytomegalovirus, encephalitis, Epstein-Barr Virus, Human Immunodeficiency Virus, Influenza Virus) or protozoans (e.g., Plasmodium falciparum, trypanosomes). Response to trauma, including cerebral trauma (including strokes and ischemias, encephalitis, encephalopathies, epilepsy, perinatal brain injury, prolonged febrile seizures, SIDS and subarachnoid hemorrhage), low birth weight (e.g. cerebral palsy), lung injury (acute hemorrhagic lung injury, Goodpasture's syndrome, acute ischemic reperfusion), myocardial dysfunction, caused by occupational and environmental pollutants (e.g. susceptibility to toxic oil syndrome silicosis), radiation trauma, and efficiency of wound healing responses (e.g. bum or thermal wounds, chronic wounds, surgical wounds and spinal cord injuries). Susceptibility to neoplasias, including breast cancer associated osteolytic metastasis, cachexia, colorectal cancer, hyperproliferative diseases, Hodgkin's disease, leukemias, lymphomas, metabolic diseases and tumors, metastases, myeolomas, and various cancers (including breast prostate ovarian, colon, lung, etc), anorexia and cachexia. Hormonal regulation including fertility/fecundity, likelihood of a pregnancy, incidence of preterm labor, prenatal and neonatal complications including preterm low birth weight, cerebral palsy, septicemia, hypothyroxinernia, oxygen dependence, cranial abnormality, early onset menopause. A subject's response to transplant (rejection or acceptance), acute phase response (e.g. febrile response), general inflammatory response, acute respiratory distress response, acute systemic inflammatory response, wound healing, adhesion, immunoinflammatory response, neuroendocrine response, fever development and resistance, acute-phase response, stress response, disease susceptibility, repetitive motion stress, tennis elbow, and pain management and response.

The phrases "disruption of the gene" and "targeted disruption" or any similar phrase refers to the site specific interruption of a native DNA sequence so as to prevent expression of that gene in the cell as compared to the wild-type copy of the gene. The interruption may be caused by deletions, insertions or modifications to the gene, or any combination thereof.

The term "haplotype" as used herein is intended to refer to a set of alleles that are inherited together as a group (are in linkage disequilibrium) at statistically significant levels (p_{corr} < 0.05). As used herein, the phrase "an IL-1 haplotype" refers to a haplotype in the IL-1 loci. An IL-1 inflammatory or proinflammatory haplotype refers to a haplotype that is indicative of increased agonist and/or decreased antagonist activities. An IL-1 inflammatory or proinflammatory haplotype also refers to a haplotype that is indicative of altered IL-1 gene expression, such as increased expression of IL-1B.

The terms "IL-1 gene cluster" and "IL-1 loci" as used herein include all the nucleic acid at or near the 2q13 region of chromosome 2, including at least the IL-1A, IL-1B and IL-1RN genes and any other linked sequences. (Nicklin et al., Genomics 19: 382-84, 1994). The terms "IL-1A", "IL-1B", and "IL-1RN" as used herein refer to the genes coding for IL-1 , IL-1 , and IL-1 receptor antagonist, respectively. The gene accession number for IL-1A, IL-1B, and IL-1RN are X03833, X04500, and X64532, respectively.

"IL-1 functional mutation" refers to a mutation within the IL-1 gene cluster that results in an altered phenotype (i.e. effects the function of an IL-1 gene or protein). Examples include: IL-1A(+4845) allele 2, IL-1B (+3954) allele 2, IL-1B (+6912) allele 2, IL-1B (+3737) allele 1, IL-1B (-1468) allele 1; and IL-1RN (+2018) allele 2.

"IL-1X (Z) allele Y " refers to a particular allelic form, designated Y, occurring at an IL-1 locus polymorphic site in gene X, wherein X is IL-1A, B, or RN and positioned at or near nucleotide Z, wherein nucleotide Z is numbered relative to the major transcriptional start site, which is nucleotide +1, of the particular IL-1 gene X. As further used herein, the term "IL-1X allele (Z)" refers to all alleles of an IL-1 polymorphic site in gene X positioned at or near nucleotide Z. For example, the term "IL-1RN (+2018) allele" refers to alternative forms of the IL-1RN gene at marker +2018. "IL-1RN (+2018) allele 1" refers to a form of the IL-1RN gene which contains a cytosine (C) at position +2018 of the sense strand. Clay et al., Hum. Genet. 97:723-26, 1996. "IL-1RN (+2018) allele 2" refers to a form of the IL-1RN gene which contains a thymine (T) at position +2018 of the plus strand. When a subject has two identical IL-1RN alleles, the subject is said to be homozygous, or to have the homozygous state. When a subject has two different IL-1RN alleles, the subject is said to be heterozygous, or to have the heterozygous state. The term "IL-1RN (+2018) allele 2,2" refers to the homozygous IL-1 RN (+2018) allele 2 state. Conversely, the term "IL-1RN (+2018) allele 1,1" refers to the homozygous IL-1 RN (+2018) allele 1 state. The term "IL-1RN (+2018) allele 1,2" refers to the heterozygous allele 1 and 2 state.

"IL-1 related" as used herein is meant to include all genes related to the human IL-1 locus genes on human chromosome 2 (2q 12-14). These include IL-1 genes of the human IL-1 gene cluster located at chromosome 2 (2q 13-14) which include: the IL-1A gene which encodes interleukin-1α, the IL-1B gene which encodes interleukin-1β, and the IL-1RN (or IL-1ra) gene which encodes the interleukin-1 receptor antagonist. Furthermore these IL-1 related genes include the type I and type II human IL-1 receptor genes located on human chromosome 2 (2q12) and their mouse homologs located on mouse chromosome 1 at position 19.5 cM. Interleukin-1α, interleukin-1β, and interleukin-1RN are related in so much as they all bind to IL-1 type I receptors, however only interleukin-1α and interleukin-1β are agonist ligands which activate IL-1 type I receptors, while interleukin-1RN is a naturally occurring antagonist ligand. Where the term "IL-1" is used in reference to a gene product or polypeptide, it is meant to refer to all gene products encoded by the interleukin-1 locus on human chromosome 2 (2q 12-14) and their corresponding homologs from other species or functional variants thereof. The term IL-1 thus includes secreted polypeptides which promote an inflammatory response, such as IL-1α and IL-1β, as well as a secreted polypeptide which antagonize inflammatory responses, such as IL-1 receptor antagonist and the IL-1 type II (decoy) receptor.

An "IL-1 receptor" or "IL-1R" refers to various cell membrane bound protein receptors capable of binding to and/or transducing a signal from an IL-1 locus-encoded ligand. The term applies to any of the proteins which are capable of binding interleukin-1 (IL-1) molecules and, in their native configuration as mammalian plasma membrane proteins, presumably play a role in transducing the signal provided by IL-1 to a cell. As used herein, the term includes analogs of native proteins with IL-1-binding or signal transducing activity. Examples include the human and murine IL-1 receptors described in U.S. Patent No. 4,968,607. The term "IL-1 nucleic acid" refers to a nucleic acid encoding an IL-1 protein.

An "IL-1 polypeptide" and "IL-1 protein" are intended to encompass polypeptides comprising the amino acid sequence encoded by the IL-1 genomic DNA sequences shown in Figures 1, 2, and 3, or fragments thereof, and homologs thereof and include agonist and antagonist polypeptides.

"Increased risk" refers to a statistically higher frequency of occurrence of the disease or condition in an individual carrying a particular polymorphic allele in comparison to the frequency of occurrence of the disease or condition in a member of a population that does not carry the particular polymorphic allele.

The term "interact" as used herein is meant to include detectable relationships or associations (e.g. biochemical interactions) between molecules, such as interactions between protein-protein, protein-nucleic acid, nucleic acid-nucleic acid and protein-small molecule or nucleic acid-small molecule in nature.

The term "isolated" as used herein with respect to nucleic acids, such as DNA or RNA, refers to molecules separated from other DNAs, or RNAs, respectively, that are present in the natural source of the macromolecule. For example, an isolated nucleic acid encoding one of the subject IL-1 polypeptides preferably includes no more than 10 kilobases (kb) of nucleic acid sequence which naturally immediately flanks the IL-1 gene in genomic DNA, more preferably no more than 5kb of such naturally occurring flanking sequences, and most preferably less than 1.5kb of such naturally occurring flanking sequence. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

A "knock-in" transgenic animal refers to a non-human animal that has had a modified gene introduced into its genome and the modified gene can be of exogenous or endogenous origin.

A "knock-out" transgenic animal refers to a non-human animal in which there is partial or complete suppression of the expression of an endogenous gene (e.g, based on deletion of at least a portion of the gene, replacement of at least a portion of the gene with a second sequence, introduction of stop codons, the mutation of bases encoding critical amino acids, or the removal of an intron junction, etc.).

A "knock-out construct" refers to a nucleic acid sequence that can be used to decrease or suppress expression of a protein encoded by endogenous DNA sequences in a cell. In a simple example, the knock-out construct is comprised of a gene, such as the IL-1RN gene, with a deletion in a critical portion of the gene, so that active protein cannot be expressed therefrom. Alternatively, a number of termination codons can be added to the native gene to cause early termination of the protein or an intron junction can be inactivated. In a typical knock-out construct, some portion of the gene is replaced with a selectable marker (such as the neo gene) so that the gene can be represented as follows: IL-1RN 5'/neo/IL-1RN 3', where IL-1RN5' and IL-1RN 3', refer to genomic or cDNA sequences which are, respectively, upstream and downstream relative to a portion of the IL-1RN gene and where neo refers to a neomycin resistance gene. In another knock-out construct, a second selectable marker is added in a flanking position so that the gene can be represented as: IL-1RN/neo/IL-1RN/TK, where TK is a thymidine kinase gene which can be added to either the IL-1RN5' or the IL-1RN3' sequence of the preceding construct and which further can be selected against (i.e. is a negative selectable marker) in appropriate media. This two-marker construct allows the selection of homologous recombination events, which removes the flanking TK marker, from non-homologous recombination events which typically retain the TK sequences. The gene deletion and/or replacement can be from the exons, introns, especially intron junctions, and/or the regulatory regions such as promoters.

"Linkage disequilibrium" refers to co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in "linkage disequilibrium". The cause of linkage disequilibrium is often unclear. It can be due to selection for certain allele combinations or to recent admixture of genetically heterogeneous populations. In addition, in the case of markers that are very tightly linked to a disease gene, an association of an allele (or group of linked alleles) with the disease gene is expected if the disease mutation occurred in the recent past, so that sufficient time has not elapsed for equilibrium to be achieved through recombination events in the specific chromosomal region. When referring to allelic patterns that are comprised of more than one allele, a first allelic pattern is in linkage disequilibrium with a second allelic pattern if all the alleles that comprise the first allelic pattern are in linkage disequilibrium with at least one of the alleles of the second allelic pattern. An example of linkage disequilibrium is that which occurs between the alleles at the IL-1RN (+2018) and IL-1RN (VNTR) polymorphic sites. The two alleles at IL-1RN (+2018) are 100% in linkage disequilibrium with the two most frequent alleles of IL-1RN (VNTR), which are allele 1 and allele 2.

The term "marker" refers to a sequence in the genome that is known to vary among individuals. For example, the IL-1RN gene has a marker that consists of a variable number of tandem repeats (VNTR).

A "mutated gene" or "mutation" or "functional mutation" refers to an allelic form of a gene, which is capable of altering the phenotype of a subject having the mutated gene relative to a subject which does not have the mutated gene. The altered phenotype caused by a mutation can be corrected or compensated for by certain agents. If a subject must be homozygous for this mutation to have an altered phenotype, the mutation is said to be recessive. If one copy of the mutated gene is sufficient to alter the phenotype of the subject, the mutation is said to be dominant. If a subject has one copy of the mutated gene and has a phenotype that is intermediate between that of a homozygous and that of a heterozygous subject (for that gene), the mutation is said to be co-dominant.

A "non-human animal" of the invention includes mammals such as rodents, non-human primates, sheep, dogs, cows, goats, etc. amphibians, such as members of the *Xenopus* genus, and transgenic avians (e.g. chickens, birds, etc.). The term "chimeric animal" is used herein to refer to non-human animals in which the recombinant gene is found, or in which the recombinant gene is expressed in some but not all cells of the animal. The term "tissue-specific chimeric animal" indicates that one of the recombinant IL-1 genes is present and/or expressed or disrupted in some tissues but not others. The term "mammal" refers to any member of the class Mammalia, except for humans.

As used herein, the term "nucleic acid" refers to polynucleotides or oligonucleotides such as deoxyribonucleic acid (DNA), and, where appropriate, ribonucleic acid (RNA). The term should also be understood to include, as equivalents, analogs of either RNA or DNA made from nucleotide analogs (e.g. peptide nucleic acids) and as applicable to the embodiment being described, single (sense or antisense) and double-stranded polynucleotides.

The terms "periodontitis" and "periodontal disease" refer to a dental disorder that results from progression of gingivitis, involving inflammation and infection of the tissue, including ligaments and bones, that support the teeth. Periodontitis is often diagnosed upon examination, which shows soft, swollen, red-purple gingiva. Deposits of plaque and calculus may be visible at the base of the teeth, with enlarged pockets in the gums. The gums are usually painless or mildly tender, unless a tooth abscess is also present. Teeth may be loose and gums may be receded. Severity of periodontitis is quantified by measuring pocket depth (PD, also termed "probing depth") in a subject's gum pockets.

The term "polymorphism" refers to the coexistence of more than one form of a gene or portion (e.g., allelic variant) thereof. A portion of a gene of which there are at least two different forms, i.e., two different nucleotide sequences, is referred to as a "polymorphic region of a gene". A specific genetic sequence at a polymorphic region of a gene is an allele. A polymorphic region can be a single nucleotide, the identity of which differs in different alleles. A polymorphic region can also be several nucleotides long.

The term "propensity to disease," also "predisposition" or "susceptibility" to disease or any similar phrase, means that certain alleles are hereby discovered to be associated with or predictive of a subject's incidence of developing a particular disease (e.g. a vascular disease). The alleles are thus over-represented in frequency in individuals with disease as compared to healthy individuals. Thus, these alleles can be used to predict disease even in pre-symptomatic or pre-diseased individuals.

"Small molecule" as used herein, is meant to refer to a composition, which has a molecular weight of less than about 5kD and most preferably less than about 4kD. Small molecules can be nucleic acids, peptides, peptidomimetics, carbohydrates, lipids or other organic or inorganic molecules.

As used herein, the term "specifically hybridizes" or "specifically detects" refers to the ability of a nucleic acid molecule to hybridize to at least approximately 6 consecutive nucleotides of a sample nucleic acid.

"Transcriptional regulatory sequence" is a generic term used throughout the specification to refer to DNA sequences, such as initiation signals, enhancers, and promoters, which induce or control transcription of protein coding sequences with which they are operably linked.

As used herein, the term "transgene" means a nucleic acid sequence (encoding, e.g., one of the IL-1 polypeptides, or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid.

A "transgenic animal" refers to any non-human mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or *in vitro* fertilization, but rather is directed to the introduction of a recombinant DNA molecule. This molecule may be integrated within a chromosome, or it may be extrachromosomally replicating DNA. In the typical transgenic animals described herein, the transgene causes cells to express a recombinant form of one of an IL-1 polypeptide, e.g. either agonistic or antagonistic forms. However, transgenic animals in which the recombinant gene is silent are also contemplated, as for example, the FLP or CRE recombinase dependent constructs described below. Moreover, "transgenic animal" also includes those recombinant non-human animals in which gene disruption of one or more genes is caused by human intervention, non-human including both recombination and antisense techniques. The term is intended to include all progeny generations. Thus, the founder animal and all F1, F2, F3, and so on, progeny thereof are included.

The term "treating" as used herein is intended to encompass curing as well as ameliorating at least one symptom of a condition or disease.

The term "vector" refers to a nucleic acid molecule, which is capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

### 4.2 Predictive Medicine

### 4.2.1. IL-1 Inflammatory Haplotypes and Their Association with Certain Diseases or Conditions.

The present invention is based at least in part, on the identification of certain inflammatory haplotype patterns and the association (to a statistically significant extent) of these patterns with the development of certain diseases or conditions. Therefore, detection of the alleles comprising a haplotype, alone or in conjunction with another means in a subject can indicate that the subject has or is predisposed to the development of a particular disease or condition. However, because these alleles are in linkage disequilibrium with other alleles, the detection of such other linked alleles can also indicate that the subject has or is predisposed to the development of a particular disease or condition. For example, the 44112332 haplotype comprises the following genotype:

| |
|---|
| allele 4 of the 222/223 marker of IL-1A |
| allele 4 of the gz5/gz6 marker of IL-1A |
| allele 1 of the -889 marker of IL-1A |
| allele 1 of the +3954 marker of IL-1B |
| allele 2 of the -511 marker of IL-1B |
| allele 3 of the gaat.p33330 marker |
| allele 3 of the Y31 marker |
| allele 2 of +2018 of IL-1RN |
| allele 1 of +4845 of IL-1A |
| allele 2 of the VNTR marker of IL-1RN |

Three other polymorphisms in an IL-1RN alternative exon (Exon lic, which produces an intracellular form of the gene product) are also in linkage disequilibrium with allele 2 of IL-1RN (VNTR) (Clay et al., (1996) Hum Genet 97:723-26). These include: IL-1RN exon lic (1812) (GenBank:X77090 at 1812); the IL-1RN exon lic (1868) polymorphism (GenBank:X77090 at 1868); and the IL-1RN exon lic (1887) polymorphism (GenBank:X77090 at 1887). Furthermore yet another polymorphism in the promoter for the alternatively spliced intracellular form of the gene, the Pic (1731) polymorphism (GenBank:X77090 at 1731), is also in linkage disequilibrium with allele 2 of the IL-1RN (VNTR) polymorphic locus. For each of these polymorphic loci, the allele 2 sequence variant has been determined to be in linkage disequilibrium with allele 2 of the IL-1RN (VNTR) locus (Clay et al., (1996) Hum Genet 97:723-26).

The 33221461 haplotype comprises the following genotype:

| |
|---|
| allele 3 of the 222/223 marker of IL-1A |
| allele 3 of the gz5/gz6 marker, of IL-1A |
| allele 2 of the -889 marker of IL-1A |
| allele 2 of the +3954 marker of IL-1B |
| allele 1 of the -511 marker of IL-1B |
| allele 4 of the gaat.p33330 marker |
| allele 6 of the Y31 marker |
| allele 1 of +2018 of IL-1RN |
| allele 2 of +4845 of IL-1A |
| allele 1 of the VNTR marker of IL-1RN |

Individuals with the 44112332 haplotype are typically overproducers of both IL-1α and IL-1β proteins, upon stimulation. In contrast, individuals with the 33221461 haplotype are typically underproducers of IL-1ra. Each haplotype results in a net proinflammatory response. Each allele within a haplotype may have an effect, as well as a composite genotype effect. In addition, particular diseases may be associated with both haplotype patterns.

The following Table 1 sets forth a number of genotype markers and various diseases and conditions to which these markers have been found to be associated to a statistically significant extent.

**TABLE 1**

| Associated Of IL-1 Haplothype Gene Markers With Certain Diseases | | | | | |
|---|---|---|---|---|---|
| GENOTYPE | IL-1A | IL-1A | IL-1B | IL-1B | IL-1RN |
| | (-889) | (+4845) | (-511) | (+3954) | (+2018) |
| DISEASE | | | | | |
| Periodontal Disease | (*2) | *2 | | *2 | |
| Coronary Artery Stenosis | | | *2 | | *2 |
| Osteoporosis | | | | | *2 |
| Insulin dependent diabetes | | | | *2 | |
| Diabetic retinopathy | | | | | *1 |
| Endstage renal diseases | | | | | (+) |
| Diabetic nephropathy | | | | | *2 |
| Hepatic fibrosis (Japanese alcoholics) | | | | | (+) |
| Alopecia areata | | | | | *2 |
| Graves' disease | | | | | *2 |
| Graves' ophthalmopathy | | | | | (-) |
| Extrathyroid disease | | | | | (+) |
| Systemic Lupus Erythematosus | | | | | *2 |
| Lichen Sclerosis | | | | | *2 |
| Arthritis | | | | | (+) |
| Juvenile chronic arthritis | *2 | | | | |
| Rheumatoid arthritis | | | | | (+) |
| Insulin dependent diabetes | | | | *2 | *2 VNTR |
| Ulcerative colitis | | | | | *2 |
| Asthma | | | *2 | *2 | |
| Multiple sclerosis | | | | (*2) | *2VNTR |
| Menopause, early onset | | | | | *2 |

Also disclosed herein are additional IL-1B alleles and haplotypes that modulate IL-1β expression and inflammatory conditions. Disclosed herein are SNPs at positions -3737 of IL-1B and -1468 of IL-1B. Individuals with a haplotype that includes IL-1B (-511) allele 1, IL-1B (-1468) allele 1, and IL-1B (-3737) allele 2 are typically overproducers of both IL-1β proteins, upon stimulation. Individuals with a haplotype that includes IL-1B (-511) allele 1, IL-1B (-1468) allele 1, and IL-1B (-3737) allele 1 are also typically overproducers of both IL-1β proteins. In contrast, individuals with a haplotype that includes IL-1B (-511) allele 2, IL-1B (-1468) allele 2, and IL-1B (-3737) allele 1 are typically underproducers of IL-1. Each allele within a haplotype may have an effect, as well as a composite genotype effect. In addition, particular diseases may be associated with both haplotype patterns. It should be noted that the SNP at IL-1B position -1468 has also been labeled IL-1B (-1464) and IL-1B (-1473). (See, Lee, et al, J. Gastroenterol. 2004, 39:429-433.)

In addition to the allelic patterns described above, as described herein, one of skill in the art can readily identify other alleles (including polymorphisms and mutations) that are in linkage disequilibrium with an allele associated with a disease or disorder. For example, a nucleic acid sample from a first group of subjects without a particular disorder can be collected, as well as DNA from a second group of subjects with the disorder. The nucleic acid sample can then be compared to identify those alleles that are over-represented in the second group as compared with the first group, wherein such alleles are presumably associated with a disorder, which is caused or contributed to by inappropriate interleukin 1 regulation. Alternatively, alleles that are in linkage disequilibrium with an allele that is associated with the disorder can be identified, for example, by genotyping a large population and performing statistical analysis to determine which alleles appear more commonly together than expected. Preferably the group is chosen to be comprised of genetically related individuals. Genetically related individuals include individuals from the same race, the same ethnic group, or even the same family. As the degree of genetic relatedness between a control group and a test group increases, so does the predictive value of polymorphic alleles which are ever more distantly linked to a disease-causing allele. This is because less evolutionary time has passed to allow polymorphisms which are linked along a chromosome in a founder population to redistribute through genetic cross-over events. Thus race-specific, ethnic-specific, and even family-specific diagnostic genotyping assays can be developed to allow for the detection of disease alleles which arose at ever more recent times in human evolution, e.g., after divergence of the major human races, after the separation of human populations into distinct ethnic groups, and even within the recent history of a particular family line.

Linkage disequilibrium between two polymorphic markers or between one polymorphic marker and a disease-causing mutation is a meta-stable state. Absent selective pressure or the sporadic linked reoccurrence of the underlying mutational events, the polymorphisms will eventually become disassociated by chromosomal recombination events and will thereby reach linkage equilibrium through the course of human evolution. Thus, the likelihood of finding a polymorphic allele in linkage disequilibrium with a disease or condition may increase with changes in at least two factors: decreasing physical distance between the polymorphic marker and the disease-causing mutation, and decreasing number of meiotic generations available for the dissociation of the linked pair. Consideration of the latter factor suggests that, the more closely related two individuals are, the more likely they will share a common parental chromosome or chromosomal region containing the linked polymorphisms and the less likely that this linked pair will have become unlinked through meiotic cross-over events occurring each generation. As a result, the more closely related two individuals are, the more likely it is that widely spaced polymorphisms may be co-inherited. Thus, for individuals related by common race, ethnicity or family, the reliability of ever more distantly spaced polymorphic loci can be relied upon as an indicator of inheritance of a linked disease-causing mutation.

Appropriate probes may be designed to hybridize to a specific gene of the IL-1 locus, such as IL-1A, IL-1B or IL-1RN or a related gene. These genomic DNA sequences are shown in Figures 3, 4 and 5, respectively, and further correspond to SEQ ID Nos. 1, 2 and 3, respectively. Alternatively, these probes may incorporate other regions of the relevant genomic locus, including intergenic sequences. Indeed the IL-1 region of human chromosome 2 spans some 400,000 base pairs and, assuming an average of one single nucleotide polymorphism every 1,000 base pairs, includes some 400 SNPs loci alone. Yet other polymorphisms available for use with the immediate invention are obtainable from various public sources. For example, the human genome database collects intragenic SNPs, is searchable by sequence and currently contains approximately 2,700 entries (http://hgbase.interactiva.de). Also available is a human polymorphism database maintained by the Massachusetts Institute of Technology (MIT SNP database (http://www.genome.wi.mit.edu/SNP/human/index.html)). From such sources SNPs as well as other human polymorphisms may be found.

For example, examination of the IL-1 region of the human genome in any one of these databases reveals that the IL-1 locus genes are flanked by a centromere proximal polymorphic marker designated microsatellite marker AFM220ze3 at 127.4 cM (centiMorgans) (see GenBank Acc. No. Z17008) and a distal polymorphic marker designated microsatellite anchor marker AFM087xa1 at 127.9 cM (see GenBank Acc. No. Z16545). These human polymorphic loci are both CA dinucleotide repeat microsatellite polymorphisms, and, as such, show a high degree of heterozygosity in human populations. For example, one allele of AFM220ze3 generates a 211 bp PCR amplification product with a 5' primer of the sequence TGTACCTAAGCCCACCCTTTAGAGC (SEQ ID No. 4) and a 3' primer of the sequence TGGCCTCCAGAAACCTCCAA (SEQ ID No. 5). Furthermore, one allele of AFM087xa1 generates a 177 bp PCR amplification product with a 5' primer of the sequence GCTGATATTCTGGTGGGAAA (SEQ ID No. 6) and a 3' primer of the sequence GGCAAGAGCAAAACTCTGTC (SEQ ID No. 7).

The human *IL1B* promoter is of interest in identifying polymorphisms associated with altered IL-1β production. Two primer sets are used to amplify the *IL-1B* promoter containing different 3' ends. Primers IL1BF1 5' cccACGCGTGAGTGAAAGGAATCCCGTTAGAAGT (SEQ ID NO: 33) and IL1BR2 5'cccACGCGTGCCTGTTGTGCCTTGTGCCTCGAAG (SEQ ID NO: 34) are used to generate *IL-1B* promoter fragment IL-1BS (+32 to -5326) (S = short; without the first exon). IL1BR1 5' cccACGCGTGGCTGCTTCAGACACCTGTGTA (SEQ ID NO: 35) is used to generate promoter fragment IL-1BL (+471 To -5326) (L=long; containing the first exon and SNP 17 (+45)).

Equivalent primers corresponding to unique sequences occurring 5' and 3' to these human chromosome 2 CA dinucleotide repeat polymorphisms will be apparent to one of skill in the art. Reasonable equivalent primers include those which hybridize within about 1 kb of the designated primer, and which further are anywhere from about 17 bp to about 27 bp in length. A general guideline for designing primers for amplification of unique human chromosomal genomic sequences is that they possess a melting temperature of at least about 50°C, wherein an approximate melting temperature can be estimated using the formula Tₘₑₗₜ = [2 x (# of A or T) + 4 x (# of G or C)].

A number of other human polymorphic loci occur between these two CA dinucleotide repeat polymorphisms and provide additional targets for determination of a prognostic allele in a family or other group of genetically related individuals. For example, the National Center for Biotechnology Information web site (www.ncbi.nlm.nih.gov/genemap/) lists a number of polymorphism markers in the region of the IL-1 locus and provides guidance in designing appropriate primers for amplification and analysis of these markers.

Accordingly, the nucleotide segments of the invention may be used for their ability to selectively form duplex molecules with complementary stretches of human chromosome 2 q 12-13 or cDNAs from that region or to provide primers for amplification of DNA or cDNA from this region. The design of appropriate probes for this purpose requires consideration of a number of factors. For example, fragments having a length of between 10, 15, or 18 nucleotides to about 20, or to about 30 nucleotides, will find particular utility. Longer sequences, e.g., 40, 50, 80, 90, 100, even up to full length, are even more preferred for certain embodiments. Lengths of oligonucleotides of at least about 18 to 20 nucleotides are well accepted by those of skill in the art as sufficient to allow sufficiently specific hybridization so as to be useful as a molecular probe. Furthermore, depending on the application envisioned, one will desire to employ varying conditions of hybridization to achieve varying degrees of selectivity of probe towards target sequence. For applications requiring high selectivity, one will typically desire to employ relatively stringent conditions to form the hybrids. For example, relatively low salt and/or high temperature conditions, such as provided by 0.02 M-0.15M NaCl at temperatures of about 50° C to about 70° C. Such selective conditions may tolerate little, if any, mismatch between the probe and the template or target strand.

Other alleles or other indicia of a disorder can be detected or monitored in a subject in conjunction with detection of the alleles described above, for example, identifying vessel wall thickness (e.g. as measured by ultrasound), or whether the subject smokes, drinks is overweight, is under stress or exercises.

### 4.2.2 Detection of Alleles

Many methods are available for detecting specific alleles at human polymorphic loci. The preferred method for detecting a specific polymorphic allele will depend, in part, upon the molecular nature of the polymorphism. For example, the various allelic forms of the polymorphic locus may differ by a single base-pair of the DNA. Such single nucleotide polymorphisms (or SNPs) are major contributors to genetic variation, comprising some 80% of all known polymorphisms, and their density in the human genome is estimated to be on average 1 per 1,000 base pairs. SNPs are most frequently biallelic- occurring in only two different forms (although up to four different forms of an SNP, corresponding to the four different nucleotide bases occurring in DNA, are theoretically possible). Nevertheless, SNPs are mutationally more stable than other polymorphisms, making them suitable for association studies in which linkage disequilibrium between markers and an unknown variant is used to map disease-causing mutations. In addition, because SNPs typically have only two alleles, they can be genotyped by a simple plus/minus assay rather than a length measurement, making them more amenable to automation.

A variety of methods are available for detecting the presence of a particular single nucleotide polymorphic allele in an individual. Advancements in this field have provided accurate, easy, and inexpensive large-scale SNP genotyping. Most recently, for example, several new techniques have been described including dynamic allele-specific hybridization (DASH), microplate array diagonal gel electrophoresis (MADGE), pyrosequencing, oligonucleotide-specific ligation, the TaqMan system as well as various DNA "chip" technologies such as the Affymetrix SNP chips. These methods require amplification of the target genetic region, typically by PCR. Still other newly developed methods, based on the generation of small signal molecules by invasive cleavage followed by mass spectrometry or immobilized padlock probes and rolling-circle amplification, might eventually eliminate the need for PCR. Several of the methods known in the art for detecting specific single nucleotide polymorphisms are summarized below. The method of the present invention is understood to include all available methods.

Several methods have been developed to facilitate analysis of single nucleotide polymorphisms. In one embodiment, the single base polymorphism can be detected by using a specialized exonuclease-resistant nucleotide, as disclosed, e.g., in Mundy, C. R. (U.S. Pat. No.4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

Disclosed is a solution-based method used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA ™ is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A. -C., et al., Genomics 8:684-692 (1990); Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA ^{™} in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A. -C., et al., Amer. J. Hum. Genet. 52:46-59 (1993)).

For mutations that produce premature termination of protein translation, the protein truncation test (PTT) offers an efficient diagnostic approach (Roest, et. al., (1993) Hum. Mol. Genet. 2:1719-21; van der Luijt, et. al., (1994) Genomics 20:1-4). For PTT, RNA is initially isolated from available tissue and reverse-transcribed, and the segment of interest is amplified by PCR. The products of reverse transcription PCR are then used as a template for nested PCR amplification with a primer that contains an RNA polymerase promoter and a sequence for initiating eukaryotic translation. After amplification of the region of interest, the unique motifs incorporated into the primer permit sequential *in vitro* transcription and translation of the PCR products. Upon sodium dodecyl sulfate-polyacrylamide gel electrophoresis of translation products, the appearance of truncated polypeptides signals the presence of a mutation that causes premature termination of translation. In a variation of this technique, DNA (as opposed to RNA) is used as a PCR template when the target region of interest is derived from a single exon.

Any cell type or tissue may be utilized to obtain nucleic acid samples for use in the diagnostics described herein. In a preferred embodiment, the DNA sample is obtained from a bodily fluid, e.g, blood, obtained by known techniques (e.g. venipuncture) or saliva. Alternatively, nucleic acid tests can be performed on dry samples (e.g. hair or skin). When using RNA or protein, the cells or tissues that may be utilized must express an IL-1 gene.

Diagnostic procedures may also be performed *in situ* directly upon tissue sections (fixed and/or frozen) of patient tissue obtained from biopsies or resections, such that no nucleic acid purification is necessary. Nucleic acid reagents may be used as probes and/or primers for such *in situ* procedures (see, for example, Nuovo, G.J., 1992, PCR in situ hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR.

A preferred detection method is allele specific hybridization using probes overlapping a region of at least one allele of an IL-1 proinflammatory haplotype and having about 5, 10, 20, 25, or 30 nucleotides around the mutation or polymorphic region. In a preferred embodiment of the invention, several probes capable of hybridizing specifically to other allelic variants involved in a restenosis are attached to a solid phase support, e.g., a "chip" (which can hold up to about 250,000 oligonucleotides). Oligonucleotides can be bound to a solid support by a variety of processes, including lithography. Mutation detection analysis using these chips comprising oligonucleotides, also termed "DNA probe arrays" is described e.g., in Cronin et al. (1996) Human Mutation 7:244. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

These techniques may also comprise the step of amplifying the nucleic acid before analysis. Amplification techniques are known to those of skill in the art and include, but are not limited to cloning, polymerase chain reaction (PCR), polymerase chain reaction of specific alleles (ASA), ligase chain reaction (LCR), nested polymerase chain reaction, self sustained sequence replication (Guatelli, J.C. et al., 1990, Proc. Natl. Acad. Sci. USA 87:1874-1878), transcriptional amplification system (Kwoh, D.Y. et al., 1989, Proc. Natl. Acad. Sci. USA 86:1173-1177), and Q- Beta Replicase (Lizardi, P.M. et al., 1988, Bio/Technology 6:1197).

Amplification products may be assayed in a variety of ways, including size analysis, restriction digestion followed by size analysis, detecting specific tagged oligonucleotide primers in the reaction products, allele-specific oligonucleotide (ASO) hybridization, allele specific 5' exonuclease detection, sequencing, hybridization, and the like.

PCR based detection means can include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be differentially detected. Of course, hybridization based detection means allow the differential detection of multiple PCR products in a sample. Other techniques are known in the art to allow multiplex analyses of a plurality of markers.

In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize 5' and 3' to at least one allele of an IL-1 proinflammatory haplotype under conditions such that hybridization and amplification of the allele occurs, and (iv) detecting the amplification product. These detection schemes are especially useful for the detection of nucleic acid molecules if such molecules are present in very low numbers.

In a preferred embodiment of the subject assay, the allele of an IL-1 proinflammatory haplotype is identified by alterations in restriction enzyme cleavage patterns. For example, sample and control DNA is isolated, amplified (optionally), digested with one or more restriction endonucleases, and fragment length sizes are determined by gel electrophoresis.

In yet another embodiment, any of a variety of sequencing reactions known in the art can be used to directly sequence the allele. Exemplary sequencing reactions include those based on techniques developed by Maxim and Gilbert ((1977) Proc. Natl Acad Sci USA 74:560) or Sanger (Sanger et al (1977) Proc. Nat. Acad. Sci USA 74:5463). It is also contemplated that any of a variety of automated sequencing procedures may be utilized when performing the subject assays (see, for example Biotechniques (1995) 19:448), including sequencing by mass spectrometry (see, for example PCT publication WO 94/16101; Cohen et al. (1996) Adv Chromatogr 36:127-162; and Griffin et al. (1993) Appl Biochem Biotechnol 38:147-159). It will be evident to one of skill in the art that, for certain embodiments, the occurrence of only one, two or three of the nucleic acid bases need be determined in the sequencing reaction. For instance, A-track or the like, e.g., where only one nucleic acid is detected, can be carried out.

In a further embodiment, protection from cleavage agents (such as a nuclease, hydroxylamine or osmium tetroxide and with piperidine) can be used to detect mismatched bases in RNA/RNA or RNA/DNA or DNA/DNA heteroduplexes (Myers, et al. (1985) Science 230:1242). In general, the art technique of "mismatch cleavage" starts by providing heteroduplexes formed by hybridizing (labeled) RNA or DNA containing the wild-type allele with the sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al (1988) Proc. Natl Acad Sci USA 85:4397; and Saleeba et al (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes). For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on an allele of an IL-1 locus haplotype is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify an IL-1 locus allele. For example, single strand conformation polymorphism (SSCP) may be used to detect differences in electrophoretic mobility between mutant and wild type nucleic acids (Orita et al. (1989) Proc Natl. Acad. Sci USA 86:2766, see also Cotton (1993) Mutat Res 285:125-144; and Hayashi (1992) Genet Anal Tech Appl 9:73-79). Single-stranded DNA fragments of sample and control IL-1 locus alleles are denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment, the movement of alleles in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) Biophys Chem 265:12753).

Examples of other techniques for detecting alleles include, but are not limited to, selective oligonucleotide hybridization, selective amplification, or selective primer extension. For example, oligonucleotide primers may be prepared in which the known mutation or nucleotide difference (e.g., in allelic variants) is placed centrally and then hybridized to target DNA under conditions which permit hybridization only if a perfect match is found (Saiki et al. (1986) Nature 324:163); Saiki et al (1989) Proc. Natl Acad. Sci USA 86:6230). Such allele specific oligonucleotide hybridization techniques may be used to test one mutation or polymorphic region per reaction when oligonucleotides are hybridized to PCR amplified target DNA or a number of different mutations or polymorphic regions when the oligonucleotides are attached to the hybridizing membrane and hybridized with labelled target DNA.

Alternatively, allele specific amplification technology which depends on selective PCR amplification may be used in conjunction with the instant invention. Oligonucleotides used as primers for specific amplification may carry the mutation or polymorphic region of interest in the center of the molecule (so that amplification depends on differential hybridization) (Gibbs et al (1989) Nucleic Acids Res. 17:2437-2448) or at the extreme 3' end of one primer where, under appropriate conditions, mismatch can prevent, or reduce polymerase extension (Prossner (1993) Tibtech 11:238. In addition it may be desirable to introduce a novel restriction site in the region of the mutation to create cleavage-based detection (Gasparini et al (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

In another embodiment, identification of the allelic variant is carried out using an oligonucleotide ligation assay (OLA), as described, e.g., in U.S. Pat. No. 4,998,617 and in Landegren, U. et al. ((1988) Science 241:1077-1080). The OLA protocol uses two oligonucleotides which are designed to be capable of hybridizing to abutting sequences of a single strand of a target. One of the oligonucleotides is linked to a separation marker, e.g., biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al. (1990) Proc. Natl. Acad. Sci. USA 87:8923-27). In this method, PCR is used to achieve the exponential amplification of target DNA, which is then detected using OLA.

Several techniques based on this OLA method have been developed and can be used to detect alleles of an IL-1 locus haplotype. For example, U.S. Patent No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996) Nucleic Acids Res 24: 3728), OLA combined with PCR permits typing of two alleles in a single microtiter well. By marking each of the allele-specific primers with a unique hapten, i.e. digoxigenin and fluorescein, each OLA reaction can be detected by using hapten specific antibodies that are labeled with different enzyme reporters, alkaline phosphatase or horseradish peroxidase. This system permits the detection of the two alleles using a high throughput format that leads to the production of two different colors.

Another embodiment of the invention is directed to kits for detecting a predisposition for developing a restenosis. This kit may contain one or more oligonucleotides, including 5' and 3' oligonucleotides that hybridize 5' and 3' to at least one allele of an IL-1 locus haplotype. PCR amplification oligonucleotides should hybridize between 25 and 2500 base pairs apart, preferably between about 100 and about 500 bases apart, in order to produce a PCR product of convenient size for subsequent analysis.

Particularly preferred primers for use in the diagnostic method of the invention include SEQ ID Nos. 8-23 and 36-39.

The design of additional oligonucleotides for use in the amplification and detection of IL-1 polymorphic alleles by the method of the invention is facilitated by the availability of both updated sequence information from human chromosome 2q13 - which contains the human IL-1 locus, and updated human polymorphism information available for this locus. For example, the DNA sequence for the IL-1A, IL-1B and IL-1RN is shown in Figures 1 (GenBank Accession No. X03833), 2 (GenBank Accession No. X04500) and 3 (GenBank Accession No. X64532) respectively. Suitable primers for the detection of a human polymorphism in these genes can be readily designed using this sequence information and standard techniques known in the art for the design and optimization of primers sequences. Optimal design of such primer sequences can be achieved, for example, by the use of commercially available primer selection programs such as Primer 2.1, Primer 3 or GeneFisher (See also, Nicklin M.H.J., Weith A. Duff G.W., "A Physical Map of the Region Encompassing the Human Interleukin-1α, interleukin-1β, and Interleukin-1 Receptor Antagonist Genes" Genomics 19: 382 (1995); Nothwang H.G., et al. "Molecular Cloning of the Interleukin-1 gene Cluster: Construction of an Integrated YAC/PAC Contig and a partial transcriptional Map in the Region of Chromosome 2q13" Genomics 41: 370 (1997); Clark, et al. (1986) Nucl. Acids. Res., 14:7897-7914 [published erratum appears in Nucleic Acids Res., 15:868 (1987) and the Genome Database (GDB) project at the URL http://www.gdb.org).

For use in a kit, oligonucleotides may be any of a variety of natural and/or synthetic compositions such as synthetic oligonucleotides, restriction fragments, cDNAs, synthetic peptide nucleic acids (PNAs), and the like. The assay kit and method may also employ labeled oligonucleotides to allow ease of identification in the assays. Examples of labels which may be employed include radio-labels, enzymes, fluorescent compounds, streptavidin, avidin, biotin, magnetic moieties, metal binding moieties, antigen or antibody moieties, and the like.

The kit may, optionally, also include DNA sampling means. DNA sampling means are well known to one of skill in the art and can include, but not be limited to substrates, such as filter papers, the AmpliCard™ (University of Sheffield, Sheffield, England S10 2JF; Tarlow, JW, et al., J. of Invest. Dermatol. 103:387-389 (1994)) and the like; DNA purification reagents such as Nucleon™ kits, lysis buffers, proteinase solutions and the like; PCR reagents, such as 10X reaction buffers, thermostable polymerase, dNTPs, and the like; and allele detection means such as the *Hinf*I restriction enzyme, allele specific oligonucleotides, degenerate oligonucleotide primers for nested PCR from dried blood.

### 4.2.3. Pharmacogenomics

Knowledge of the particular alleles associated with a susceptibility to developing a particular disease or condition, alone or in conjunction with information on other genetic defects contributing to the particular disease or condition allows a customization of the prevention or treatment in accordance with the individual's genetic profile, the goal of "pharmacogenomics". Thus, comparison of an individual's IL-1 profile to the population profile for a vascular disorder, permits the selection or design of drugs or other therapeutic regimens that are expected to be safe and efficacious for a particular patient or patient population (i.e., a group of patients having the same genetic alteration).

In addition, the ability to target populations expected to show the highest clinical benefit, based on genetic profile can enable: 1) the repositioning of already marketed drugs; 2) the rescue of drug candidates whose clinical development has been discontinued as a result of safety or efficacy limitations, which are patient subgroup-specific; and 3) an accelerated and less costly development for candidate therapeutics and more optimal drug labeling (e.g. since measuring the effect of various doses of an agent on the causative mutation is useful for optimizing effective dose).

The treatment of an individual with a particular therapeutic can be monitored by determining protein (e.g. IL-1α, IL-1β, or IL-1Ra), mRNA and/or transcriptional level. Depending on the level detected, the therapeutic regimen can then be maintained or adjusted (increased or decreased in dose). In a preferred embodiment, the effectiveness of treating a subject with an agent comprises the steps of: (i) obtaining a preadministration sample from a subject prior to administration of the agent; (ii) detecting the level or amount of a protein, mRNA or genomic DNA in the preadministration sample; (iii) obtaining one or more post-administration samples from the subject; (iv) detecting the level of expression or activity of the protein, mRNA or genomic DNA in the post-administration sample; (v) comparing the level of expression or activity of the protein, mRNA or genomic DNA in the preadministration sample with the corresponding protein, mRNA or genomic DNA in the postadministration sample, respectively; and (vi) altering the administration of the agent to the subject accordingly.

Cells of a subject may also be obtained before and after administration of a therapeutic to detect the level of expression of genes other than an IL-1 gene to verify that the therapeutic does not increase or decrease the expression of genes which could be deleterious. This can be done, e.g., by using the method of transcriptional profiling. Thus, mRNA from cells exposed in vivo to a therapeutic and mRNA from the same type of cells that were not exposed to the therapeutic could be reverse transcribed and hybridized to a chip containing DNA from numerous genes, to thereby compare the expression of genes in cells treated and not treated with the therapeutic.

### 4.3 Therapeutics For Diseases and Conditions Associated with IL-1 Polymorphisms

Therapeutic for diseases or conditions associated with an IL-1 polymorphism or haplotype refers to any agent or therapeutic regimen (including pharmaceuticals, nutraceuticals and surgical means) that prevents or postpones the development of or alleviates the symptoms of the particular disease or condition in the subject. The therapeutic can be a polypeptide, peptidomimetic, nucleic acid or other inorganic or organic molecule, preferably a "small molecule" including vitamins, minerals and other nutrients. Preferably the therapeutic can modulate at least one activity of an IL-1 polypeptide, e.g., interaction with a receptor, by mimicking or potentiating (agonizing) or inhibiting (antagonizing) the effects of a naturally-occurring polypeptide. An agonist can be a wild-type protein or derivative thereof having at least one bioactivity of the wild-type, e.g., receptor binding activity. An agonist can also be a compound that upregulates expression of a gene or which increases at least one bioactivity of a protein. An agonist can also be a compound which increases the interaction of a polypeptide with another molecule, e.g., a receptor. An antagonist can be a compound which inhibits or decreases the interaction between a protein and another molecule, e.g., a receptor or an agent that blocks signal transduction or post-translation processing (e.g., IL-1 converting enzyme (ICE) inhibitor). Accordingly, a preferred antagonist is a compound which inhibits or decreases binding to a receptor and thereby blocks subsequent activation of the receptor. An antagonist can also be a compound that downregulates expression of a gene or which reduces the amount of a protein present. The antagonist can be a dominant negative form of a polypeptide, e.g., a form of a polypeptide which is capable of interacting with a target peptide, e.g., a receptor, but which does not promote the activation of the receptor. The antagonist can also be a nucleic acid encoding a dominant negative form of a polypeptide, an antisense nucleic acid, or a ribozyme capable of interacting specifically with an RNA. Yet other antagonists are molecules which bind to a polypeptide and inhibit its action. Such molecules include peptides, e.g., forms of target peptides which do not have biological activity, and which inhibit binding to receptors. Thus, such peptides will bind to the active site of a protein and prevent it from interacting with target peptides. Yet other antagonists include antibodies that specifically interact with an epitope of a molecule, such that binding interferes with the biological function of the polypeptide. In yet another preferred embodiment, the antagonist is a small molecule, such as a molecule capable of inhibiting the interaction between a polypeptide and a target receptor. Alternatively, the small molecule can function as an antagonist by interacting with sites other than the receptor binding site.

Modulators of IL-1 (e.g. IL-1α, IL-1β or IL-1 receptor antagonist) or a protein encoded by a gene that is in linkage disequilibrium with an IL-1 gene can comprise any type of compound, including a protein, peptide, peptidomimetic, small molecule, or nucleic acid. Preferred agonists include nucleic acids (e.g. encoding an IL-1 protein or a gene that is up- or down-regulated by an IL-1 protein), proteins (e.g. IL-1 proteins or a protein that is up- or down-regulated thereby) or a small molecule (e.g. that regulates expression or binding of an IL-1 protein). Preferred antagonists, which can be identified, for example, using the assays described herein, include nucleic acids (e.g. single (antisense) or double stranded (triplex) DNA or PNA and ribozymes), protein (e.g. antibodies) and small molecules that act to suppress or inhibit IL-1 transcription and/or protein activity.

### 4.3.1. Effective Dose

Toxicity and therapeutic efficacy of such compounds can be determined by standard pharmaceutical procedures in cell cultures or experimental animals, e.g., for determining The LD50 (the dose lethal to 50% of the population) and the Ed50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissues in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

### 4.3.2. Formulation and Use

Compositions for use in accordance with the present disclosure may be formulated in a conventional manner using one or more physiologically acceptable carriers or excipients. Thus, the compounds and their physiologically acceptable salts and solvates may be formulated for administration by, for example, injection, inhalation or insufflation (either through the mouth or the nose) or oral, buccal, parenteral or rectal administration.

For such therapy, the compounds of the invention can be formulated for a variety of loads of administration, including systemic and topical or localized administration. Techniques and formulations generally may be found in Remmington's Pharmaceutical Sciences, Meade Publishing Co., Easton, PA. For systemic administration, injection is preferred, including intramuscular, intravenous, intraperitoneal, and subcutaneous. For injection, the compounds of the invention can be formulated in liquid solutions, preferably in physiologically compatible buffers such as Hank's solution or Ringer's solution. In addition, the compounds may be formulated in solid form and redissolved or suspended immediately prior to use. Lyophilized forms are also included.

For oral administration, the compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulfate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the compositions may take the form of tablets or lozenges formulated in conventional manner. For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt. Other suitable delivery systems include microspheres which offer the possibility of local noninvasive delivery of drugs over an extended period of time. This technology utilizes microspheres of precapillary size which can be injected via a coronary catheter into any selected part of the e.g. heart or other organs without causing inflammation or ischemia. The administered therapeutic is slowly released from these microspheres and taken up by surrounding tissue cells (e.g. endothelial cells).

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the oligomers of the invention are formulated into ointments, salves, gels, or creams as generally known in the art. A wash solution can be used locally to treat an injury or inflammation to accelerate healing.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### 4.4 Assays to Identify Therapeutics

Based on the identification of mutations that cause or contribute to the development of a disease or disorder that is associated with an IL-1 polymorphism or haplotype, the invention further features cell-based or cell free assays for identifying therapeutics. In one embodiment, a cell expressing an IL-1 receptor, or a receptor for a protein that is encoded by a gene which is in linkage disequilibrium with an IL-1 gene, on the outer surface of its cellular membrane is incubated in the presence of a test compound alone or in the presence of a test compound and another protein and the interaction between the test compound and the receptor or between the protein (preferably a tagged protein) and the receptor is detected, e.g., by using a microphysiometer (McConnell et al. (1992) Science 257:1906). An interaction between the receptor and either the test compound or the protein is detected by the microphysiometer as a change in the acidification of the medium. This assay system thus provides a means of identifying molecular antagonists which, for example, function by interfering with protein- receptor interactions, as well as molecular agonist which, for example, function by activating a receptor.

Cellular or cell-free assays can also be used to identify compounds which modulate expression of an IL-1 gene or a gene in linkage disequilibrium therewith, modulate translation of an mRNA, or which modulate the stability of an mRNA or protein. Accordingly, in one embodiment, a cell which is capable of producing an IL-1, or other protein is incubated with a test compound and the amount of protein produced in the cell medium is measured and compared to that produced from a cell which has not been contacted with the test compound. The specificity of the compound vis a vis the protein can be confirmed by various control analysis, e.g., measuring the expression of one or more control genes. In particular, this assay can be used to determine the efficacy of antisense, ribozyme and triplex compounds.

Cell-free assays can also be used to identify compounds which are capable of interacting with a protein, to thereby modify the activity of the protein. Such a compound can, e.g., modify the structure of a protein thereby effecting its ability to bind to a receptor. In a preferred embodiment, cell-free assays for identifying such compounds consist essentially in a reaction mixture containing a protein and a test compound or a library of test compounds in the presence or absence of a binding partner. A test compound can be, e.g., a derivative of a binding partner, e.g., a biologically inactive target peptide, or a small molecule.

Accordingly, one exemplary screening assay of the present disclosure includes the steps of contacting a protein or functional fragment thereof with a test compound or library of test compounds and detecting the formation of complexes. For detection purposes, the molecule can be labeled with a specific marker and the test compound or library of test compounds labeled with a different marker. Interaction of a test compound with a protein or fragment thereof can then be detected by determining the level of the two labels after an incubation step and a washing step. The presence of two labels after the washing step is indicative of an interaction.

An interaction between molecules can also be identified by using real-time BIA (Biomolecular Interaction Analysis, Pharmacia Biosensor AB) which detects surface plasmon resonance (SPR), an optical phenomenon. Detection depends on changes in the mass concentration of macromolecules at the biospecific interface, and does not require any labeling of interactants. In one embodiment, a library of test compounds can be immobilized on a sensor surface, e.g., which forms one wall of a micro-flow cell. A solution containing the protein or functional fragment thereof is then flown continuously over the sensor surface. A change in the resonance angle as shown on a signal recording, indicates that an interaction has occurred. This technique is further described, e.g., in BIAtechnology Handbook by Pharmacia.

Another exemplary screening assay of the present disclosure includes the steps of (a) forming a reaction mixture including: (i) an IL-1 or other protein, (ii) an appropriate receptor, and (iii) a test compound; and (b) detecting interaction of the protein and receptor. A statistically significant change (potentiation or inhibition) in the interaction of the protein and receptor in the presence of the test compound, relative to the interaction in the absence of the test compound, indicates a potential antagonist (inhibitor). The compounds of this assay can be contacted simultaneously. Alternatively, a protein can first be contacted with a test compound for an appropriate amount of time, following which the receptor is added to the reaction mixture. The efficacy of the compound can be assessed by generating dose response curves from data obtained using various concentrations of the test compound. Moreover, a control assay can also be performed to provide a baseline for comparison.

Complex formation between a protein and receptor may be detected by a variety of techniques. Modulation of the formation of complexes can be quantitated using, for example, detectably labeled proteins such as radiolabeled, fluorescently labeled, or enzymatically labeled proteins or receptors, by immunoassay, or by chromatographic detection.

Typically, it will be desirable to immobilize either the protein or the receptor to facilitate separation of complexes from uncomplexed forms of one or both of the proteins, as well as to accommodate automation of the assay. Binding of protein and receptor can be accomplished in any vessel suitable for containing the reactants. Examples include microtitre plates, test tubes, and micro-centrifuge tubes. In one embodiment, a fusion protein can be provided which adds a domain that allows the protein to be bound to a matrix. For example, glutathione-S-transferase fusion proteins can be adsorbed onto glutathione sepharose beads (Sigma Chemical, St. Louis, MO) or glutathione derivatized microtitre plates, which are then combined with the receptor, e.g. an 35S-labeled receptor, and the test compound, and the mixture incubated under conditions conducive to complex formation, e.g. at physiological conditions for salt and pH, though slightly more stringent conditions may be desired. Following incubation, the beads are washed to remove any unbound label, and the matrix immobilized and radiolabel determined directly (e.g. beads placed in scintillant), or in the supernatant after the complexes are subsequently dissociated. Alternatively, the complexes can be dissociated from the matrix, separated by SDS-PAGE, and the level of protein or receptor found in the bead fraction quantitated from the gel using standard electrophoretic techniques such as described in the appended examples. Other techniques for immobilizing proteins on matrices are also available for use in the subject assay. For instance, either protein or receptor can be immobilized utilizing conjugation of biotin and streptavidin. Transgenic animals can also be made to identify agonists and antagonists or to confirm the safety and efficacy of a candidate therapeutic. Transgenic animals of the invention can include non-human animals containing a restenosis causative mutation under the control of an appropriate endogenous promoter or under the control of a heterologous promoter.

The transgenic animals can also be animals containing a transgene, such as reporter gene, under the control of an appropriate promoter or fragment thereof. These animals are useful, e.g., for identifying drugs that modulate production of an IL-1 protein, such as by modulating gene expression. Methods for obtaining transgenic non-human animals are well known in the art. In preferred embodiments, the expression of the restenosis causative mutation is restricted to specific subsets of cells, tissues or developmental stages utilizing, for example, cis-acting sequences that control expression in the desired pattern. In the present invention, such mosaic expression of a protein can be essential for many forms of lineage analysis and can additionally provide a means to assess the effects of, for example, expression level which might grossly alter development in small patches of tissue within an otherwise normal embryo. Toward this end, tissue-specific regulatory sequences and conditional regulatory sequences can be used to control expression of the mutation in certain spatial patterns. Moreover, temporal patterns of expression can be provided by, for example, conditional recombination systems or prokaryotic transcriptional regulatory sequences. Genetic techniques, which allow for the expression of a mutation can be regulated via site-specific genetic manipulation *in vivo,* are known to those skilled in the art.

The transgenic animals disclosed all include within a plurality of their cells a causative mutation transgene, which alters the phenotype of the "host cell". In an illustrative embodiment, either the *cre*/*loxP* recombinase system of bacteriophage P1 (Lakso et al. (1992) PNAS 89:6232-6236; Orban et al. (1992) PNAS 89:6861-6865) or the FLP recombinase system of *Saccharomyces cerevisiae* (O'Gorman et al. (1991) Science 251:1351-1355; PCT publication WO 92/15694) can be used to generate *in vivo* site-specific genetic recombination systems. Cre recombinase catalyzes the site-specific recombination of an intervening target sequence located between *loxP* sequences. *loxP* sequences are 34 base pair nucleotide repeat sequences to which the Cre recombinase binds and are required for Cre recombinase mediated genetic recombination. The orientation of *loxP* sequences determines whether the intervening target sequence is excised or inverted when Cre recombinase is present (Abremski et al. (1984) J. Biol. Chem. 259:1509-1514); catalyzing the excision of the target sequence when the *loxP* sequences are oriented as direct repeats and catalyzes inversion of the target sequence when *loxP* sequences are oriented as inverted repeats.

Accordingly, genetic recombination of the target sequence is dependent on expression of the Cre recombinase. Expression of the recombinase can be regulated by promoter elements which are subject to regulatory control, e.g., tissue-specific, developmental stage-specific, inducible or repressible by externally added agents. This regulated control will result in genetic recombination of the target sequence only in cells where recombinase expression is mediated by the promoter element. Thus, the activation of expression of the causative mutation transgene can be regulated via control of recombinase expression.

Use of the *cre*/*loxP* recombinase system to regulate expression of a causative mutation transgene requires the construction of a transgenic animal containing transgenes encoding both the Cre recombinase and the subject protein. Animals containing both the Cre recombinase and the restenosis causative mutation transgene can be provided through the construction of "double" transgenic animals. A convenient method for providing such animals is to mate two transgenic animals each containing a transgene.

Similar conditional transgenes can be provided using prokaryotic promoter sequences which require prokaryotic proteins to be simultaneous expressed in order to facilitate expression of the transgene. Exemplary promoters and the corresponding transactivating prokaryotic proteins are given in U.S. Patent No. 4,833,080.

Moreover, expression of the conditional transgenes can be induced by gene therapy-like methods wherein a gene encoding the transactivating protein, e.g. a recombinase or a prokaryotic protein, is delivered to the tissue and caused to be expressed, such as in a cell-type specific manner. By this method, the transgene could remain silent into adulthood until "turned on" by the introduction of the transactivator.

In an exemplary embodiment, the "transgenic non-human animals" of the invention are produced by introducing transgenes into the germline of the non-human animal. Embryonal target cells at various developmental stages can be used to introduce transgenes. Different methods are used depending on the stage of development of the embryonal target cell. The specific line(s) of any non-human animal used to practice this invention are selected for general good health, good embryo yields, good pronuclear visibility in the embryo, and good reproductive fitness. In addition, the haplotype is a significant factor. For example, when transgenic mice are to be produced, strains such as C57BL/6 or FVB lines are often used (Jackson Laboratory, Bar Harbor, ME). Preferred strains are those with H-2b, H-2d or H-2q haplotypes such as C57BL/6 or DBA/1. The line(s) used to practice this invention may themselves be transgenics, and/or may be knockouts (i.e., obtained from animals which have one or more genes partially or completely suppressed) .

In one embodiment, the transgene construct is introduced into a single stage embryo. The zygote is the best target for microinjection. In the mouse, the male pronucleus reaches the size of approximately 20 micrometers in diameter which allows reproducible injection of 1-2 pl of DNA solution. The use of zygotes as a target for gene transfer has a major advantage in that in most cases the injected DNA will be incorporated into the host gene before the first cleavage (Brinster et al. (1985) PNAS 82:4438-4442). As a consequence, all cells of the transgenic animal will carry the incorporated transgene. This will in general also be reflected in the efficient transmission of the transgene to offspring of the founder since 50% of the germ cells will harbor the transgene.

Normally, fertilized embryos are incubated in suitable media until the pronuclei appear. At about this time, the nucleotide sequence comprising the transgene is introduced into the female or male pronucleus as described below. In some species such as mice, the male pronucleus is preferred. It is most preferred that the exogenous genetic material be added to the male DNA complement of the zygote prior to its being processed by the ovum nucleus or the zygote female pronucleus. It is thought that the ovum nucleus or female pronucleus release molecules which affect the male DNA complement, perhaps by replacing the protamines of the male DNA with histones, thereby facilitating the combination of the female and male DNA complements to form the diploid zygote. Thus, it is preferred that the exogenous genetic material be added to the male complement of DNA or any other complement of DNA prior to its being affected by the female pronucleus. For example, the exogenous genetic material is added to the early male pronucleus, as soon as possible after the formation of the male pronucleus, which is when the male and female pronuclei are well separated and both are located close to the cell membrane. Alternatively, the exogenous genetic material could be added to the nucleus of the sperm after it has been induced to undergo decondensation. Sperm containing the exogenous genetic material can then be added to the ovum or the decondensed sperm could be added to the ovum with the transgene constructs being added as soon as possible thereafter.

Introduction of the transgene nucleotide sequence into the embryo may be accomplished by any means known in the art such as, for example, microinjection, electroporation, or lipofection. Following introduction of the transgene nucleotide sequence into the embryo, the embryo may be incubated *in vitro* for varying amounts of time, or reimplanted into the surrogate host, or both. In vitro incubation to maturity is within the scope of this invention. One common method in to incubate the embryos in vitro for about 1-7 days, depending on the species, and then reimplant them into the surrogate host.

For the purposes of this invention a zygote is essentially the formation of a diploid cell which is capable of developing into a complete organism. Generally, the zygote will be comprised of an egg containing a nucleus formed, either naturally or artificially, by the fusion of two haploid nuclei from a gamete or gametes. Thus, the gamete nuclei must be ones which are naturally compatible, i.e., ones which result in a viable zygote capable of undergoing differentiation and developing into a functioning organism. Generally, a euploid zygote is preferred. If an aneuploid zygote is obtained, then the number of chromosomes should not vary by more than one with respect to the euploid number of the organism from which either gamete originated.

In addition to similar biological considerations, physical ones also govern the amount (e.g., volume) of exogenous genetic material which can be added to the nucleus of the zygote or to the genetic material which forms a part of the zygote nucleus. If no genetic material is removed, then the amount of exogenous genetic material which can be added is limited by the amount which will be absorbed without being physically disruptive. Generally, the volume of exogenous genetic material inserted will not exceed about 10 picoliters. The physical effects of addition must not be so great as to physically destroy the viability of the zygote. The biological limit of the number and variety of DNA sequences will vary depending upon the particular zygote and functions of the exogenous genetic material and will be readily apparent to one skilled in the art, because the genetic material, including the exogenous genetic material, of the resulting zygote must be biologically capable of initiating and maintaining the differentiation and development of the zygote into a functional organism.

The number of copies of the transgene constructs which are added to the zygote is dependent upon the total amount of exogenous genetic material added and will be the amount which enables the genetic transformation to occur. Theoretically only one copy is required; however, generally, numerous copies are utilized, for example, 1,000-20,000 copies of the transgene construct, in order to insure that one copy is functional. As regards the present invention, there will often be an advantage to having more than one functioning copy of each of the inserted exogenous DNA sequences to enhance the phenotypic expression of the exogenous DNA sequences.

Any technique which allows for the addition of the exogenous genetic material into nucleic genetic material can be utilized so long as it is not destructive to the cell, nuclear membrane or other existing cellular or genetic structures. The exogenous genetic material is preferentially inserted into the nucleic genetic material by microinjection. Microinjection of cells and cellular structures is known and is used in the art.

Reimplantation is accomplished using standard methods. Usually, the surrogate host is anesthetized, and the embryos are inserted into the oviduct. The number of embryos implanted into a particular host will vary by species, but will usually be comparable to the number of off spring the species naturally produces.

Transgenic offspring of the surrogate host may be screened for the presence and/or expression of the transgene by any suitable method. Screening is often accomplished by Southern blot or Northern blot analysis, using a probe that is complementary to at least a portion of the transgene. Western blot analysis using an antibody against the protein encoded by the transgene may be employed as an alternative or additional method for screening for the presence of the transgene product. Typically, DNA is prepared from tail tissue and analyzed by Southern analysis or PCR for the transgene. Alternatively, the tissues or cells believed to express the transgene at the highest levels are tested for the presence and expression of the transgene using Southern analysis or PCR, although any tissues or cell types may be used for this analysis.

Alternative or additional methods for evaluating the presence of the transgene include, without limitation, suitable biochemical assays such as enzyme and/or immunological assays, histological stains for particular marker or enzyme activities, flow cytometric analysis, and the like. Analysis of the blood may also be useful to detect the presence of the transgene product in the blood, as well as to evaluate the effect of the transgene on the levels of various types of blood cells and other blood constituents.

Progeny of the transgenic animals may be obtained by mating the transgenic animal with a suitable partner, or by *in vitro* fertilization of eggs and/or sperm obtained from the transgenic animal. Where mating with a partner is to be performed, the partner may or may not be transgenic and/or a knockout; where it is transgenic, it may contain the same or a different transgene, or both. Alternatively, the partner may be a parental line. Where *in vitro* fertilization is used, the fertilized embryo may be implanted into a surrogate host or incubated *in vitro,* or both. Using either method, the progeny may be evaluated for the presence of the transgene using methods described above, or other appropriate methods.

The transgenic animals produced in accordance with the present invention will include exogenous genetic material. Further, in such embodiments the sequence will be attached to a transcriptional control element, e.g., a promoter, which preferably allows the expression of the transgene product in a specific type of cell.

Retroviral infection can also be used to introduce the transgene into a non-human animal. The developing non-human embryo can be cultured *in vitro* to the blastocyst stage. During this time, the blastomeres can be targets for retroviral infection (Jaenich, R. (1976) PNAS 73:1260-1264). Efficient infection of the blastomeres is obtained by enzymatic treatment to remove the zona pellucida (Manipulating the Mouse Embryo, Hogan eds. (Cold Spring Harbor Laboratory Press, Cold Spring Harbor, 1986). The viral vector system used to introduce the transgene is typically a replication-defective retrovirus carrying the transgene (Jahner et al. (1985) PNAS 82:6927-6931; Van der Putten et al. (1985) PNAS 82:6148-6152). Transfection is easily and efficiently obtained by culturing the blastomeres on a monolayer of virus-producing cells (Van der Putten, *supra*; Stewart et al. (1987) EMBO J. 6:383-388). Alternatively, infection can be performed at a later stage. Virus or virus-producing cells can be injected into the blastocoele (Jahner et al. (1982) Nature 298:623-628). Most of the founders will be mosaic for the transgene since incorporation occurs only in a subset of the cells which formed the transgenic non-human animal. Further, the founder may contain various retroviral insertions of the transgene at different positions in the genome which generally will segregate in the offspring. In addition, it is also possible to introduce transgenes into the germ line by intrauterine retroviral infection of the midgestation embryo (Jahner et al. (1982) *supra*).

A third type of target cell for transgene introduction is the embryonal stem cell (ES). ES cells are obtained from pre-implantation embryos cultured *in vitro* and fused with embryos (Evans et al. (1981) Nature 292:154-156; Bradley et al. (1984) Nature 309:255-258; Gossler et al. (1986) PNAS 83: 9065-9069; and Robertson et al. (1986) Nature 322:445-448). Transgenes can be efficiently introduced into the ES cells by DNA transfection or by retrovirus-mediated transduction. Such transformed ES cells can thereafter be combined with blastocysts from a non-human animal. The ES cells thereafter colonize the embryo and contribute to the germ line of the resulting chimeric animal. For review see Jaenisch, R. (1988) Science 240:1468-1474.

The present invention is further illustrated by the following examples which should not be construed as limiting in any way. The contents of all cited references (including literature references, issued patents, published patent applications as cited throughout this application) are hereby expressly incorporated by reference. The practice of the present invention will employ, unless otherwise indicated, conventional techniques that are within the skill of the art. Such techniques are explained fully in the literature. See, for example, Molecular Cloning A Laboratory Manual, (2nd ed., Sambrook, Fritsch and Maniatis, eds., Cold Spring Harbor Laboratory Press: 1989); DNA Cloning, Volumes I and II (D. N. Glover ed., 1985); Oligonucleotide Synthesis (M. J. Gait ed., 1984); U.S. Patent No. 4,683,195; U.S. Patent No. 4,683,202; and Nucleic Acid Hybridization (B. D. Hames & S. J. Higgins eds., 1984).

### 5. EXAMPLES

### Example 1 Genotyping

All human subjects were unrelated, Caucasian, healthy blood donors from Sheffield (n = 112). Subjects were typed at the loci indicated in Table 1.

**Table 2. Markers Used in Haplotype Study**

| **Marker** | **Gene** | **Reference** |
|---|---|---|
| 2221223 | IL1A | Todd& Naylor, Nucleic Acids Res. 19: 3756(1991) |
| gz5/gz6 | IL1A | Zuliani, et al., Am. J. Hum. Genet. 46: 963-69 (1990) |
| -889 | IL1A | McDowell, et al., Arth. & Rheum. 38: 221 -8(1995) |
| +3954 | IL1B | di Giovine, et al., Cytokine 7(6): 606 (1995) |
| -511 | IL1B | di Giovine, Hum. Molec. Genet. 1(6): 450 (1992) |
| gaat.p33330 | between IL1B and IL1RN | Murray, et al., Coop. Hum. Link. Center, unpublished |
| Y31 | between IL1B and IL1RN | Spurr, et al., Cytogenet. & Cell Genet. 73: 255-73 (1996) |
| VNTR | IL1RN | Tarlow, *et al., Hum. Genet.* 91: 403-4 (1993) |

The primer sequences and fluorescent labels used in PCR amplification of markers were as in Table 3.

**Table 3. Primer Sequence and Flourescent Label for Genotyping**

| **Marker** | **Label** | **Primer Sequence** |
|---|---|---|
| 2221223 | HEX | ATGTATAGAATTCCATTCCTG (SEQ ID NO. 8) |
| | | TAAAATCAAGTGTTGATGTAG (SEQ ID NO. 9) |
| gz51 gz6 | FAM | GGGA7TACAGGCGTGAGCCACCGCG (SEQ ID NO. 10) |
| | | TTAGTATTGCTGGTAGTATTCATAT (SEQ ID NO. 11) |
| -889 | NONE | TGTTCTACCACCTGAACTAGG (SEQ ID NO. 12) |
| | | TTACATATGAGCCTTCCATG (SEQ ID NO. 13) |
| +3954 | NONE | CTCAGGTGTCCTCGAAGAAATCAAA (SEQ ID NO. 14) |
| | | GCTTTMGCTGTGAGTCCCG (SEQ ID NO. 15) |
| -511 | NONE | TGGCATTGATCTGGTTCATC (SEQ ID NO. 16) |
| | | GTTTAGGAATCTTCCCACTT(SEO ID NO. 17) |
| gaat.p33330 | FAM | GAGGCGTGAGAATCTCAAGA (SEQ ID NO. 18) |
| | | GTGTCCTCAAGTGGATCTGG (SEQ 10 NO. 19) |
| Y31 | HEX | GGGCAACAGAGCAATGTTTCT (SEQ ID NO. 20) |
| | | CAGTGTGTCAGTGTACTGTT (SEQ ID NO. 21) |
| VNTR | NONE | CTCAGCAACACTCCTAT (SEQ ID NO. 22) |
| | | TCCTGGTCTGCAGGTAA (SEQ ID NO. 23) |
| -1468 | NONE | CCT ATT TCC CTC GTG TCT CAA ATA CT (SEQ ID NO: 36) |
| | | TCA AAT TAG TAT GTG CCA GGT ATC GT |
| -3737 | NONE | CTA AGC CCT CCT TGT TCT AGA CCA |
| | | CTC TTG GAT CCA GGT TCT CAG ATT |

Reaction conditions were as described in Table 4.

**Table 4. Reaction Conditions**

| Marker | Conditions |
|---|---|
| 222/223 | 50 mM KCI, 10 mM Tris-HCI pH 9.0,1.5 mM MgCI₂, 200:mM dNTPs, 25 ng primers, 50 ng template, 0.004% W-1 (Gibco-BRL) 0.2 u Taq, PCR was done at 30 cycles of 94°C for 1". 55°C for 1", 72°C for 1" |
| gz5/gz6 | as per marker 2221223, except 1 u of Perfect Match (StrataGene) was added |
| -889 | per marker 222/223, except PCR was done for 1 cycle at 96°C for 1", 40 cycles of 94°C for 1", 46°C for 1" 72°C for 1" and 1 cycle of 72°C for 4", products were cleaved with Ncol for analysis |
| +3954 | as per marker 222/223, except PCR was done for 35 cycles with annealing at 67.5°C, products were cleaved with Taq 1 for analysis |
| -511 | as per marker 2221223, except PCR was done for 1 cycle at 95°C for 2", 35 cycles of 95°C for 1", 53°C for 1" 74°C for 1" and 1 cycle of 74°C for 4", products were cleaved with Aval and Bsu361 for analysis |
| gaat.p33330 | per marker 222/223 |
| Y31 | per marker 222/223 |
| VNTR | per marker 222/223 except with 1.7 mM MgCI₂, 1 cycle at 96°C for 1"; 30 cycles of 94°C for 1", 60°C for 1", 70°C for 1" and 1 cycle at 70°C for 2" |

2221223, gz5/gz6, gaat.p33330 and Y31 PCR products were examined by agarose gel electrophoresis and the remainder of the PCR products were pooled according to the intensity of ethidium bromide staining. 2 gl of the pool was analyzed on an ABI 373A automated sequencer and allele sizes were determined using the Genescan and Genotyper software. Alleles were globally binned using a simple computer program and numbered in order of size.

-889 PCR products were digested with NcoI and the resulting fragments sized on 8% PAGE. Allele 1 produces 83 and 16 bp fragments. Allele 2 produces a 99 bp fragment.

+3954 PCR products were digested with restriction enzyme Taq I. Allele 1 produces fragments of 97, 85 and 12 bp, and allele 2 produces fragments of 182 and 12 bp.

-511 PCR products were digested with AvaI and Bsu36I and the fragments were sized by 8% PAGE. Allele 1 produces 190 and 114 bp fragments when digested with AvaI and a 304 bp fragment when digested with Bsu36I. Allele 2 produces a 304 bp fragment when digested with AvaI and 190 and 114 bp fragments when digested with Bsu36I.

VNTR PCR products were sized by electrophoresis on 2% agarose gel at 90V for 45 minutes. Allele 1 has 4 repeats and the PCR product is 412 bp, allele 2 has 2 repeats and the PCR product is 240 bp, allele 3 has 3 repeats and the PCR product is 326 bp, allele 4 has 4 repeats and the PCR product is 498 bp, allele 5 has 6 repeats and the PCR product is 584 bp.

Intergenic distances were determined by estimation based on the insert sizes of relevant PAC clones from a contig spanning the IL-1 gene cluster (Nicklin, et al., Genomics 19:382-4 (1994)*)*. Intragenic distances were determined from the relevant nucleotide sequence obtained form the GENBANK database.

### Example 2 Method for Estimating Linkage Disequilibrium

Because four of the markers studied herein are multiallelic, a preliminary analysis was carried out to determine which allelic combinations between pairs of loci contributed to the greatest disequilibrium, in order that the disequilibrium would not be masked when the alleles were grouped into biallelic systems. The E.H. program of Xie and Ott (Handbook of Human Genetic Link-age, 1994, John Hopkins University Press, 188-98), incorporated by reference herein, was used to estimate haplotype frequencies under H₀ (no linkage) and H₁ (allelic linkage allowed). It was found that the elaborate allele grouping, strategy had some advantages over commonly used methods, in that disequilibrium was detected between almost all pairwise combinations of markers examined and there was good correlation between disequilibrium and physical distance.

More specifically, the E.H. program of Xie and Ott was used to determine maximum likelihood estimates of disequilibriumn (Dⱼ) between each pairwise combination of alleles, where Dᵢⱼ = hᵢⱼ - pᵢqⱼ are the frequencies for allele i at locus 1 and allele j at locus 2 respectively, and hᵢⱼ is the frequency of the haplotype ij. The program calculated maximum likelihood values for the haplotype frequencies (and hence allele frequencies) under H₀ (no association) and haplotype frequencies under H₁ (allelic association allowed). For markers with greater than two alleles, the E. H. estimate for allele frequencies correlated poorly with the allele frequencies as estimated directly from the sample population, and therefore gave no confidence to the Dᵢⱼ estimates given. It was therefore necessary to group alleles of the multi-allelic markers into a biallelic system. Analysis of the markers in a biallelic format has the added advantages that the notation ^Dᵢⱼ, pⱼ, and qⱼ can be simplified to ^D, p, and q respectively, where p and q are defined to be the frequencies of the rarer alleles at both loci (such that without loss of generality p α q α 0.5), and ^D is the estimated disequilibrium between those alleles.

Under a biallelic system, power is also much simpler to determine using equations as detailed by Hill (Hill, Heredity, 33: 229-39 (1974)). In addition, the sign of ^D becomes informative, such that ^D > 0 when the rarer alleles at each of the two loci are associated, and ^D < 0 when the rare allele at one locus is associated with the common allele at the other locus.

Because the method of allele grouping clearly affected the power to detect disequilibriurn (Zouros, et al., Genet. 85: 543-50 (1977); Weir, et al., Genet. 88: 633-42 (1976)), a preliminary analysis was conducted to ensure that the grouping did not mask disequilibrium between subsets of alleles. In this analysis, δᵢⱼ = (O'ᵢⱼ - Eᵢⱼ)//Eᵢⱼ was calculated for each haplotype, where Eᵢⱼ is the expected number of haplotypes ij assuming equilibrium (Eᵢⱼ = 2n pᵢqⱼ, where n = number of individuals in the study), and O'ⱼⱼ is a basic estimate for the observed haplotype count determined as follows. All genotypes that could be unambiguously resolved were haplotype counted. Each double heterozygote (i₁i₂/j₁j₂) could be resolved into two possible haplotype sets, [i₁J₁,i₂j₂] or [i₁,J₂,i₂j₁]. Using the haplotype frequencies as estimated from the unambiguous haplotype count, the probability of each set was calculated and used as a "partial" count. In this way the ambiguous genotypes were also haplotype counted, and the total counts (ambiguous plus unambiguous) constituted the O'ᵢⱼ's used in δᵢⱼ. Once established, the magnitude and sign of the δᵢⱼ's were used to determine which allelic combinations showed greatest deviation from the null hypothesis of no association. This information was used to group alleles at the multiallelic loci into biallelic systems to enable efficient use of the E.H. program.

In order to compare the degree of disequilibrium between different pairwise combinations of loci, a frequency independent measure of disequilibrium ∼D, the proportion of maximum possible disequilibrium in the given direction) was calculated, where ∼D = ^D/|Dₘₐₓ| (Thompson, et al., Am. J. Hum. Genet. 42: 113-24 (1988)). The relationship between p and q are such that p α q α 0.5, and it can therefore be written that -pq α D α p(1-q) such that when ^D< 0, Dₘₐₓ = -pq and when ^D>0, Dₘₐₓ = p(1-q). Output from the E.H. program included log-likelihoods for the maximum likelihood parameter values under Hₒ and H₁, and since -21n (L_{O}/L₁)∼X²₁. where L_{O} and L₁ are the likelihoods under H_{O} and H₁, p- values could then be determined for each test.

The asymptotic variance for ^D, under H_{O}: D = 0 and H₁ were computed using the formula as defined by Hill (Heredity 33: 229-39 (1974)) for genotypic data. Using these, the power for each pairwise comparison could be calculated.

Common haplotypes containing all 8 loci were identified from the preliminary analysis of δᵢⱼ described above, and backed up by the magnitude and sign of the disequilibria once the alleles at the multiallelic loci had been grouped. For these loci, the allele in the group which contributed most to the disequilibriurn has been identified on the haplotype. To estimate the population haplotype frequencies, rates of carriage of at least one copy of the relevant alleles in the population were determined. These do not represent true haplotypes since phase is unknown. Monte Carlo simulation techniques were used to test for significant deviation from a simulated null distribution for these combined carriages under the assumption of no association.

### Example 3 Estimation of Lineage Disequilibrium in the IL-1 Gene Cluster

A number of biallelic and multiallelic markers in and around the IL-1 genes have been identified. However, the extent of linkage disequilibrium between the markers, and the prevalence of multimarker haplotypes in the general population have not until now been identified.

Figure 1 shows the relative positions of the 8 marker loci used in this study. DNA samples from 212 unrelated healthy volunteers were genotyped for each of these markers, and the resulting estimates of allele frequencies are shown in Table 5.

**Table 5. Estimated frequencies of marker alleles**

| 222/223 | freq. | gz5/gz6 | freq. | -889 | freq. | +3953 | freq. |
|---|---|---|---|---|---|---|---|
| 1(126 bp) | 0.005 | 1(79 bp) | 0.003 | 1(Ncol) | 0.714 | 1(2 Taql) | 0.812 |
| 2 (128 bp) | 0.018 | 2 (82 bp) | 0.005 | 2 | 0.286 | 2 | 0.188 |
| 3 (130 bp) | 0.378 | 3 (88 bp) | 0.676 | | | | |
| 4 (132 bp) | 0.299 | 4 (91bp) | 0.316 | | | | |
| 5 (134 bp) | 0.016 | | | | | | |
| 6 (136 bp) | 0.208 | | | | | | |
| 7 (138 bp) | 0.055 | | | | | | |
| 8 (140 bp) | 0.003 | | | | | | |
| 9 (142 bp) | 0.010 | | | | | | |
| 10 (144 bp) | 0.008 | | | | | | |
| *total | 384 | | 392 | | 398 | | 398 |
| -511 | freq. | gaat.p33330 | freq. | Y31 | freq. | VNTR | freq. |
| 1 | 0.618 | 1 (189 bp) | 0.658 | 1 (148 bp) | 0.092 | 1 | 0.744 |
| 2 (Bsu361) | 0.382 | 2 (193 bp) | 0.002 | 2 (158 bp) | 0.008 | 2 | 0.256 |
| | | 3 (197 bp) | 0.255 | 3 (160 bp) | 0.454 | | |
| | | 4 (201 bp) | 0.084 | 4 (162 bp) | 0.062 | | |
| | | | | 5 (164 bp) | 0.003 | | |
| | | | | 6 (166 bp) | 0.122 | | |
| | | | | 7 (168 bp) | 0.035 | | |
| | | | | 8 (170 bp) | 0.030 | | |
| | | | | 9 (172 bp) | 0.095 | | |
| | | | | 10 (174 bp) | 0.087 | | |
| | | | | 11 (176 bp) | 0.003 | | |
| | | | | 12 (178 bp) | 0.011 | | |
| | 398 | | 404 | | 370 | | 398 |

| | | | | | | | |
|---|---|---|---|---|---|---|---|
| *number of chromosomes analyzed Note - Allele names (and sizes) are given in boldface. | | | | | | | |

To determine the linkage disequilibria between pairwise combinations of loci, the computer program of Xie and Ott was used. This program was found to be most efficient when used with biallelic systems, therefore alleles at the multiallelic loci were grouped in the most appropriate way for each pairwise comparison, such that disequilibrium between subsets of alleles was not masked.

In Table 6, the disequilibria between pairs of loci are expressed as ∼D, the ratio of ^D to its maximum value Dₘₐₓ and are shown together with the approximate physical distances between the loci in kilobase pairs.

**Table 6. Disequilibrium (∼D = ^D/|Dₘₐₓ| ) and physical distances between markers**

| | | | | |
|---|---|---|---|---|
| | 222/223 | gz5/gz6 | -889 | +3953 |
| 222/223 | - | +0.872 | +0.829 | +0.710 |
| gz5/gz6 | 2.5 | - | -0.889 | -0.695 |
| -889 | 7 | 4.5 | - | +0.804 |
| +3953 | 55 | 55 | 50 | - |
| -511 | 60 | 60 | 55 | 4.5 |
| gaat.p33330 | 260 | 260 | 255 | 205 |
| Y31 | 310 | 310 | 305 | 255 |
| VNTR | 380 | 380 | 375 | 325 |
| | -511 | gaat.p33330 | Y31 | VNTR |
| 222/223 | +0.535 | +0433 | +0.364 | -0.499 |
| gz5/gz6 | +0.540 | +0.517 | -0.503 | +0.286 |
| -889 | -0.264 | +0.337 | +0.318 | -0.207 |
| +3954 | -0.617 | +0.409 | -0.475 | 0.439 |
| -511 | - | +0.691 | -0.456 | +0.448 |
| gaat.p33330 | 200 | - | +0.639 | +0.442 |
| Y31 | 250 | 50 | - | -0.765 |
| VNTR | 320 | 120 | 70 | - |

| | | | | |
|---|---|---|---|---|
| Note - disequilibrium values are shown at the top right, approximate physical distances in Kb are shown at the bottom left. Intergenic distances are given to the nearest 5 Kb. | | | | |

Table 7 shows the power to detect 50% Dₘₐₓ for each locus combination, and the p values for each corresponding D.

**Table 7. Power to detect 50% Dₘₐₓ and p values of -2Ln (L_{O}L₁)**

| | | | | |
|---|---|---|---|---|
| | 222/223 | gz5/gz6 | -889 | +3953 |
| 22/223 | - | -100(+) | -100(+) | 98(+) |
| gz5/gz6 | <1x10⁻¹⁰ | - | 87(-) | 60(-) |
| -889 | <1x10⁻¹⁰ | <3x10⁻⁸ | - | -100(+) |
| +3954 | -1x10⁻⁷ | *-9x10⁻³ | <1x10⁻¹⁰ | - |
| -511 | -9x10⁻¹⁰ | -4x10⁻¹⁰ | *-9.4x10⁻² | *-2.6x10⁻² |
| gaat.p3330 | -9x10⁻⁸ | -2x10⁻⁹ | *-1.7x10⁻² | -5x10⁻⁴ |
| Y31 | -1x10⁻⁴ | -4x10⁻⁴ | -6x10⁻⁴ | -1x10⁻⁷ |
| VNTR | -1x10⁻³ | -1x10⁻³ | *-3x10⁻¹ | *-1.2x10⁻¹ |
| | -511 | gaat.p33330 | Y31 | VNTR |
| 22/223 | ∼100(+) | ∼100(+) | ∼100(+) | 93(-) |
| gz5/gz6 | ∼100(+) | ∼100(+) | 98(-) | ∼100(+) |
| -889 | 96(-) | 89(+) | ∼100(+) | 78(-) |
| +3954 | 79(-) | 97(+) | ∼100(+) | 52(-) |
| -511 | - | ∼100(+) | ∼100(-) | ∼100(+) |
| gaat.p33330 | <1x10⁻¹⁰ | - | 49(+) | ∼100(+) |
| Y31 | ∼2x10⁻⁴ | ∼7x10⁻³ | - | 89(-) |
| VNTR | ∼8x10⁻⁶ | ∼1x10⁻⁹ | 2x10⁻⁷ | - |

| | | | | |
|---|---|---|---|---|
| Note - Power is shown at the top right with the sign of disequilibrium in brackets; pointwise p-values are shown (uncorrected) at the bottom left. For an overall significance level of p = 0.05, pointwise significance level is 0.0018 for 28 comparisons. * Not significant at p = 0.0018 threshhold | | | | |

Significant linkage disequilibrium (p_{corr} < 0.05) was detected between most combinations of loci, with only a few exceptions. These include the comparisons between the VNTR and the more distant biallelic markers, +3954, and -889, in which the disequilibrium is in the negative direction and consequently the power is reduced (Table 7). The correlation between disequilibrium ∼D and physical distance was r= -0.752 (p < 0.0001, one tailed) (Figure 2).

In order to compare different grouping methods for the multiallelic markers, ∼D was calculated for all the comparisons involving 222/223 using two additional grouping strategies. The first of these was a "common allele versus the rest" approach, and the second was a grouping based on allele size, using the bimodal distribution of allele frequency versus size which was observed for all the multiallelic markers examined. The results of this analysis are shown in Table 8, where ∼D values for the three grouping methods are compared.

**Table 8. ∼D values for three methods of grouping alleles at the multiallelic marker loci**

| | δᵢⱼ | common vs. rest | allele size |
|---|---|---|---|
| gz5/gz6 | 0.87 | 0.79 | 0.77 |
| -899 | 0.83 | 0.81 | 0.98 |
| +3954 | 0.71 | *0.74 | 0.77 |
| -511 | 0.54 | *0.15 | 0.61 |
| gaat.p33330 | 0.43 | *0.03 | 0.53 |
| Y31 | 0.36 | *0.12 | 0.16 |
| VNTR | 0.5 | 0.48 | *0.04 |

| | | | |
|---|---|---|---|
| Note- Values re given for the disequilibrium between 222/223 and the other markers listed. * indicates not significant at p = 0.05 level, even before correction for multiple testing. | | | |

It can be seen that the disequilibrium is not detected in several instances using these other grouping strategies, notably 222/223 with -511 and gaat.p33330 in the common versus rest approach, 222/223 with Y31 in both the common versus rest and allele size approaches, and 222/223 with VNTR in the allele size approach.

Examination of which alleles of the multiallelic loci were contributing greatest to the disequilibrium, from the determination of δᵢⱼ revealed the existence of 2 haplotypes containing alleles of all 8 loci. These were confirmed by examination of the haplotype frequencies and disequilibrium values obtained after the grouping. The first haplotype: alleles 44112332 (expressed in chromosome order, see Fig. 1) is the most common (carriage of 34/198), and is present 7 times more frequently than expected (expected = 4.5/198) (p < 0.000001). The second haplotype: alleles 33221461 (carriage of 2/206) was present 4 times more frequently than expected (expected 0.5/206), but this was not statistically significant (p - 0.106). However, examination of a larger sample size might assist in increasing the statistical significance of this finding.

The data presented indicate a significant degree of linkage disequilibrium across an approximately 400 Kb stretch of chromosome 2q13. The disequilibrium was strong both for the three markers within the IL-1α gene, as might be expected, but was also strong between some of the more distantly separated markers (-899/+3954;∼D = +0.804, physical distance = 50 Kb) (Table 6). However, ∼D was considerably diminished between the extreme ends of the cluster. Within the IL-1β gene, a moderate value of ∼D (+3954/-511; ∼D = -0.617) was obtained, although this was not significant when corrected for multiple comparisons, probably reflecting the reduction in power when disequilibriurn is in the negative direction (Thompson, et al, Am J Hum. Genet. 42: 113-24 (1988)).

Overall, there is a good correlation between physical distance and linkage disequilibrium (Figure 2); r = -0.752. The reliability of r itself depends partly on the reliability of the estimates of both physical distance and ∼D. Over the short distances, the physical distances are accurate since they are determined from known DNA sequence, whereas the longer range estimates are less precise. The power can be taken tentatively as one indicator of the reliability of ∼D, since if power is low this indicates that the sample size was too small, and with low sample sizes the estimates for ∼D may be unreliable.

The success of the elaborate grouping strategy is indicated by Table 8, which shows several instances where disequilibriurn between particular loci is apparently low or not detected when other commonly used grouping methods are employed. The disadvantages of the grouping strategy used here are that it is rather laborious since the information used for the grouping was based on an approximate estimate of the "observed" haplotype frequencies (see Example 2). For the more polymorphic markers the higher heterozygosity meant that the estimate of δᵢⱼ, was less precise since there was a higher proportion of ambiguous haplotypes. Notwithstanding this drawback, care was taken to take into account both the sign and magnitude of δᵢⱼ, and the frequencies of the alleles concerned.

The method could be simplified, in a sufficiently large study, by just considering the unambiguous haplotypes when determining the grouping. The determination of δᵢⱼ uses the maximum amount of prior knowledge for the grouping of the multiallelic markers, and this may be the reason why disequilibrium between almost all pairwise combinations of markers was detected.

The two haplotypes containing all 8 markers, as well as other shorter haplotypes, are of particular interest since it is likely that particular combinations of alleles of the IL- I genes may act in concert to determine an overall inflammatory phenotype. An understanding of which markers are in strong linkage disequilibrium not only allows for more rational design of genetic studies but also may provide clues to disease mechanism. Therefore, in addition to the alleles identified herein, the IL-1 (44112332) haplotype may contain the following alleles:
allele 2 of the 1731 marker of the IL1RN gene(A at position 173 1);
allele 2 of the 1812 marker of the IL1RN gene (A at position 1812);
allele 2 of the 1868 marker of the IL1RN gene(G at position 1868);
allele 2 of the 1887 marker of the IL1RN gene(C at position 1887);
allele 2 of the 8006 marker of the IL1RN gene (contains a HpaII or MspI site)
allele 2 of the 8061 marker of the IL1RN gene (lacks a MwoI site)
allele 2 of the 9589 marker of the IL1RN gene (contains an SspI site)

Furthermore, the following PCR primers may be used to amplify these alleles:
TTACGCAGATAAGAACCAGTTTGG (SEQ ID NO. 24)
TTTCCTGGACGCTTGCTCACCA (SEQ ID NO. 25)
   (used for 1731, 1812, 1868, and 1887)
TTCTATCTGAGGAACAACCAACTAGTAGC (SEQ ID NO. 26)
CACCAGACTTGACACAGGACAGGCACATC (SEQ ID NO. 27)
   (used for 8006)
CGACCCTCTGGGAGAAAATCCAGCAAG (SEQ ID NO. 28)
   (used with SEQ ID NO. 20 for 8006)
ACACAGGAAGGTGCCAAGCA (SEQ ID NO. 29)
TGCAGACAGACGGGCAAAGT (SEQ ID NO. 30)
   (used for 8006 and 9589)
TTGTGGGGACCAGGGGAGAT (SEQ ID NO. 31), and
AGCCTGGCACTCTGCTGAAT (SEQ ID NO. 32)
   (used for 9589).

### Example 4 The IL-1 (441123-32) Haplotype is Associated with Diabetic Nephropathy

The presence of the two haplotypes described herein was investigated in healthy and diseased populations to determine if the haplotypes were associated with inflammatory disease. 81 non-insulin dependant diabetes mellitus (NIDDM) patients with nephropathy were compared with 198 ethnically matched healthy subjects in example 3 and 147 NIDDM patients without nephropathy. Genotyping was carried out as in example 1.

The IL-1 (44112332) haplotype was carried by 24 of 79 of the NIDDM nephropathy patients and 25 of 141 NIDDM without nephropathy patients. However, the second haplotype (3322146 1) was not found in the nephropathy patients (0/8 1). The IL-1 (44112332) haplotype was significantly over represented in the patient group compared with the healthy control group (24/79 vs. 34/198; p = 0.015) and the NIDDM without nephropathy group (24/79 vs. 25/141; p = 0.03).

### Example 5 An IL-1 Haplotype is Associated with Inflammatory Disease

This is a prophetic example. Other diseases are examined as per Example 4. The IL-1 (44112332) haplotype is found to be associated with coronary artery disease, osteoporosis, nephropathy in diabetes mellitus, alopecia areata, Graves disease, systemic lupus erythematosus, lichen sclerosis and ulcerative colitis.

Likewise, the IL-1 (33221461) haplotype is associated with periodontal disease, juvenile chronic arthritis, psoriasis, insulin dependant diabetes and diabetic retinopathy.

### Example 6 Novel Markers are Linked to an IL-1 Haplotype

This is a prophetic example. Additional markers are identified by sequence and restriction enzyme analysis of the 2q13-14 region. These new markers are identified as belonging to an IL-1 haplotype in the manner described in Examples 2 and 3.

### Example 7 The IL-1 (44112332) Haplotype Is Used to Predict Disease

### Susceptibility

This is a prophetic example. A patient with a family history of ulcerative colitis is genotyped for the presence of the IL-1 (44112332) haplotype. Genotyping is performed as in Example I and the patient is determined to carry one or more alleles of the haplotype. The patient is thus treated with IL-1 antagonists to prevent disease.

A second patient with a family history of coronary artery disease is genotyped at the IL-1 gene cluster. The patient is found to carry one or more alleles; of the IL-1 (44112332) haplotype and be homozygous for the VNTR allele 2. Thus, the patient is 5.4 times as likely to develop coronary artery disease as the general population and is treated vigorously to prevent disease.

### Example 8 Additional Haplotypes are Statistically Significant

This is a prophetic example. An additional 400 chromosomes are typed as per Example 1 and linkage disequilibriurn assessed as per Example 2. The IL-1 (33221461) haplotype is found to be present about 4 times more frequently than expected (p - 0.05).

In a similar manner, the following markers are determined to be present in the IL- 1 (44112332) haplotype (p << 0.05).
allele 2 of the 1731 marker of the IL1RN gene
allele 2 of the 1812 marker of the IL1RN gene
allele 2 of the 1868 marker of the IL1RN gene
allele 2 of the 1887 marker of the IL1RN gene
allele 2 of the 8006 marker of the IL1RN gene
allele 2 of the 8061 marker of the IL1RN gene
allele 2 of the 9589 marker of the IL1RN gene

### Example 9. Identification and characterization of Single nucleotide polymorphisms in the human IL-1B gene associated with altered IL-1β expression and inflammatory conditions.

The IL-1B gene and other members of the IL-1 gene cluster were studied. In particular, IL-1B was investigated because several diseases are associated with SNPs in its upstream region. Several novel SNPs were identified, as shown in Tables 9A-E. Reporter gene assays were used to test allelic combinations of SNPs in the IL-1B promoter region, some of which represent frequent population haplotypes. A control construct has the wild-type (designated allele 1) at all SNP-containing alleles. Differential transcriptional activity of individual alleles in the reporter assays was further studied by analyzing binding of nuclear proteins to the allelic DNA in electromobility shift assays.

### High-Density SNP mapping

Twenty-five healthy volunteers of different, self-described, ethnicity were selected. Each subject gave informed, written consent and the protocol was approved by the institutional review board at the University of Sheffield (U.K.). Genomic DNA was prepared from blood samples. The DNA was sent to Genome Therapeutics Corporation (Waltham, MA, USA) for sequencing and high density SNP mapping. Publicly available genomic sequence across the IL-1 region to were utilized to establish the reference sequence of this region for SNP identification. In addition, a commercially available BAC library was screened to identify BAC clones spanning the IL-1 region. Positive BAC clones were used to generate a small insert library of plasmids with 3 to 3.5 kb inserts. Four genes (IL-1A, IL-1B, IL-RN and IL-1F5) were interrogated and SNPs were identified for each gene in the exon regions and 5 kb upstream of the gene. SNP detection was performed automatically using the PolyPhred program on an ABI 3700 instrument, followed by visual inspection of the identified SNPs listed in Tables 9A-E.

**Table 9A. IL-1α SNP discovery**

| **SNP#** | **Relative to +1** | **Location** | **Type** | **SNP Sequence** |
|---|---|---|---|---|
| **1** | -3885 | Promoter | **C/T** | GCATGAGCCA\**C**GGCACCCAGCCACT (SEQ ID NO: ) |
| **2** | -3764 | Promoter | **C/T** | TGAACTAGAA\**C**TCAAGAAATTGA (SEQ ID NO: ) |
| **3** | -3726 | Promoter | **T/C** | CACACTCTCA\**T**ATGAATTCTCCAT (SEQ ID NO: ) |
| **4** | -3608 | Promoter | **G/A** | CATATTCTGG/**A**ACCTTCAATAAA (SEQ ID NO: ) |
| **5** | -3115 | Promoter | **C/T** | *AAAAGTTATG\**T**TTTTCTCTTCATTCA* (SEQ ID NO:.) |
| **6** | -2756 | Promoter | **C/T** | TCTTTATAAG\**C**CATCACTTGGTG (SEQ ID NO: ) |
| **9** | -2269 | Promoter | **G/A** | *CGAGAGGTGG*/***G**TGCCTGAAGCCACC **(SEQ ID NO: )*** |
| **10** | -2118 | Promoter | **C/T** | TGTTCACAGT/**C**CCAGAAAAGCGGGC (SEQ ID NO: ) |
| **11** | -1506 | Promoter | **C/T** | TAAAGAGGAA/**C**CAAGGTAAGCAGAA (SEQ ID NO: ) |
| **12** | -1202 | promoter | **C/T** | ACACAAGCTG\**C**TTTCCTCCCAGATC (SEQ ID NO: ) |
| **13** | -889 | Promoter | **C/T** | CCAGGCAACA\**C**CATTGAAGGCTC (SEQ ID NO: ) |
| **14** | +145 | Promoter | **C/T** | AAAGCTACAG/**C**CTCTCCTTTCTTT (SEQ ID NO: ) |
| **15** | +251 | Promoter | **T/A** | CTGATTCGTT\**T**TACTGAGGGACG (SEQ ID NO: ) |
| **16** | +263 | Promoter | **G/A** | ACTGAGGGAC\**G**GCAGAACTAGTTTC (SEQ ID NO: ) |
| **17** | +1864 | Promoter | **A/C** | CTTGAATCTT\**A**AATACTTTTGTT (SEQ ID NO: ) |
| **18** | +1892 | Promoter | **G/A** | CTCACTAGAG\**G**TCCAGAGACCT (SEQ ID NO: ) |
| **19** | +2822 | Promoter | **G/A** | AAGAAGAGAC\**G**GTTGAGTTT (SEQ ID NO: ) |
| **24** | +4718 | Promoter | **A/C** | TCTGGATTGG\**A**ATATTCCTA (SEQ ID NO: ) |
| **25** | +4728 | Promoter | **T/C** | ATTCCTAATA/**T**CCCCTCCAG (SEQ ID NO: ) |
| **26** | +4845 | Intron 1 | **G/T** | GCCTAGGTCA\**G**CACCTTTTAG (SEQ ID NO: ) |
| **30** | +6627 | Intron 1 | **G/A** | GCCCCCACCT\**G**CCCACCCCA (SEQ ID NO: ) |
| **31** | +8811 | Intron 1 | **A/C** | CCTTTTTCTA/**A**CATCTTGTTCTCTA (SEQ ID NO: ) |
| **32** | +9460 | Intron 3 | **G/A** | TTTGCCTTCT\**G**CTTTTAAGTT (SEQ ID NO: ) |
| **33** | +9613 | Intron 3 | **C/T** | AAATACTTCT/**C**GAAGCCGAGC (SEQ ID NO: ) |
| **34** | +9749 | Exon 4 | **C/G** | CTGAGTGTGA\**C**CAGGCATCCTC (SEQ ID NO: ) |

**Table 9B. IL-1β SNP discovery**

| **SNP#** | **Relative to +1** | **Location** | **Type** | **SNP Sequence** |
|---|---|---|---|---|
| **1** | -5164 | Promoter | **T/C** | GCCTGGGTCC/**C**AGACTTGACAAA (SEQ ID NO: ) |
| **2** | -3959 | Promoter | **T/C** | AGAAAAGACA/**T**AGAGTAGGA (SEQ ID NO: ) |
| **3** | -3893 | Promoter | **G/A** | TCCAAAGGAA/**G**GACAAGGTC (SEQ ID NO: ) |
| **4** | -3737 | Promoter | **C/T** | GGGAGGAGAATGGAATGT/**C**CCTTGGACTCT (SEQ ID NO: ) |
| **5** | -3686 | Promoter | **A/G** | GAAGAAGCCC/**A**TTGGAGATGATG (SEQ ID NO: ) |
| **6** | -3254 | Promoter | **T/C** | GATAACTGGC/**T**GCGAAGCCCATGAT (SEQ ID NO: ) |
| **7** | -2905 | Promoter | **A/G** | GGAAGACAGG/**A**TCTGATACATAC (SEQ ID NO: ) |
| **8** | -2394 | Promoter | **G/A** | CCTGTCACTG/**G**CTTTGATCCTCCTT (SEQ ID NO: ) |
| **9** | -2378 | Promoter | **G/A** | ATCCTCCTTC/**G**TTCAGCTTGTAATC (SEQ ID NO: ) |
| **10** | -1468 | Promoter | **G/C** | CACTCCCTTG/**G**ATAATGCAGAGCGAG (SEQ ID NO: ) |
| **14** | -511 | Promoter | **C/T** | AGAGAGCTCC/**T**GAGGCAGAGAAC (SEQ ID NO: ) |
| **15** | -31 | Promoter | **T/C** | TTTTGAAAGC/**C**ATAAAAACAGCGAGGGAG (SEQ ID NO: ) |
| **17** | +45 | Exon 1 5'UTR | **T/C** | GCTCTGGGAT/**T**CTCTTCAGCCAATCTTCAT (SEQ ID NO: ) |
| **19** | +746 | Intron 2 | **C/T** | ACTACTTTCC/**C**ATTACAAGTCCCTCCAG (SEQ ID NO: ) |
| **24** | +3876 | Intron 4 | **G/A** | AAATTTTGCC/**G**CCTCGCCTCACGAG (SEQ ID NO: ) |
| **25** | +3954 | Exon 5 | **C/T** | CTATCTTCTT/**C**GACACATGGGATAACG (SEQ ID NO: ) |
| **30** | +5548 | Intron 6 | **G/A** | CCTTCTCCCC/**G**CCCCCATCCCTAGG (SEQ ID NO: ) |
| **35** | +6506 | Exon 7 3'UTR | **C/A** | ATAGCCTGGA/**C**TTTCCTGTTGTCT (SEQ ID NO: ) |
| **37** | +691 | Exon 7 3' UTR | **G/C** | CTTTAATTAA/**G**ACT**G**AAAATATATAAGC (SEQ ID NO: ) |
| **38** | +7214 | Unknown | **C/T** | CAGTGCACAT/**T**TGGAACAGGATC (SEQ ID NO: ) |

**Table 9C. IL-RNic SNP discovery**

| **SN P#** | **Relative to +1** | **Location** | **Type** | **SNP Sequence** |
|---|---|---|---|---|
| **14** | -4864 | | **G/A** | GTTGGGTCAC/**G**TACCCGACGTGCTA (SEQ ID NO: ) |
| **15** | -4744 | | **T/G** | ACTGTTCACA/**G**AGCCAAGATATGGA (SEQ ID NO: ) |
| **18** | -3662 | | **T/G** | *TGTTTGCTTGl**T**TCTTCTCTCTCAGC **(SEQ ID NO: )*** |
| **19** | -3417 | | **A/G** | AGCTGGGTCT/**A**TGAGTTGTGGTGGC (SEQ ID NO: ) |
| **21** | -3230 | | **C/T** | GTGTGTGTGT/**C**TGTGTTTGTGTGTG (SEQ ID NO: ) |
| **22** | -3188 | | **G/A** | GAGAGAATGA/**G**AATATGAGTGGTGG (SEQ ID NO: ) |
| **25** | -2448 | | **T/C** | TATCTTGCTC/**T**TCCATTCCTGATGC (SEQ ID NO: ) |
| **26** | -2343 | | **T/C** | GTCACCATCA/**T**TGGGGTTGTGGATC (SEQ ID NO: ) |
| **29** | | Promoter | **C/T** | TGAGCCAAGG/**C**GGAAGAGAACAGGA (SEQ ID NO: ) |
| **30** | -1002 | Promoter | **T/C** | GCAGATAGCA/**T**CAGGTCCATTTTGC (SEQ ID NO: ) |
| **31** | -501 | Promoter | **G/C** | CTCTCAGAGA/**G**GGCTTCCCTGGCCA (SEQ ID NO: ) |
| **32** | -500 | Promoter | **G/A** | TCTCAGAGAG/**G**GCTTCCCTGGCCAC (SEQ ID NO: ) |
| **33** | -388 | Promoter | **C/T** | TATTTTATTTG/**C**TAACTTGTTTCTTG (SEQ ID NO: ) |
| **34** | -252 | Promoter | **A/C** | GCACACATGC/**A**TGAGCTGGCGGCAG (SEQ ID NO: ) |
| **35** | -41 | Promoter | **G/A** | GGGAGGGGAG/**G**CTGGGCTCCTCCTT (SEQ ID NO: ) |
| **36** | +40 | Exon 1 5' UTR | **G/A** | CCCAGGTACT/**G**CCCGGGTGCTACTT (SEQ ID NO: ) |
| **37** | +96 | Exon 1 5' UTR | **G/A** | GGAAGACCTC/**A**GAAGACCTCCTGTC (SEQ ID NO: ) |
| **38** | +115 | Exon 1 5' UTR | **G/C** | CCTGTCCTAT/**G**AGGCCCTCCCCATG (SEQ ID NO: ) |
| **39** | +162 | Intron 1 | **T/C** | *TCTCATTTTT*/***T**CACCTGAGAAATGA **(SEQ ID NO: )*** |
| **40** | +304 | Intron 1 | **A/G** | GGAGGCATCC/**A**TGGGAGACCATGCA (SEQ ID NO: ) |
| **42** | +2027 | Intron 1 | **G/T** | GTGCATACTC/**T**GACTGGAAACTGGA (SEQ ID NO: ) |
| **43** | +2060 | Intron 1 | **G/C** | AAAGGATAGA/**G**ATGGAACCATGTGC (SEQ ID NO: ) |
| **44** | +2245 | Intron 1 | **A/C** | AGGGTAAATT/**A**TTTTTAGGATCCAA (SEQ ID NO: ) |
| **61** | +11732 | Exon 3 | **T/C** | AACTAGTTGC/**T**GGATACTTGCAAGG (SEQ ID NO: ) |
| **62** | +11787 | Intron 3 | **T/C** | GCCAGGAAAG/**C**CAATGTATGTGGGC (SEQ ID NO: ) |
| **63** | +11797 | Intron 3 | **G/A** | CAATGTATGT/**G**GGCATCACGTCACT (SEQ ID NO: ) |
| **64** | +11819 | Intron 3 | **G/C** | CACTTTGCCC/**G**TCTGTCTGCAGCAG (SEQ ID NO: ) |
| **65** | +11860 | Intron 3 | **T/G** | TGCACAAACCCTAGG/**T**CAATGTCCTAATC (SEQ ID NO: ) |
| **66** | +11855 | Intron 3 | **A/C** | AAACCCTAGG/**T**GCAATGTCCTAATC (SEQ ID NO: ) |
| **67** | +11890 | Intron 3 | **T/C** | TGTATTCAAG/**T**TTGAAGCTGGGAGG (SEQ ID NO: ) |
| **68** | +11892 | Intron 3 | **G/A** | ATTCAAGTTT/**G**AAGCTGGGAGGGCC (SEQ ID NO: ) |
| **76** | +13070 | Intron 3 | **G/A** | AAAAATACCC/**G**GGGTCTCTTCATTA (SEQ ID NO: ) |
| **77** | +13092 | Intron 3 | **T/C** | TTATTGCTGC/**T**TCCTCTTCTATTAA (SEQ ID NO: ) |
| **78** | +13307 | Intron 3 | **A/T** | AAACAACCAA/**A**ATTTTTTCTTATGA (SEQ ID NO: ) |
| **79** | +13414 | Intron 4 | **T/A** | GCAGAGTGCC/**T**GGCTTGCGCTGGGC (SEQ ID NO: ) |
| **80** | +13520 | Intron 4 | **T/G** | GCATGGCGGC/**T**GACTTCCAAAAGGG (SEQ ID NO: ) |
| **81** | +13575 | Intron 4 | **G/A** | CGCTTATTAT/**G**ACTTCTGCTTGCAT (SEQ ID NO: ) |
| **82** | +13614 | Intron 4 | **C/T** | AAGCCAGTCA/**C**GTGGCTAAGTCTAG (SEQ ID NO: ) |
| **89** | +14818 | Exon | **T/C** | GCTCAGACAG/**C**GGCCCCACCACCAG (SEQ ID NO: ) |

**Table 9D. IL-RNsec SNP discovery**

| **SN P#** | **Relative** | **Location** | **Type** | **SNP Sequence** |
|---|---|---|---|---|
| **61** | +2014 | Exon 2 | **T/C** | AACTAGTTGC/**T**GGATACTTGCAAGG (SEQ ID NO: ) |
| **62** | +2069 | Intron 2 | **T/C** | GCCAGGAAAG/**C**CAATGTATGTGGGC (SEQ ID NO: ) |
| **63** | +2080 | Intron 2 | **G/A** | CAATGTATGT/**G**GGCATCACGTCACT (SEQ ID NO: ) |
| **64** | +2101 | Intron 2 | **G/C** | CACTTTGCCC/**G**TCTGTCTGCAGCAG (SEQ ID NO: ) |
| **66** | +2142 | Intron 2 | **A/C** | AAACCCTAGG/**T**GCAATGTCCTAATC (SEQ ID NO: ) |
| **67** | +2180 | Intron 2 | **T/C** | TGTATTCAAG/**T**TTGAAGCTGGGAGG (SEQ ID NO: ) |
| **68** | +2183 | Intron 2 | **G/A** | ATTCAAGTTT/**G**AAGCTGGGAGGGCC (SEQ ID NO: ) |
| **76** | +3192 | Intron 2 | **G/A** | AAAAATACCC/**G**GGGTCTCTTCATTA (SEQ ID NO: ) |
| **77** | +3214 | Intron 2 | **T/C** | TTATTGCTGC/**T**TCCTCTTCTATTAA (SEQ ID NO: ) |
| **78** | +3428 | Intron 2 | **A/T** | AAACAACCAA/**A**ATTTTTTCTTATGA (SEQ ID NO: ) |
| **79** | +3535 | Intron 3 | **T/A** | GCAGAGTGCC/**T**GGCTTGCGCTGGGC (SEQ ID NO: ) |
| **80** | +3641 | Intron 3 | **T/G** | GCATGGCGGC/**T**GACTTCCAAAAGGG (SEQ ID NO: ) |
| **81** | +3696 | Intron 3 | **G/A** | CGCTTATTAT/**G**ACTTCTGCTTGCAT(SEQ ID NO: ) |
| **82** | +3735 | Intron 3 | **C/T** | AAGCCAGTCA/**C**GTGGCTAAGTCTAG (SEQ ID NO: ) |
| **89** | +4939 | Exon 4 | **T/C** | GCTCAGACAG/**C**GGCCCCACCACCAG (SEQ ID NO: ) |
| **90** | +5221 | Exon 4 3' UTR | **G/C** | GGCGTCACAA/**C**AACCTGGTCACAGG (SEQ ID NO: ) |
| **91** | +6047 | Exon 4 3'UTR | **G/A** | CTCCCCCACC/**A**GGCTGGGAGCTCTG (SEQ ID NO: ) |
| **93** | +6410 | | **T/C** | GCAAAAAAGA/**T**ATGGGGCAGCACTG (SEQ ID NO: ) |
| **94** | +6440 | | **A/G** | AACAGCCTCT/**A**CTGGAAACAACCCA (SEQ ID NO: ) |
| **95** | +6179 | | **A/G** | AAAGTTCCCT/**A**CTTCCTGTGACTTC (SEQ ID NO: ) |

**Table 9E. IL-1F5 SNP discovery**

| **SNP#** | **Relative to +1** | **Location** | **Type** | **SNP Sequence** |
|---|---|---|---|---|
| **15** | -2346 | Promoter | **T/C** | ATTCAGGGAA/**T**GGAGGCTTAT (SEQ ID NO: ) |
| **16** | | Promoter | **T/C** | AGAGAACTTA/**T**GTTGGTGAGG (SEQ ID NO: ) |
| **17** | -2063 | Promoter | **A/G** | TTGACAGTAT/**A**CACAGTCATA (SEQ ID NO: ) |
| **18** | -1953 | Promoter | **C/T** | CTCACTGCAA/**C**CTCCACCTCC (SEQ ID NO: ) |
| **19** | | | A/T | *TGCAACCTCC*/***A**CCTCCCAGGT **(SEQ ID NO: )*** |
| **20** | -1834 | Promoter | **C/T** | GGTTTTGCCA/**C**GTTGGCCAGG (SEQ ID NO: ) |
| **21** | -1019 | Promoter | **C/T** | *GCTATTTTGC*/***C**ACATGAAAAA **(SEQ ID NO: )*** |
| **22** | -926 | Promoter | **T/A** | GTGGAGGAAA/**T**AGTGTTGTCAT (SEQ ID NO: ) |
| **23** | | | **G/C** | GGAGGAAATA/**G**TGTTGTCATTG (SEQ ID NO: ) |
| **24** | | | **G/A** | GAAATGGGAA/**G**GGAAAGAAG (SEQ ID NO: ) |
| **25** | | UTR | **T/C** | AAAGT\**C**GCGCTTCCCTGAGC (SEQ ID NO: ) |
| **26** | | | **C/T** | GCCAGGATCC/**C**TGCTCAGAGCTCCG (SEQ ID NO: ) |
| **27** | | Intron | **T/C** | CTGAGGGGGA/**T**AAGTGAACCTCCCA (SEQ ID NO: ) |
| **28** | | Intron | **T/C** | CAGCACCTCA/**C**TGCTCTTAGAGTTT (SEQ ID NO: ) |
| **29** | | Intron | **G/A** | CTGTGCCCCT/**A**CCTCCCTGCTACTC (SEQ ID NO: ) |
| **30** | | Intron | **T/C** | TTACCATCAG/**T**AGTCTGTCCA (SEQ ID NO: ) |
| **31** | | Intron | **A/T** | TTCTGGGCTT/**A**CAACAGAGGA (SEQ ID NO: ) |
| **32** | | Intron | **T/C** | GCACTTCCCA/**T**ATGTGTTCTA (SEQ ID NO: ) |
| **33** | | Intron | **G/T** | ATTGGAGGCA/**G**GATGGGGCAA (SEQ ID NO: ) |
| **34** | | Intron | **G/A** | TTCTCTTCCA/**G**AGGAGATGGG (SEQ ID NO: ) |
| **35** | | Intron | **G/A** | CCCAGGCATT/**G**GGGCAGCACAAACC (SEQ ID NO: ) |
| **36** | | Intron | **G/C** | TGAAGACACA/**G**GCTCTGGGGCCACC (SEQ ID NO: ) |
| **37** | | Intron | **G/A** | TGAAGCTTCT/**A**TGCAGAAGTGGGAA (SEQ ID NO: ) |
| **38** | | Intron | **G/A** | TGCAGAAGTG/**G**GAATGTTTTCCAAG (SEQ ID NO: ) |
| **39** | | Intron | **G/C** | CACTGCAACA/**G**TCCCTCTCTA (SEQ ID NO: ) |
| **40** | | Intron | **T/C** | CAGGCCGTCT/**T**ACAGCAGTCCTGTG (SEQ ID NO: ) |
| **41** | | | **A/G** | GTGGTCCCCA/**A**TCGGTGGCTGGATG (SEQ ID NO: ) |
| **42** | | UTR | **G/A** | ATTTCTGTGG/**G**GTGGGGTGGG (SEQ ID NO: ) |
| **43** | | UTR | **C/G** | TGAGGAGGTG/**G**TGATGTCAGAAGAA (SEQ ID NO: ) |
| **44** | | | **G/A** | GGTGGTGGCA/**G**TATAGGTGATTTTT (SEQ ID NO: ) |
| **45** | | UTR | **T/C** | TGTTAATTTA/**T**CTGTATTTCCTAAT (SEQ ID NO: ) |
| **46** | | UTR | **G/C** | TTAATCTTGA/**G**GTAAGCAGAGTAGA (SEQ ID NO: ) |
| **47** | | UTR | **T/C** | GTAAGCAGAG/**T**AGACATCATCTCTGA (SEQ ID NO: ) |
| **48** | | UTR | **A/C** | GGAGGCTGTG/**A**TGAGTTTGTGTGGC (SEQ ID NO: ) |
| **49** | | UTR | **T/C** | ACAGAGGAGA/**C**GCGGGGAAGACTAT (SEQ ID NO: ) |
| **50** | | UTR | **C/T** | TAATTGGTTA/**T**GGCAGCTCTAGGAA (SEQ ID NO: ) |
| **51** | | UTR | **A/G** | AATTGGTTAC/**G**GCAGCTCTAGGAAA (SEQ ID NO: ) |
| **52** | | | **T/A** | TTACAGCAGC/**T**CTAGGAAACTAATA (SEQ ID NO: ) |
| **53** | | | **C/A** | TACAGCAGCT/**C**TAGGAAACTAATAC (SEQ ID NO: ) |
| **54** | | UTR | **G/T** | GAAGCTAGCT/**T**CCATGCTATGAGCA (SEQ ID NO: ) |
| **55** | | UTR | **C/T** | AAGAGACTTA/**T**GTGGTAAAAAATGAA (SEQ ID NO: ) |
| **56** | | UTR | **G/A** | TGAGATAATC/**A**ATGTTTGTTGTTTT (SEQ ID NO: ) |
| **57** | | UTR | **T/C** | TTTGTTGAGT/**C**ATCTGGTTCTGCAA (SEQ ID NO: ) |

In the IL-1A gene, the mapping revealed a total of 25 SNPs. Two SNPs were in the exon regions and resulted in non-synonomous changes. One of these in Exon 5 was public and found at position +4845, the other in Exon 4 was at low (2%) frequency and later attempts to confirm in another population showed no variation. Of the 11 SNPs in the promoter region, 3 were found in only one subject (2%) and the other 8 were also in the public database (NCBI dbSNP). The remaining 12 SNPs were either intronic (n=9) or found in the 3' untranslated region (n=3) of Exon 7. Ten of these were also found in the public database and the remaining 2 were at very low frequency (2-4%) in our population.

To identify variants in the IL-1B (IL-1F2) gene that are most likely to be functional, the seven exons and adjacent intronic regions, 5 kb upstream of the gene, and 2 kb in the 3' region, comprising 12 kb, were sequenced in a panel of 25 ethnically diverse, healthy individuals. Twenty SNPs were identified (Table 9B). It is notable that in seven exons sequenced only one SNP was identified in a coding region, the well-described synonymous SNP at +3954. There were three intronic SNPs, three SNPs in the 3' UTR of exon 7, and one SNP in the 5' UTR of exon 1 identified. The remaining 12 SNPs were in the promoter/enhancer region. Two of the 12 promoter SNPs were not in public databases and six of the 12 had minor allele frequencies ≤4%. The presence of 5 previously reported SNPs at positions -511 (SNP 14), -31 (SNP15), -1468 (SNP10), +3954 (SNP 25), and +3877 (SNP 24) in the IL-1B gene was confirmed. These SNPs have been associated with clinical conditions. Also the occurrence of five other SNPs was identified: -3893 (SNP3); -3737 (SNP 4), +5548 (SNP30) and +6911 (SNP37), and +7214 (SNP 38).

### Screening functional SNPs

There were no non-synonymous SNPs identified in the IL-1B exons. SNPs were identified that altered transcriptional activity and/or transcription factor binding. By setting all SNPs to allele 1 except for the individual SNP of focus, screening of promoter activity following transfection of the SNP constructs into THP-1 cells was performed. Transfection analysis with varied-length parental constructs showed that deletion of the distal fragment between SNP 1 and SNP 10 enhanced promoter activity, indicating strong repressors within this region. Stronger promoter activity of plasmids containing this region between SNP 17 and the start of the exon also revealed the existence of enhancer(s) in the 5'UTR In order to avoid interference of the repressors and enhancers while identifying individual functional SNPs in the promoter region, SNPs 2 to 17 were interrogated in the background of plasmids with and without the 5' UTR region downstream of SNP 15 (potential enhancer). SNPs 10 to 17 were also interrogated in the plasmid backgrounds with and without potential strong interference from upstream promoter regions. Because the initial BAC clone was found to contain allele 2 at both SNPs 14 and 15, we also screened individual SNPs for transcriptional activity on this background. The results reported here utilized the longer promoter plasmid containing upstream sequences including SNP 2.

The SNPs were annotated and schematically formatted with respect to their position within the gene, cDNA, and protein as a basis for developing a clear strategy for probing allele specific differences in gene function.

Five functional SNPs; B4, B7, B10, B14 and B15, were identified by demonstrating allelic differences in transcription. SNPs B4, B10, B14 and B15 occur in high frequency (>25%) in the population used for SNP discovery; while B7 was found in only 2% of subjects sequenced.

### Generation of IL-1B gene promoter constructs

Human *IL1B* promoter was amplified by PCR from BAC clone RP11-67L14 (IL1B, BAC PAC Resources, Children's Hospital Oakland Research Institute) using *Pfu* turbo DNA polymerase (Stratagene, CA). Two primer sets were used to amplify the *IL-1B* promoter containing different 3' ends. Primers IL1BF1 5' cccACGCGTGAGTGAAAGGAATCCCGTTAGAAGT (SEQ ID NO: 33) and IL1BR2 5'cccACGCGTGCCTGTTGTGCCTTGTGCCTCGAAG (SEQ ID NO: 34) were used to generate *IL-1B* promoter fragment IL-1BS (+32 to -5326) (S = short; without the first exon). IL1BR1 5' cccACGCGTGGCTGCTTCAGACACCTGTGTA (SEQ ID NO: 35) was used to generate promoter fragment IL-1BL (+471 To -5326) (L=long; containing the first exon and SNP 17 (+45)). Amplified *IL-LB* promoter fragments were cloned into pCR-Blunt II-TOPO vector using the Zero Blunt TOPO PCR cloning Kit (Invitrogen, CA), and then subcloned into pGL3-Basic vector (Promega, WI) upstream of the luciferase reporter gene to generate parental constructs with differing lengths of the promoter region. The sequencing of BAC DNA clones revealed that the SNPs at positions 14 and 15 were the minor alleles. These SNPs were sequentially converted to the major allele sequences by site-directed mutagenesis so that the parental serial constructs for both a longer plasmid (containing SNPs 2-15 by utilizing the Kpnl site just upstream of SNP 2) and a short plasmid (containing the promoter region out to the BamH1 site between SNPs 9 and 10) were uniformly allele 1 at all of the 13 SNPs (12 in 5' flanking region and 1 in the first exon). Site-directed mutagenesis of individual SNP to allele 2 sequences was carried out by QuikChange XL site-directed mutagenesis kit (Stratagene, CA). Successful mutagenesis of the *IL-1B* promoter was confirmed by sequencing analysis and re-cloned into the original pGL3-basic vector to avoid possible mutations introduced by PCR in the vector. DNA used in cell transfection was prepared by the EndoFree Plasmid Maxi kit (Qiagen, CA) to ensure minimal endotoxin contamination. IL-1B SNP construct genotypes are listed in Table 6.

### Cell Culture and Reagents

The human monocytic cell line, THP-1, was obtained from American Type Culture Collection (ATCC, VA). THP-1 cells were grown in RPMI 1640 medium (ATCC, VA) supplemented with 10% fetal bovine serum (Hyclone, UT). Cells were subcultured every other day and maintained at a cell density between 2 × 10⁵ to 7 × 10⁵ cells/ml. Lipopolysaccharide (LPS; from Escherichia coli 055:B5) and phorbol-12-myristate-13-acetate (PMA) were obtained from Sigma (MO). Anti-NF-κB p50 (H-119) and anti-NF-κB p65(c-20) were purchased from Santa Cruz Biotechnology, Inc. (CA)

### Cell Transfection and Reporter Activity Assay

Prior to transfection, THP-1 cells were counted, washed, and re-suspended in plain RPMI medium at 1.5 - 2 × 10⁷ cells/ml. For transfection, 200 ng pGL-IL1 promoter constructs and 6.25 ng phRL-TK (Promega, WI) were mixed with 750 µl RPMI, 50 µl 1 M TRIS pH 7.4, 100 µl DEAE-dextran (2mg/ml) in phosphate buffered saline, and 100 µl THP-1 cells. The mixtures were incubated at 37 °C for 30 minutes. Cells were spun down, washed, re-suspended in 0.5-ml culture medium, and transferred into 24-well culture dish. Eighteen to twenty-four hours post transfection, cells were stimulated by phorbol-12-myristate-13-acetate (PMA, 20 ng/ml) and lipopolysaccharide (LPS) for 6 hours. Cells were then lysed in 100 µl passive lysis buffer (Promega, WI). Cell lysate (20 µl) was analyzed for both the firefly and *Renilla* luciferase activities using the dual-luciferase reporter assay system (Promega, WI). Luciferase activity was measured by 1450 MicroBeta luminometer (PerkinElmer, MA). Promoter activity was normalized to *Renilla* activity from co-transfected phRL-TK. Each construct was transfected and assayed for promoter activity in triplicate and transfection for each construct was performed at least three times.

### Electromobility Shift Assay (EMSA)

THP-1 cells were cultured as described above and either harvested without treatment or treated with 100 ng/ml of LPS and 20 ng/ml of PMA for various time periods. Nuclear extracts were prepared according to Andrews N.C. and Faller D.V.³¹ Briefly, cells were resuspended in cold buffer A (10 mM Hepes-KOH, pH 7.9 - 1.5 mM MgCl₂ -10 mM KCl - 0.5 mM DTT - 2 mM AEBSF) and incubated on ice for 10 minutes. Nuclear pellets were then re-suspended in cold buffer C (20 mM Hepes-KOH, pH 7.9 - 25% glycerol - 420 mM NaCl -1.5 mM MgCl₂ - 0.2 mM EDTA - 0.5 mM DTT - 2 mM AEBSF) for 30 minutes on ice. After centrifugation for 2 minutes at 4°C, nuclear extracts were stored at -80°C. Oligonucleotides of each SNP were synthesized (MWG Biotech, High Point, NC) and purified by 10% polyacrylamide gel. Complementary single-stranded oligonucleotides were annealed and labeled with α-³²P-dGTP by Klenow (exo-) fill-in. For protein-DNA binding, 7 µg of nuclear extracts were incubated with 5.0 X 10⁴ cpm of ³²-P labeled probes for 20 minutes at room temperature in a volume of 20 µl binding buffer (12 mN Hepes-KOH, pH 7.9 - 1.5 mM MgCl₂ - 60 mM KCl - 0.2 mM EDTA - 0.5 mM DTT - 0.4 mM AEBSF - 10% glycerol) and 2 µg of Poly (dI-dC) (Sigma, St. Louis, MO). Protein-DNA complexes were resolved on 4% polyacrylamide gel and visualized by exposure to x-ray films. For cold oligonucleotide competition assays, nuclear extracts were incubated with cold probe for 10 minutes at room temperature before the addition of labeled probe. In antibody supershift aassays, 1-2 µg of antibody was added after binding of nuclear extracts to labeled probe, and was incubated for 15 minutes at room temperature before gel loading. Sequences of oligonucleotides are as follows:

| Oligo name | 5' - 3' |
|---|---|
| B4C | cggagaatggaatgtcccttggactctgcatga (SEQ ID NO: ) |
| B4C-r | cgcgtcatgcagagtccaagggacattccattctccgagct (SEQ ID NO: ) |
| B4T | cggagaatggaatgttccttggactctgcatga (SEQ ID NO: ) |
| B4T-r | cgcgtcatgcagagtccaaggaacattccattctccgagct (SEQ ID NO: ) |
| B10G | ctcactcccttggataatgcagagcgagca (SEQ ID NO: ) |
| B10G-r | cgcgtgctcgctctgcattatccaagggagtgagagct (SEQ ID NO: ) |
| B10C | ctcactcccttgcataatgcagagcgagca (SEQ ID NO: ) |
| B10C-r | cgcgtgctcgctctgcattatgcaagggagtgagagct (SEQ ID NO: ) |
| B14C | ccaattgacagagagctcccgaggcagagaacagca (SEQ ID NO: ) |
| B14C-r | cgcgtgctgttctctgcctcgggagctctctgtcaattggagct (SEQ ID NO: ) |
| B14T | ccaattgacagagagctcctgaggcagagaacagca (SEQ ID NO: ) |
| B14T-r | cgcgtgctgttctctgcctcaggagctctctgtcaattggagct (SEQ ID NO: ) |
| B15T | ccttctgcttttgaaagctataaaaacagcgagggaga (SEQ ID NO: ) |
| B15T-r | cgcgtctccctcgctgtttttatagctttcaaaagcagaaggagct (SEQ ID NO: ) |
| B15C | ccttctgcttttgaaagccataaaaacagcgagggaga (SEQ ID NO: ) |
| B15C-r | cgcgtctccctcgctgtttttatggctttcaaaagcagaaggagct (SEQ ID NO: ) |

### SNP B4 (-3737)

SNP B4 did not show transcription differences by allele, on the background of all other SNPs set at allele 1 (Figure 10).

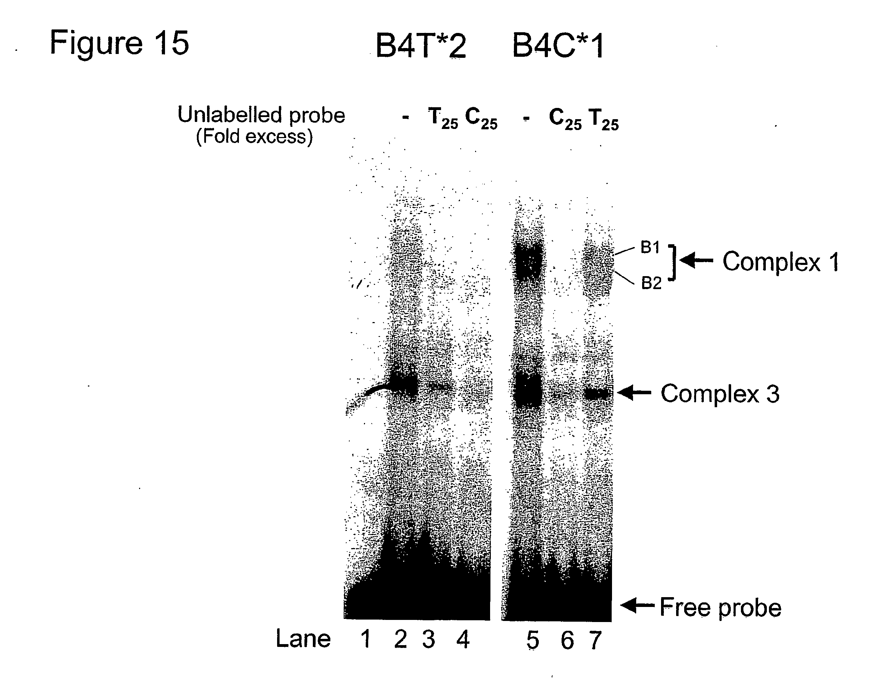

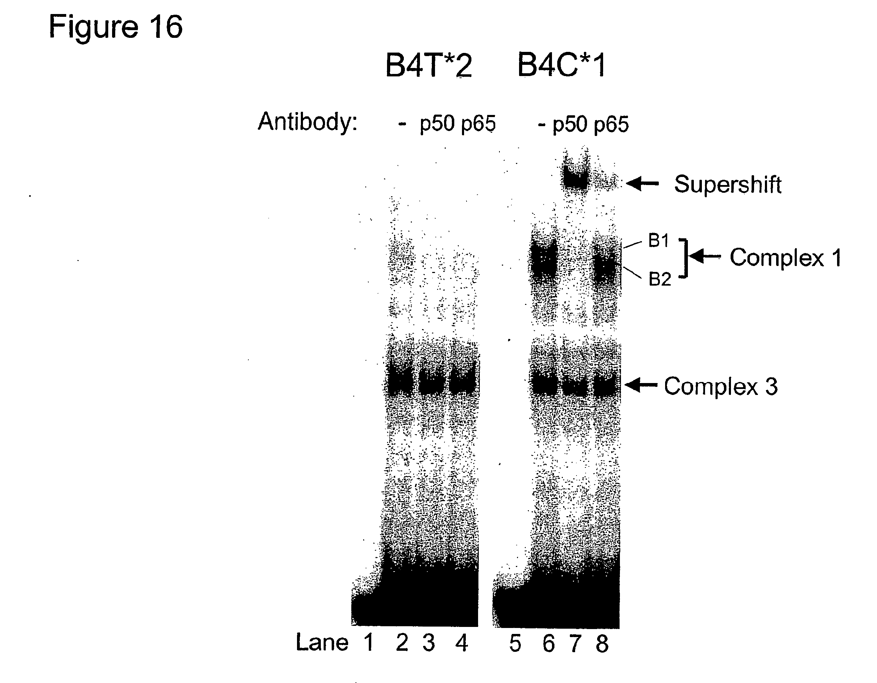

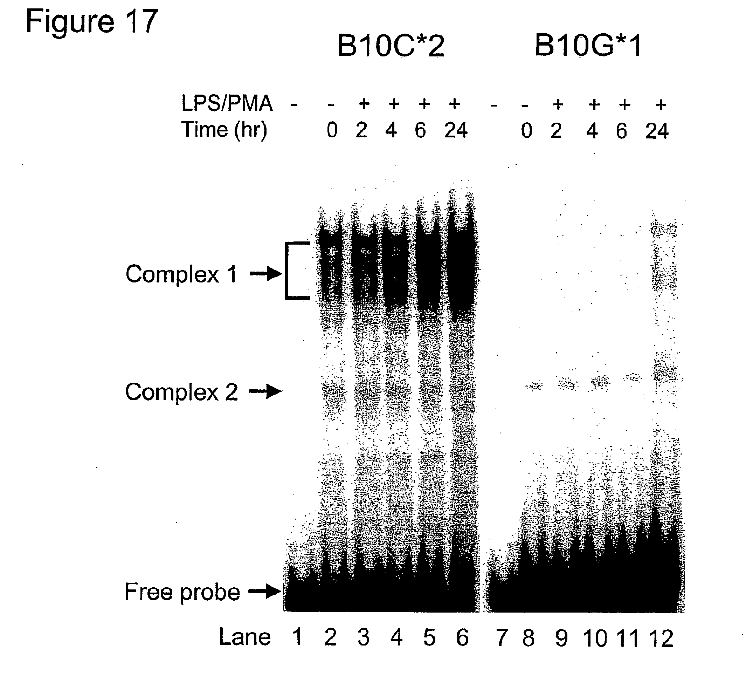

EMSA analysis shows significant protein binding differences between probes containing SNP B4 allele-1 and allele-2. (Figures 15-17) Both allele 1 and 2 show constant binding of a fast migrating band (complex 3) at 0, 4, 6 and 24 hours of LPS stimulation of cells. Allele 1 shows strong binding of two high mobility complexes following LPS stimulation. Complex 1 consists of two bands close to each other and is present beginning at 4 hours. At 24 hours, the lower band of complex 1 disappeared and complex 2 became evident (Figure 15), and competition with unlabeled probe demonstrated binding specificity (Figure 16). Allele 2 also bound complex 2 at 24 hours but there was no evidence of complex 1 at any of the time points. Because of the presence of a NF-κB regulatory element approximating SNP B4, antibodies for both p50 and p65 subunits of NF-κB were used in the EMSA incubation of allele-1 and allele-2 probes. As shown in Figure 17, lanes 7 and 8, p50 antibody is able to supershift both bands of complex 1 and p65 antibody is able to supershift only the upper band of the complex 1. This result indicates that LPS/PMA stimulated NF-kB binding preferentially to SNP B4 allele 1.

### SNP B10 (-1468)

In the background construct with all other SNPs set to allele 1, A) the presence of allele 2 at SNP B10 produced slightly lower transcriptional activity. (See Figure 11).

EMSA analysis using probes containing SNP10 allele-1 and allele-2 demonstrated clear differences in protein binding profiles (Figure 18-19) with SNP B10 allele 2 binding a group of high mobility (HMG) proteins (complex 1) more strongly than allele 2. Competition with unlabelled probe showed that this strong binding preference was allele-specific (Figure 19).

### SNP B14(-511)

The results of transcriptional analysis for individual affects of varying alleles at only SNP 14 are included in Figure 12. In background constructs in which all other SNPs are allele 1 (Fig 20), SNP B14 was converted to allele 2 (Fig 20) and showed higher promoter activity.

EMSA with probes containing allelic variants of SNP 14 showed 2 distinct complexes formed with this probe but no allelic differences in the mobility of protein complexes. (Figure 20). However, enhanced promoter activity by SNP 14 allele 2 indicates that there may be subtle differences in protein compositions between the two complexes bound to regulatory elements surrounding SNP 14 allele 1 and 2 that lead to functional impact on transcriptional activity.

### SNP 15(-31)

The effect of allelic variation at only SNP B 15 on transcriptional activity is also included in Figures 21-22. With the conversion of SNP B15 to allele 2, (Figure 21) there was a significant reduction of promoter activity as compared to the background construct.

### (Figure 21)

The EMSA protein profile of SNP B 15 is strikingly different for the allele-1 (T) and allele-2 (C) probes. (Figure 22). As shown in Figure 22, two intense bands formed with the allele 1 probe whereas 3 distinct bands formed with allele 2. Cold competition assay to determine the specificity of binding of proteins to these allelic probes were carried out (Figure 22) and indicated that the protein binding of complex I is highly specific for allele 2, whereas complex II and III have higher affinity for allele 1.

### Example 10. Determination of severity of periodontitis in subjects having IL-1B

### genotypes.

One indication of the severity of periodontitis in a subject is the pocket depth (PD). Severe periodontitis is indicated in a subject having a PD>2 in one or more gum pockets. The PD was measured in subjects having various IL-1B genotypes as shown in Table 10A (for example, a subject having genotype BP1 has two alleles of IL-1B (-511) allele 1, two alleles of IL-1B (-1468) allele 1, and two alleles of IL-1B (-3737) allele 2. Table 10B shows the percentage distribution of IL-1B haplotypes in subjects having varying degrees of periodontitis, as measured by the number of gum pockets per subject (fewer than 2 pockets, between 2 and 4 pockets, between 4 and 10 pockets, greater than 10 pockets).

**Table 10A**

| genotypes | SNP IL-1 B Allele | | |
|---|---|---|---|
| | -511 | -1468 | -3737 |
| BP1 | 1.1 | 1.1 | 2.2 |
| BP2 | 2.2 | 2.2 | 1.1 |
| BP3 | 1.1 | 1.1 | 1.1 |
| BP4 | 2.2 | 1.1 | 1.1 |

**Table 10B**

| | | | | |
|---|---|---|---|---|
| genotype | % distribution | | | |
| | 0-2 gum pockets2 to 4 gum pockets4 to 10 gum pockets>10 gum pockets | | | |
| BP1 | 0.429 | 0.089 | 0.304 | 0.179 |
| BP2 | 0.833 | 0.000 | 0.167 | 0.000 |
| BP3 | 0.375 | 0.094 | 0.188 | 0.344 |
| BP4 | 0.364 | 0.091 | 0.000 | 0.545 |

As shown in Table 10B and Figure 23, subjects having genotypes BP1, BP3 or BP4 have an increased likelihood of developing severe periodontitis, while subjects having the BP2 genotype have a decreased likelihood of developing severe periodontitis.

Example 11. Determination of severity of periodontitis in ethnic populations.

Previously, specific IL-1 gene variations (IL-1A(+4845), IL-1A(-889), and IL-1B(+3954)) have been associated with increased severity of periodontitis in Caucasians in multiple studies. These IL-1 SNPs are in low prevalence in Asian populations that have been studied, including Japanese, Koreans, and Chinese. Since the prevalence of a polymorphism and its penetrance relative to disease expression affect the sample size needed to identify association between the allele of interest and the disease expression, most of the previous studies in Asian populations have been too small to truly evaluate whether IL-1A(+4845) and IL-1B(+3954) are associated with periodontal disease. This example details the specific IL- gene variations that are predictive of more severe periodontitis in Japanese adults. This was performed by analyzing haplotypes across the IL-1 gene cluster for gene variations associated with clinical variance in periodontitis, using the high-density SNP mapping of all exons and regulatory regions of the genes for IL-1α (IL-1A), IL-1β (IL-1B), and the gene for IL-1 receptor antagonist (IL-1RN), which span the cluster of nine IL-1 genes on chromosome 2q13-14.

There are several predominant IL-1 haplotypes that are defined by six SNPs. Of the possible 64 haplotypes, 23 haplotypes accounted for >98% of the haplotypes in Caucasians and African-Americans. The same haplotypes accounted for >97% of the haplotypes observed in Japanese patients. (See Table 11A). Haplotypes containing allele 2 at IL-1RN(+2018) represented only 3.7% of the haplotypes, which would produce an expected rate of individuals homozygous at that locus of approximately 1/1000 subjects. Allele 2 at IL-1A(+4845) and at IL-1B(+3954) were also so infrequent that only approximately 1/100 individuals would be expected to be homozygous at that locus. Haplotypes were estimated using haplo.score version 1.0 (Schaid et al,2002). This software applies a version of the EM algorithm (Excoffier and Slatkin, 1995) to properly account for phase ambiguity.

| **Table 11A. Frequency of IL-1 haplotypes in subjects of diverse ethnicities.** | | | | | | | | | |
|---|---|---|---|---|---|---|---|---|---|
| **Gene:** | **IL-1A** | **IL-1B** | | | | **IL-1RN** | | | |
| | **4845** | **3954** | **-511** | **-1468** | **-3737** | **2018** | **Freq Japanese** | **Freq Caucasians** | **Freq Black** |
| **Haplotype** | | | | | | | | | |
| **IL1C1** | **1** | **1** | **1** | **1** | **2** | **1** | **0.458** | **0.339** | **0.241** |
| **IL1C2** | **2** | **2** | **1** | **1** | **1** | **1** | **0.039** | **0.145** | **0.029** |
| **IL1C3** | **1** | **1** | **2** | **2** | **1** | **2** | **0.03** | **0.137** | **0.05** |
| **IL1C4** | **1** | **1** | **2** | **2** | **1** | **1** | **0.279** | **0.097** | **0.056** |
| **IL1C5** | **1** | **1** | **1** | **1** | **2** | **2** | **0.007** | **0.071** | **ND** |
| **IL1C6** | **1** | **1** | **2** | **1** | **1** | **1** | **0.048** | **0.038** | **0.331** |
| **IL1C7** | **2** | **1** | **1** | **1** | **2** | **1** | **0.015** | **0.031** | **0.013** |
| **IL1C8** | **1** | **2** | **1** | **1** | **1** | **1** | **ND** | **0.021** | **ND** |
| **IL1C9** | **2** | **1** | **2** | **2** | **1** | **2** | **ND** | **0.02** | **ND** |
| **IL1C10** | **2** | **2** | **1** | **1** | **1** | **2** | **ND** | **0.019** | **ND** |
| **IL1C11** | **2** | **1** | **2** | **1** | **1** | **1** | **0.025** | **0.016** | **0.039** |
| **IL1C12** | **1** | **1** | **1** | **1** | **1** | **1** | **0.027** | **0.015** | **0.061** |
| **IL1C13** | **2** | **2** | **2** | **2** | **1** | **1** | **ND** | **0.01** | **ND** |
| **IL1C14** | **2** | **2** | **1** | **1** | **2** | **1** | **ND** | **0.007** | **0.019** |
| **IL1C15** | **2** | **2** | **2** | **2** | **1** | **2** | **ND** | **0.007** | **ND** |
| **IL1C16** | **1** | **1** | **2** | **1** | **1** | **2** | **ND** | **0.006** | **0.009** |
| **IL1C17** | **2** | **1** | **2** | **2** | **1** | **1** | **0.008** | **0.006** | **ND** |
| **IL1C18** | **1** | **1** | **1** | **2** | **1** | **1** | **0.021** | **ND** | **ND** |
| **IL1C19** | **1** | **1** | **2** | **1** | **2** | **1** | **0.01** | **ND** | **ND** |
| **IL1C20** | **2** | **1** | **1** | **1** | **1** | **1** | **0.005** | **ND** | **0.049** |
| **IL1C21** | **1** | **1** | **1** | **1** | **1** | **2** | **?** | **?** | **0.008** |
| **IL1C22** | **1** | **2** | **2** | **1** | **1** | **1** | **?** | **?** | **0.035** |
| **IL1C23** | **2** | **2** | **2** | **1** | **1** | **1** | **?** | **?** | **0.047** |
| | | | | | | | | | |
| | | | | | | **Totals** | **0.972** | **0.985** | **0.987** |

Three SNPs in the IL-1B promoter have been identified that are functional and highly prevalent in Japanese subjects. These SNPs include IL-1B (-511), IL-1B (-1468), and IL-1B (-3737). Figure 24 shows the relative haplotype frequency distribution of the alleles listed in Table 11A in Caucasian and Japanese subjects. The present inventors have determined that the predominant haplotypes, based on these 3 SNPs, show significant differences in IL-1β levels in gingival fluid (GCF). Figure 25 demonstrates that Caucasian subjects having the H1 or H3 genotype have increased inflammation, as measured by comparison with all other haplotypes using a score statistic. A score statistic is a statistic used to evaluate the statistical significance of parameter estimates computed via maximum likelihood methods. It is also sometimes called the *efficient score statistic.* The test is based on the behavior of the log-likelihood function at the point where the respective parameter estimate is equal to 0.0 (zero); specifically, it uses the derivative (slope) of the log-likelihood function evaluated at the null hypothesis value of the parameter (parameter = 0.0).

In subjects of Japanese ethnicity, it has been determined that predisposition to periodontal disease and the severity of periodontal disease is affected by IL-1 haplotype. One method of measuring periodontal disease uses the generalized Bleeding on Probing test (BOP). As shown in Figure 26, those Japanese subjects that have a genotype that is homozygous at IL-1B (-511) allele 2, homozygous at IL-1B (-1468) allele 2, and homozygous at IL-1B (-373) allele 1 have decreased risk for periodontitis as measured by generalized BOP. This decreased risk is observed at two different percentage increases in plaque-covered surfaces.

Another method of measuring periodontal disease that enables the association to be made between specific IL-1 haplotypes and severity of periodontitis involves measurement of probing depth and probing attachment level of a subject. These two measurements are taken from individuals, whose haplotypes are identified, and the mean percentage of sites measured having probing depth greater than 4 mm, or probing attachment level greater or equal to than 4 mm are determined. As shown in Figure 27, Japanese subjects having a H1, H3 or H4 IL-1 haplotype have an increased severity of periodontitis compared to subjects having an H2 haploytpe, as measured by these methods.

## Claims

1. A method for predicting a human subject's susceptibility to periodontal disease comprising the step of identifying in a genomic DNA sample from the subject an allelic pattern that comprises one of the following sets of IL-1B alleles:
(1) (-511) allele 1, (-1468) allele 1, and (-3737) allele 2;
(2) (-511) allele 2, (-1468) allele 2, and (-3737) allele 1;
(3) (-511) allele 1, (-1468) allele 1, and (-3737) allele 1; or
(4) (-511) allele 2, (-1468) allele 1, and (-3737) allele 1,
wherein the presence of any one of allelic patterns 1, 3 or 4 indicates that said subject has increased susceptibility to periodontal disease, and wherein the presence of allelic pattern 2 indicates that the subject has a decreased susceptibility to periodontal disease.

2. The method of claim 1, wherein the subject is homozygous for each of the alleles in the allelic pattern.

3. The method of claim 1 or 2, wherein said subject is of Asian ethnicity.

## Patentansprüche

1. Verfahren zur Vorhersage der Anfälligkeit eines menschlichen Individoums für eine periodontale Erkrankung, aufweisend den Schritt des Identifizierens in einer genomischen DNA-Probe von dem Individuum eines Allelmusters, das einen der folgenden Sätze von IL-1B-Allelen aufweise:
(1) (-511) Allel 1, (-1468) Allel 1, und (-3737) Allel 2;
(2) (-511) Allel 2, (-1468) Allel 2, und (-3737) Allel 1;
(3) (-511) Allel 1, (-1468) Allel 1, und (-3137) Allel 1; oder
(4) (-511) Allel 2, (1468) Allel 1, und (-3737) Allel 1,
wobei das Vorliegen eines der Allelmuster 1, 3 odr 4 anzeigt, dass das Individuum eine erhöhte Anfälligkeit für eine periodontale Erkrankung hat, und wobei das Vorliegen des Allelmusters 2 anzeigt, dass das Individuum eine verringerte Anfälligkeit für eine periodontale Erkrankung hat.

2. Verfahren nach Anspruch 1, wobei das Individuum für jedes der Allele in dem Allelmuster homozygot ist.

3. Verfahren nach Anspruch 1 oder 2, wobei das Individuum von asiatischer Ethnizität ist.

## Revendications

1. Procédé pour prédire la susceptibilité chez un sujet humain à une parodonthopathie, comprenant l'étape d'identification dans un échantillon d'ADN génomique d'un sujet, d'un motif allélique qui comprend un des jeux d'allèles IL-1B suivants :
(1) allèle 1 (-511), allèle 1 (-1468), et allèle 2 (-3737) ;
(2) allèle 2 (-511), allèle 2 (-1468), et allèle 1 (-3737) ;
(3) allèle 1 (-511), allèle 1 (-1468), et allèle 1 (-3737) ; ou
(4) allèle 2 (-511), allèle 1 (-1468), et allèle 1 (-3737) ;
dans lequel la présence d'un quelconque des motifs alléliques 1, 3 ou 4 indique que ledit sujet a une susceptibilité accrue à une parodonthopathie, et la présence d'un motif allélique 2 indique que le sujet a une susceptibilité réduite à une parodonthopathie.

2. Procédé de la revendication 1, dans lequel le sujet est homozygote pour chacun des allèles du motif allélique.

3. Procédé selon la revendication 1 ou 2, dans lequel ledit sujet est d'ethnicité asiatique.
